# EUROPEAN PATENT APPLICATION

(11) **EP 2 522 726 A1**
(43) Date of publication of application: **14.11.2012**
(21) Application number: 11165939.7
(22) Date of filing: 12.05.2011
(51) Int. Cl.: C12N 15/09

(54) **Zinc finger nucleases for p53 editing**

(71) Applicant: Fundació Privada Centre de Regulació Genòmica (CRG), 08003 Barcelona (ES)
(72) Inventor: Isalan, Mark, 08003 Barcelona (ES); Herrmann, Frank, 08003 Barcelona (ES)
(74) Representative: Moore, Michael Richard

(57) **Abstract**

A method for replacing a p53 gene sequence with an exogenous nucleic acid sequence is described. The method comprises contacting the p53 gene sequence with one or more zinc finger nuclease (ZFN) in the presence of a donor nucleic acid comprising the exogenous nucleic acid sequence. The ZFN comprises a zinc finger peptide (ZFP) portion comprising four or more zinc finger domains, and one or more endonuclease portion. Also disclosed are ZFNs that recognise at least 12bp of a p53 gene sequence and that are capable of cleaving the p53 gene sequence, e.g. to promote homologous recombination and enable replacement of mutated p53 gene sequences. The methods and ZFNs have particularly utility in treating cancers associated with the p53 gene and protein. A method for identifying useful nucleic acid binding polypeptides is also described. The method may be based on a yeast one-hybrid assay.

## Description

### Field of the Invention

This invention relates to novel zinc finger peptides, in particular zinc finger nucleases, having desirable properties, including binding specificity and/or nuclease activity against selected polynucleotide and genomic sequences. The invention further relates to zinc finger nucleases for use in the alteration or repair of target genes *in vivo,* such as for the repair or mutation of p53 genes involved in cancer.

### Background of the Invention

The tumour suppressor p53 serves as a "guardian of the genome" (Lane (1992), Nature 358: 15-16) and has been studied intensively for over 30 years. p53 is activated as a transcription factor in response to cellular stresses, such as DNA damage, hypoxia and cell-cycle aberrations. It can thus help to promote the repair and survival of damaged cells by inducing cell-cycle arrest, or it can promote the permanent removal of damaged cells by inducing programmed cell death or senescence (Levine & Oren (2009), Nat. Rev. Cancer 9: 749-758).

As the p53 gene is either mutated or deleted in more than 50% of human tumours (Hollstein et al., (1991), Science 253: 49-53), functional p53 is clearly very important in protecting against cancer development. The vast majority of p53 mutations in human tumours are single missense mutations that cluster in the core DNA-binding domain of the protein (i.e. between amino acid residues 100 to 300). These mutations can lead to both the disruption of normal p53 function and the accumulation of high levels of mutant p53 with various gain-of-function activities (reviewed in Brosh & Rotter (2009), Nat. Rev. Cancer 9: 701-713; and Soussi & Lozano (2005), Biochem. Biophys. Res. Commun. 331: 834-842).

Furthermore, it has been noted that tumours with mutant p53 often present increased chemo- and radio-resistance, making many p53-mutant cancers harder to treat using conventional therapies. Therefore, mutant p53 is an appealing molecular target for tumour suppression therapies. Moreover, the restoration of p53 function is considered an important issue in cancer therapy (Lu & El-Deiry (2009), Apoptosis 14: 597-606; Ventura et al., (2007), Nature 445: 661-665; and Xue et al., (2007), Nature 445: 656-660). Several therapeutic strategies have therefore been pursued to date, including expressing wild-type (wt) p53 in gene therapy, eliminating mutant p53 cancer cells with adenovirus, and directly restoring normal p53 function with small molecules that alter mutant p53 conformation (reviewed in Chen et al., (2010), Biochem. Pharmacol. 80: 724-730).

Despite p53 being a desirable target, the necessary tools to carry out the most direct approach to p53 cancer therapy, i.e. to modify genomes at will at disease loci, are still lacking. Therefore, it would be desirable to have alternative and/or more effective therapeutic molecules and treatments for cancers and other genetic disorders, particularly those that include mutant p53 genes.

A relatively recently developed technology, zinc finger nucleases (see e.g. Kim et al., (1996), Proc. Natl. Acad. Sci. USA 93: 1156-1160; and Smith et al., (2000), Nucleic Acids Res. 28: 3361-3369), has shown some utility as a possible genome engineering tool (reviewed in Carroll (2008), Gene Ther. 15: 1463-1468; and Cathomen & Joung (2008), Mol. Ther. 16: 1200-1207). In such approaches, zinc finger proteins are first (re)engineered to bind a desired DNA sequences (reviewed in Pabo et al., (2001), Annu. Rev. Biochem. 70: 313-340), and a nuclease domain is then attached to the zinc finger peptide to create a so-called 'zinc finger nuclease' (ZFN). Once bound to its target site the DNA sequence adjacent the zinc finger binding site may be cut by the nuclease. Thus, a ZFN may effectively allow a type of 'genome sculpting' where externally provided DNA can be recombined precisely into a genome (Bibikova et al., (2001), Mol. Cell Biol. 21: 289-297) around the nuclease-cut site, resulting in site-specific gene repair, mutation, insertion or deletion.

ZFN were first demonstrated for use in gene targeting of a mutant Drosophila yellow gene (e.g. Bibikova et al., (2002), Genetics 161: 1169-1175); and have since been used in model organisms such as *C. elegans* and Drosophila (Morton et al., (2006), Proc. Natl. Acad. Sci. USA 103: 16370-16375; and Beumer et al., (2008), Proc. Natl. Acad. Sci. USA 105: 19821-19826); zebrafish (e.g. Doyon et al., (2008), Nat. Biotechnol. 26: 702-708); mouse (Carbery et al., (2010), Genetics); and plants (Osakabe et al., (2010), Proc. Natl. Acad. Sci. USA 107: 12034-12039). In addition, ZFNs have been used to target disease loci, such as IL2RG (mutated in severe combined immunodeficiency; SCID-X1), where first gene repair (Urnov et al., (2005), Nature 435: 646-651), and then exogenous gene integration (Moehle et al., (2007), Proc. Natl. Acad. Sci. USA 104: 3055-3060) were reported. More recently, the technology has been extended to mammalian systems such as stem cells (e.g. Zou et al., (2009), Cell. Stem Cell 5: 97-110); the induction of cellular HIV resistance (e.g. Holt et al., (2010), Nat. Biotechnol. 28: 839-847); and even whole rat knockouts (e.g. Geurts et al., (2009), Science 325: 433). However, it would be beneficial to have further ZFNs that have utility in targeting diseased genes for therapeutic applications.

The specificity of ZFNs depends on artificially-engineered DNA-binding domains: multi-zinc finger arrays that recognise long DNA sequences. However, whereas established screening systems exist for 1- to 2-finger libraries (e.g. phage display) and 3-finger mini-libraries of pre-selected modules (e.g. bacterial two-hybrid; Hurt et al., (2003), Proc. Natl. Acad. Sci. USA 100: 12271-12276), no straightforward system exists to optimise the 4-to 6-finger type scaffolds which are generally considered necessary for specific zinc finger targeting within complex genomes, such as humans. Therefore, suboptimal zinc finger candidates often have to be abandoned because of a lack of straightforward optimisation protocols.

Hence, it would be highly desirable to have alternative or improved methods and strategies for the selection and/or testing and/or optimisation and/or improvement of zinc finger domains for specific nucleic acid binding / recognition.

Accordingly, the present invention seeks to overcome or at least alleviate one or more of the problems in the prior art.

### Summary of the Invention

In general, the present invention provides new zinc finger peptides (ZFPs) and zinc finger nucleases (ZFNs), which comprise a ZFP and a nuclease domain. The ZFPs and/or ZFNs of the invention may be used for the modulation of gene expression *in vitro* and/or *in vivo,* and may also be useful for ex *vivo* applications. The ZFNs of the invention may have particularly utility in a method or use for replacing or mutating target nucleic acid sequences comprising the region recognised by the ZFP or ZFN. More specifically, the ZFPs and ZFNs of the invention may be capable of binding to and/or cleaving a p53 gene sequence. The ZFNs may, therefore, be used in conjunction with a donor nucleic acid molecule capable of homologous recombination with the target nucleic acid sequence, so as to replace (e.g. to correct) a mutated gene sequence in order to restore normal function to the target gene. Alternatively, the ZFPs or ZFNs may be used to mutate, delete or modify the expression of the target gene, such as for the creation of gene knock-outs. The ZFNs of the invention include paired 4-finger nucleic acid binding domains.

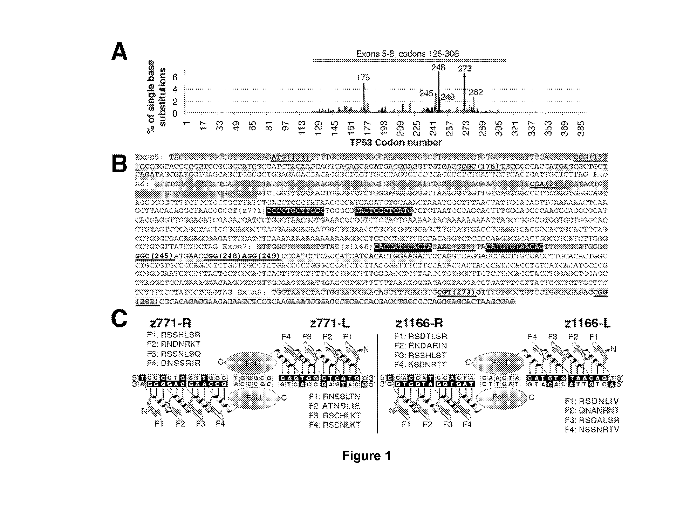

Rational design was used to create non-optimised ZFPs and ZFNs able to recognise and bind desired nucleic acid sequences. Since classical phage display and other selection / optimisation platforms could not handle the complexity of ZFPs containing 4 or more fingers, a novel assay was developed to optimise poly-zinc finger peptides from libraries. The utility of the novel selection and optimisation platform of the invention was exemplified by the identification of a number of functional ZFNs that target different sites (both exonic and intronic) located within the human p53 gene. The target sites in the p53 gene are advantageously in close proximity to the mutation hotspots of p53 in somatic cancers (see Figure 1). Thus, the invention is also generally directed to a novel method for identifying and/or selecting desirable nucleic acid binding polypeptides, and in particular ZFPs that recognise a target nucleic acid sequence. A specific embodiment of the novel method is a yeast one-hybrid assay. The invention encompasses research tools, therapeutic compositions and treatments for diseases such as cancer; particularly cancer associated with p53.

Accordingly, in one aspect of the invention there is provided a method for cleaving a p53 gene sequence, the method comprising: contacting a nucleic acid molecule comprising the p53 gene sequence to be cleaved with one or more zinc finger nuclease (ZFN), the ZFN comprising a zinc finger peptide (ZFP) portion comprising four or more engineered zinc finger domains and one or more endonuclease portion. Advantageously, for the targeting of nucleic acid sequences within complex genomes the ZFP portion recognises at least 12bp (e.g. 12bp, 15bp, 18bp or more) of the p53 gene sequence. The amino acids sequences of the nucleic acid recognition regions of the ZFP domains may be designed rationally and/or by random mutagenesis and screening or selection. In a preferred embodiment the nucleic acid recognition regions of the ZFP domains are created first by rational design followed by optimisation through a selection protocol. Therefore, the ZFP is generally not naturally occurring and is thus an engineered ZFP. Following cleavage of the target nucleic acid sequence, a gene sequence or segment may be mutated, deleted or replaced. If the target gene sequence contains mutations, then replacement of the sequence with a normal (wild-type, wt) sequence may be desirable. The target sequence may be an animal genomic sequence, such as from human, rat, mouse, pig, sheep or cow. In some aspects and embodiments this and other methods of the invention may exclude subject-matter that is excluded from patentability in a particular territory, such as, methods for the treatment of the human or animal body by surgery or therapy under Article 53(c) EPC. Thus, the methods of the invention may be used in vivo, in vitro and/or ex vivo.

In another aspect the invention provides a method for replacing a p53 gene sequence with an exogenous nucleic acid sequence. The method may comprise contacting a nucleic acid molecule comprising the p53 gene sequence to be replaced with one or more zinc finger nuclease (ZFN) in the presence of a donor nucleic acid comprising the exogenous nucleic acid sequence. Beneficially the ZFN comprises a zinc finger peptide (ZFP) portion comprising four or more zinc finger domains, and one or more endonuclease portion. Suitably, the ZFP portion recognises at least 12bp of the p53 gene sequence in a region of the p53 gene sequence to be replaced. The ZFN cleaves the p53 gene sequence within the region of the p53 gene sequence to be replaced, and cleavage of the p53 gene sequence by the ZFN stimulates incorporation of the exogenous nucleic acid sequence. The exogenous nucleic acid sequence may be incorporated into the p53 gene sequence by homologous recombination. Thus, the method of the invention may suitably comprise transforming the nucleic acid(s) into a cell in which homologous recombination can be performed, such as yeast or mammalian cells.

Advantageously, the exogenous nucleic acid sequence is different to the p53 gene sequence to be replaced at one or more of the at least 12bp recognised by the ZFP portion. In this way, the ZFN does not bind to and cleave the donor exogenous gene sequence. The p53 gene sequence to be replaced and/or the p53 gene sequence recognised by the ZFN is associated with a p53 gene, but may not necessarily comprise a p53 exon sequence. For example, it may alternatively comprise a p53 intronic sequence, or a promoter, enhancer or other related chromosomal sequence associated with the p53 gene. In one embodiment, the p53 gene sequence to be replaced is a mutated p53 gene sequence, and the exogenous nucleic acid sequence is a wild-type p53 gene sequence. Alternatively, the exogenous nucleic acid sequence encodes a wild-type p53 protein, but may have one or more silent mutations, e.g. in order to create a beneficial nucleic acid sequence without affecting the sequence of the encoded protein. In some embodiments, the exogenous nucleic acid sequence encodes a wild-type p53 protein, but may have one or more DNA mutations, deletions or insertions, in order to alter the function of the endogenous p53 locus. Suitably, the exogenous p53 gene sequence comprises a region of the core DNA-binding domain of the p53 protein, for example, a portion of the gene encoding for at least some of the amino acid residues between positions 100 and 300 of wild-type p53. More suitably, the exogenous nucleic acid sequence comprises at least 50bp, at least 100bp, at least 200bp, at least 300bp or at least 500bp of the region encoding for the amino acids between positions 100 and 300 of p53. In a preferred embodiment the exogenous nucleic acid comprises a polynucleotide sequence encoding for at least amino acids 100 to 300 of p53. Alternatively, the exogenous p53 gene sequence may comprise one or more of exons 5 to 8 or exons 6 to 8 of p53 and/or the intervening intronic sequences. Particularly preferred are mammalian p53 gene sequences, such as primate or porcine.

In yet another aspect, there is provided a method for mutating a p53 gene sequence, which comprises contacting a nucleic acid molecule comprising the p53 gene sequence to be mutated with one or more zinc finger nuclease (ZFN), the ZFN comprising a zinc finger peptide (ZFP) portion comprising four or more engineered zinc finger domains and one or more endonuclease portion. The ZFP portion beneficially recognises at least 12bp of the p53 gene sequence in a region of the p53 gene sequence to be mutated, and the ZFN cleaves the p53 gene sequence within the region of the p53 gene sequence to be mutated, such that cleavage of the p53 gene sequence by the ZFN stimulates mutation of the p53 gene sequence by non-homologous end joining (NHEJ).

In some preferred embodiments, the methods of the invention further comprise introducing at least two polynucleotides into a cell containing the p53 gene sequence to be replaced. In these embodiments, a first polynucleotide may comprise a nucleic acid sequence encoding a ZFN, and a second polynucleotide may comprise an exogenous nucleic acid sequence. The first polynucleotide may encode for one or more (e.g. two) different ZFNs. Alternatively, the methods may comprise introducing two or more polynucleotides into a cell, which each encode for a different ZFN, and optionally another polynucleotide comprising an exogenous nucleic acid sequence.

Thus, in other embodiments, the methods may comprise contacting the nucleic acid molecule comprising the p53 gene sequence with a first and a second ZFN, the first ZFN comprising a first ZFP portion and a first endonuclease portion, and the second ZFN comprising a second ZFP portion and a second endonuclease portion. The first and second ZFP portions may each comprise four or more engineered zinc finger domains, the first ZFP portion recognising a first p53 gene sequence comprising at least 12bp and the second ZFP portion recognising a second p53 gene sequence comprising at least 12bp. Suitably, the first and second p53 gene sequences do not overlap with each other. More suitably, the two sequences are located within approximately 100bp of each other within the p53 gene sequence, still more preferably within approximately 50bp of each other. In some cases, the closest edges of the recognition sequences may be within 30bp, within 20bp or within 10bp of each other. In these embodiments, the first and second endonuclease portions of each polypeptide are beneficially partial nucleic acid cleavage domains, which are unable to cleave the target nucleic acid in isolation, but are able to interact (e.g. associate or bind to one another) to constitute a functional endonuclease domain. The partial endonuclease domains may be capable or homodimerisation (associating with another identical domain) or may only be capable of heterodimerisation in order to form a functional endonuclease domain. Once both ZFNs are simultaneously bound to their respective target sites, the p53 gene sequence may therefore be cleaved.

Suitably, the p53 gene sequence is located within a p53 gene, and the nucleic acid molecule comprising the p53 gene sequence is a chromosome. Advantageously, therefore, the nucleic acid molecule comprising the p53 gene sequence is chromosomal DNA within a cell; and so the ZFN is desirably expressed in the cell. The cell may be an animal cell, and suitable a mammalian cell such as a human, rat, mouse, pig, sheep or cow cell.

The methods of the invention may also comprise obtaining a cell from an animal, such as a pig; altering a p53 gene sequence in the genome of the cell by a method according to the invention, or using a polypeptide according to the invention; and optionally re-implanting the cell (or cells derived therefrom) into an animal. The cell may, for example, be an egg or a stem cell.

The invention further provides polypeptides, such as zinc finger nucleases (ZFNs), for use in altering a p53 gene sequence, e.g. for use in mutating or replacing a p53 gene sequence. Generally, the use involves the same procedures as described above and elsewhere herein in relation to the methods of the invention.

In yet another aspect of the invention there is provided a polypeptide comprising a non-naturally occurring zinc finger peptide (ZFP), the ZFP preferably comprising four or more engineered zinc finger domains, wherein the amino acid sequence of the nucleic acid recognition region (i.e. positions -1,+1,+2,+3,+4,+5,+6) of each of the four engineered zinc finger domains (numbered F1, F2, F3, F4 in N to C terminal order) is substantially identical to the zinc finger nucleic acid recognition region sequences in the groups: (i) F1: RSSHLSR (SEQ ID NO: 16); F2: RNDNRKT (SEQ ID NO: 17); F3: RSSNLSQ (SEQ ID NO: 18); F4: DNSSRIR (SEQ ID NO: 19); (ii) F1: RNSSLTN (SEQ ID NO: 20); F2: ATNSLIE (SEQ ID NO: 21); F3: RS(G/C)HLKT (SEQ ID NO: 22); F4: RSDNLKT (SEQ ID NO: 23); (iii) F1: RSDTLSR (SEQ ID NO: 24); F2: RKDARIN (SEQ ID NO: 25); F3: RSSHLST (SEQ ID NO: 26); F4: KSDNRTT (SEQ ID NO: 27); (iv) F1: RSDNLIV (SEQ ID NO: 28); F2: QNANRNT (SEQ ID NO: 29); F3: RSDALSR (SEQ ID NO: 30); F4: NSSNRTV (SEQ ID NO: 31). For example, the amino acid recognition sequences may be identical in at least 6 of the 7 positions of the corresponding recognition sequences. Preferably, the amino acid recognition sequences are identical to those given above. In some preferred embodiments, a ZFN of the invention comprises the amino acid recognition sequences of F1 to F4 given in any of groups (i) to (iv) above. Polypeptides comprising such ZFP domains are particularly suitable for targeting of human p53 genomic sequences. In an alternative embodiment, the polypeptide of the invention may comprise a ZFP or ZFN comprising four zinc finger domains having nucleic acid recognition sequences substantially the same or identical to the group: (v) F1: RSSNLIV (SEQ ID NO: 32); F2: QNANRNT (SEQ ID NO: 33); F3: RSSALSR (SEQ ID NO: 34); F4: NSSNRTV (SEQ ID NO: 35). Such a polypeptide may be particularly useful for targeting pig, sheep and/or mouse genomic p53 sequences. In some embodiments, however, the ZFP or ZFN of the invention may comprise an array of more than four zinc finger domains. In such embodiments the ZFP or ZFN may comprise four adjacent zinc finger domains in N-terminal to C-terminal order having one or more groups of zinc finger domains having the recognition sequences of groups (i) to (v) above. The four-finger groups need not necessarily correspond to the N-terminal four zinc finger domains.

The polypeptide of the invention may have a variety of uses and, therefore, may further comprise one or more effector domain selected from transcriptional repressor domains, transcriptional activator domains, transcriptional insulator domains, chromatin remodelling, condensation or decondensation domains, nucleic acid or protein cleavage domains, dimerisation domains, enzymatic domains, signalling / targeting sequences or domains. Suitably, the one or more effector domain is an endonuclease domain or an active or inactive fragment or portion thereof. Particularly the effector may comprise at least a fragment of a Type IIS restriction endonuclease, such as a *Fok*l endonuclease. Advantageously, the nuclease portion is a partial (e.g. half) domain which may dimerise to form an active endonuclease.

Thus, a preferred embodiment of the invention relates to a polypeptide which is a ZFN fusion polypeptide, comprising a ZFP having at least four zinc finger domains (F1 to F4) selected from one of the groups: (i) F1: RSSHLSR; F2: RNDNRKT; F3: RSSNLSQ; F4: DNSSRIR; (ii) F1: RNSSLTN; F2: ATNSLIE; F3: RS(G/C)HLKT; F4: RSDNLKT; (iii) F1: RSDTLSR; F2: RKDARIN; F3: RSSHLST; F4: KSDNRTT; (iv) F1: RSDNLIV; F2: QNANRNT; F3: RSDALSR; F4: NSSNRTV; and (v) F1: RSSNLIV; F2: QNANRNT; F3: RSSALSR; F4: NSSNRTV; and a covalently linked Fokl partial endonuclease domain. Such polypeptides are beneficially used in pairs to specifically target and cleave a desired gene. Preferred pairs of ZFNs include a polypeptide of group (i) in combination with a polypeptide of group (ii); and a polypeptide of group (iii) in combination with a polypeptide of group (iv) or (v) above. Accordingly, the invention may provide compositions comprising at least a first and a second polypeptide of the invention.

It will be appreciated that the ZFPs and ZFNs of the invention may target nucleic acid sequences in any desirable polynucleotide, gene or genome. However, preferred target sequences are within the p53 gene or fragments thereof. Suitable target sites include human p53 gene sequences, such as TCCCCTGCTTGGCTGGGCGCAGTGGCTCATGC (SEQ ID NO: 36) and CCACCATCCACTACAACTACATGTGTAACAGT (SEQ ID NO: 37); porcine p53 gene sequences, such as CCACCATCCACTACAACTtCATGTGcAACAGc (SEQ ID NO: 38); ovine p53 gene sequences, such as CCACCATCCACTACAACTtCATGTGTAACAGc (SEQ ID NO: 39); and mouse p53 gene sequences, such as CCACCATCCACTACAAGTACATGTGTAAtAGc (SEQ ID NO: 40); or sequences comprising 12 or more consecutive nucleotides thereof. In some preferred embodiments, the ZFP of the invention recognises a target nucleic acid sequence comprising one or more sequence selected from: (i) 5'- GCCAAGCAGGGG -3' (SEQ ID NO: 41); (ii) 5'- CAGTGGCTCATG -3' (SEQ ID NO: 42); (iii) 5'- TAGTGGATGGTG -3' (SEQ ID NO: 43); and (iv) 5'- CATGTGTAACAG -3' (SEQ ID NO: 44). In an alternative embodiment, the target nucleic acid may comprise the sequence (v) 5'-CATGTGCAACAG -3' (SEQ ID NO: 45), which is found in the porcine p53 gene. In specific embodiments, target nucleic acid sequences (i) to (v) (i.e. SEQ ID NOs: 41 to 45) are recognised by ZFPs or ZFNs of groups (i) to (v), respectively.

The invention further encompasses nucleic acids / polynucleotides that encode one or more polypeptide, ZFP or ZFN of the invention, as well as the ZFP and ZFN libraries and fusion proteins described herein. Suitable expression vectors and viral vectors are therefore encompassed.

In other aspects, the invention provides for therapeutic (medical) and non-therapeutic (e.g. diagnostic and research) uses for the polypeptides, ZFPs, ZFNs and polynucleotides of the invention. Aspects and embodiments of the invention therefore include formulations, medicaments and pharmaceutical compositions comprising the zinc finger peptides. In one aspect, for example, the invention relates to a polypeptide, ZFP, ZFN or polynucleotide of the invention for use in medicine. Methods of treating diseases in humans or other animals are also encompassed. In particular, the compositions and peptides of the invention may be useful for deleting, mutating or replacing target nucleic acid sequences, e.g. target genes or portions of target genes within cells. Such uses and methods may be *in vivo*, *in vitro* or *ex vivo.* Thus, the ZFPs and ZFNs of the invention may be used in the treatment of various diseases and conditions of the human or animal body, relating to aberrant gene expression or expression of aberrant genes. More specifically, the ZFPs, ZFNs, polynucleotides and therapeutics of the invention may be used for the treatment of cancer, particularly cancer related to aberrant expression of p53 or expression of aberrant p53. Treatment may also include preventative as well as therapeutic treatments and alleviation of a disease or condition. Alternatively, the ZFNs of the invention may be used in the generation of p53 knock-out animals, e.g. for use as cancer models.

The invention, therefore, also encompasses pharmaceutical compositions comprising the one or more of the polypeptides and/or the polynucleotides of the invention; as well as the use of the polypeptides and/or polynucleotides in the manufacture of a medicament.

The invention is further directed to a method for identifying nucleic acid binding moieties, such as polypeptides that bind to a desired nucleic acid target sequence. Thus, in one aspect there is provided a method for identifying a polypeptide that binds to a nucleic acid bait sequence element (e.g. a target binding site) within a cell. The method may comprise providing a cell comprising a nucleic acid bait construct capable of expressing a detectable reporter-selection gene when the polypeptide binds to the bait sequence element, the nucleic acid bait construct comprising the reporter-selection gene and a single copy of the bait sequence element; and transforming the cell with an expression vector comprising the polypeptide gene and capable of expressing the polypeptide in the cell; wherein expression of the reporter-selection gene indicates that the polypeptide has bound to the bait sequence element in the cell. Suitably, the cell is a yeast cell.

The method may suitably comprise simultaneously transforming the cell with the nucleic acid bait construct and the expression vector. A preferred embodiment is a form of 'yeast one-hybrid' assay. Thus, the expression vector may advantageously be formed by homologous recombination within the cell, between a nucleic acid prey construct comprising a nucleic acid sequence encoding a transcription factor activation domain, and a nucleic acid construct comprising a nucleic acid sequence encoding the polypeptide. In a particularly advantageous embodiment, the expression vector comprises a nucleic acid sequence that encodes a fusion protein in which the polypeptide is located N-terminal to a transcription factor activation domain. The bait sequence element suitably comprises at least 12bp (e.g. 12bp, 15bp, or 18bp), although longer sequences, such as at least 24bp may be used. Generally, the sequence is contiguous, although non-contiguous sequences may also be suitable, depending on the polypeptide concerned. The bait sequence element may be any desirable sequence, e.g. a natural or mutated gene sequence. In some embodiments it is a p53 gene sequence, for example, comprising one of the following sequences (SEQ ID NOs: 41 to 45): (i) 5'- GCCAAGCAGGGG -3'; (ii) 5'- CAGTGGCTCATG -3'; (iii) 5'-TAGTGGATGGTG -3'; and (iv) 5'- CATGTGTAACAG -3'; and (v) 5'- CATGTGCAACAG - 3'. In particularly suitable embodiments the polypeptide is or comprises a zinc finger peptide (ZFP).

This aspect of the invention may further comprise detecting a background or basal level expression of the reporter-selection gene before the cell is exposed to the polypeptide expression vector, and expression of the reporter-selection gene above the background or basal expression level is indicative that the polypeptide has bound to the bait sequence element in the cell. Reporter-selection gene expression Inhibitor molecules may be used to suppress background expression levels, in which case any detectable expression of the reporter-selection gene product may indicate that the polypeptide has bound to the bait sequence element and caused activation of gene expression.

It will be appreciated that polypeptides, ZFPs and ZFNs of the invention may be further derivatised or conjugated to additional molecules, and that such derivatives and conjugates fall within the scope of the invention.

It should also be appreciated that, unless otherwise stated, optional features of one of more aspects of the invention may be incorporated into any other aspect of the invention and all such combinations are envisaged and considered to be disclosed herein.

All references cited herein are incorporated by reference in their entirety. Unless otherwise defined, all technical and scientific terms used herein have the same meaning as commonly understood by one of ordinary skill in the art to which this invention belongs.

### Brief Description of the Drawings

The invention is further illustrated by the accompanying drawings in which:
**Figure 1** Illustrates the targeting of the human p53 gene by ZFPs and corresponding ZFNs of the invention. (**A**) Mutation hotspots in somatic cancers from the IARC TP53 Mutation Database R13. (**B**) Nucleic acid sequence of a segment of the human p53 gene from exon 5 to exon 8 (Human Genome: NW_001838403). The sequences of exons 5, 6, 7 and 8 are highlighted in grey and contain nearly all mutation hotspots (underlined black; codon number in brackets). Binding sequences for ZFNs are highlighted in black with white letters. (**C**) Canonical model of ZFNs z771 and z1166 against two target sites in the p53 gene. Arrows indicate possible amino acid-base contacts. Zinc finger α-helix sequences, involved in DNA recognition, are indicated (F1 = Finger 1, etc.).

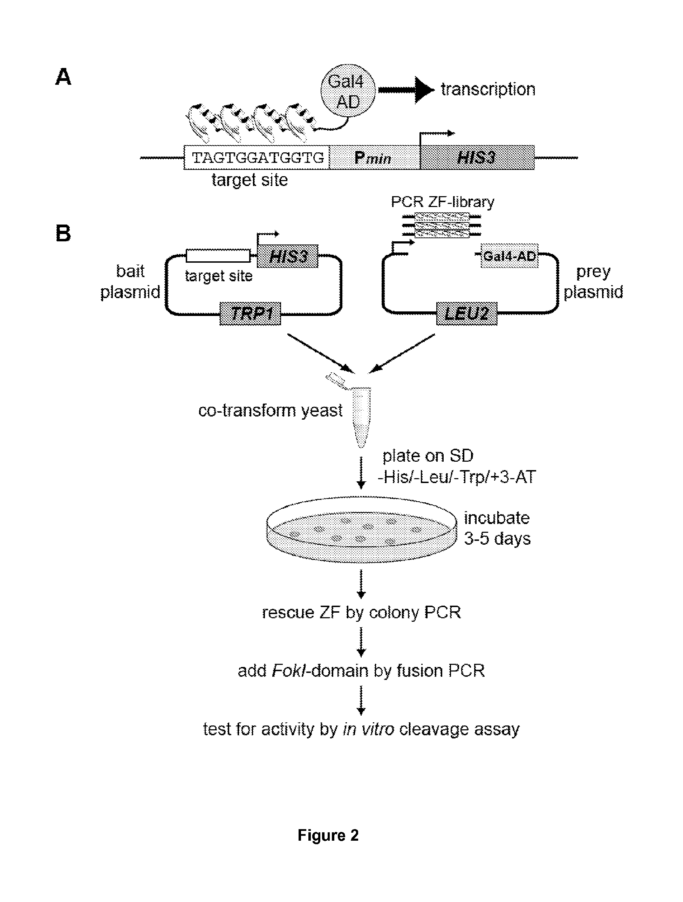

**Figure 2** Schematic illustration of a yeast one-hybrid based selection for ZFPs. (**A**) The ZFP target site is cloned upstream of a minimal promoter (Pₘᵢₙ) and the HIS3 reporter in the bait plasmid. Any interaction between a ZFP activation domain (AD) fusion protein (ZFP:Gal4-AD) and the target sequence stimulates transcription of HIS3 allowing selection of cells on His-selective medium. (**B**) The ZFP-library:Gal4-AD fusion construct is generated *in vivo* by yeast recombination of a PCR-generated 4-finger library cassette with the linearised prey plasmid. According to the illustrated scheme, bait plasmid, linearised prey plasmid and library PCR cassette are co-transformed into yeast. After incubation for 3 to 5 days, expression from the HIS3 reporter is detected in colonies that are able to grow on a selection medium that lacks histidine and contains 3-AT (see Examples). ZFPs from positive clones are rescued by colony-PCR and can be fused to a *Fok*l-nuclease domain for further testing, e.g. by an *in vitro* cleavage assay.

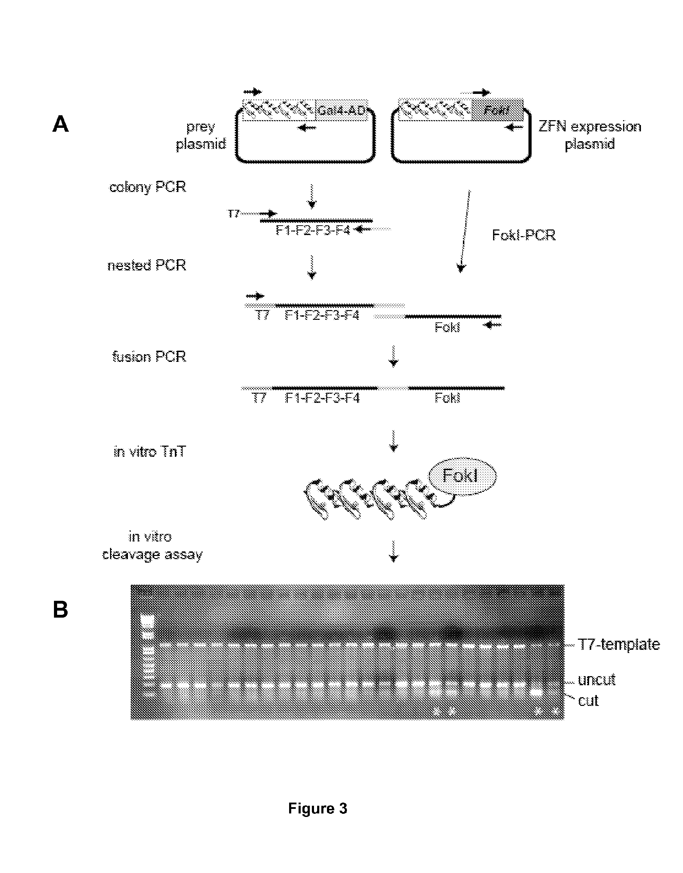

**Figure 3** Schematic illustration of an experimental scheme to validate yeast one-hybrid-selected ZFN clones. (**A**) Assembly PCR was used to recover zinc finger sequences from positive yeast colonies and to fuse them to a *Fok*l nuclease domain and T7 promoter for *in vitro* transcription-translation expression (TnT). (**B**) Selected clones were tested for cleavage activity *in vitro* against DNA template containing the appropriate ZFN palindromic binding sequence (see Examples 3 and 4). ZFN clones marked with an asterisk showed cleavage activity against the appropriate target DNA sequence and were subcloned for further tests.

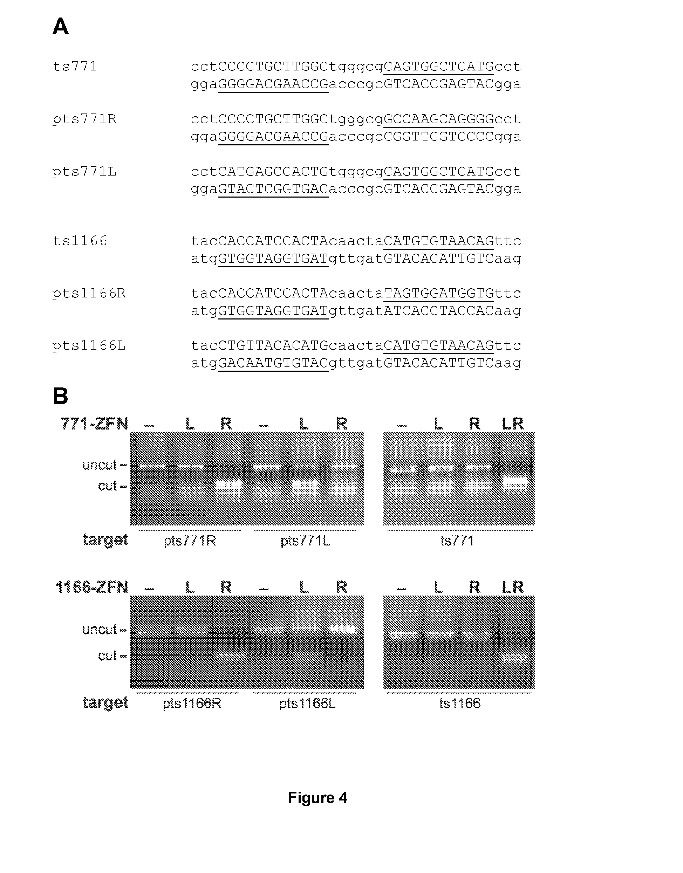

**Figure 4** *In vitro* cleavage of DNA target sites by p53 ZFNs of the invention. (**A**) Polynucleotide sequences comprising palindromic target sites (pts) or heterodimer target sites (ts) for targeting by homodimeric or heterodimeric z771 and z1166 ZFNs. Both strands of DNA are shown with the top strand written 5' to 3' and the bottom strand written 3' to 5'. The primary strand of each 12bp target site, as bound by ZFNs, is underlined. (**B**) Analysis of homodimer and heterodimer cleavage reactions. *In vitro* expressed ZFNs were incubated with a linear target DNA substrate (uncut) and cleavage products (cut) were analysed by agarose gel electrophoresis. Cleavage of the target DNA results in two DNA molecules of approximately the same size.

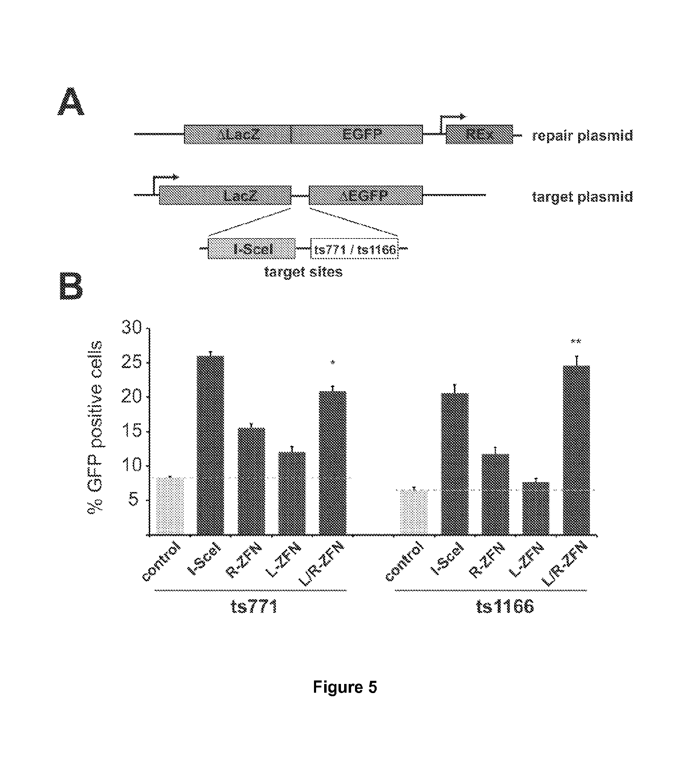

**Figure 5** Episomal gene repair assay in HEK293T cells. (**A**) Schematic representation of the experimental setup. (**B**) Cells transfected with repair plasmid, target plasmid and ZFN expression vectors were analysed after 48 hrs by flow cytometry. The bar graph displays the fraction of EGFP-positive cells normalised for transfection efficiency. Statistically significant increases in homologous recombination (HR), compared to non-induced HR control (dashed grey line) are indicated with asterisks (** = p < 0.0001 and * = p < 0.002).

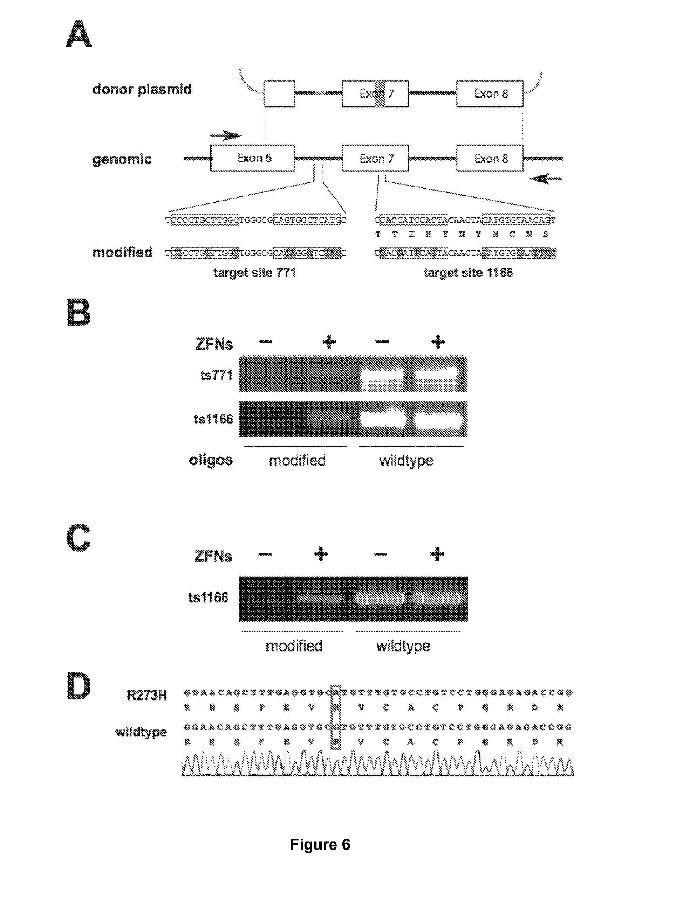

**Figure 6** Targeted genomic recombination of p53 gene with p53 donor plasmid. (**A**) Schematic illustration of the genomic p53 locus and the donor plasmid with modified DNA sequences at z771 and z1166 target sites ('barcodes'; highlighted with grey boxes). Barcodes allow selective PCR but do not change the p53 protein sequence. Black arrows indicate primers for genomic PCR that hybridise to the chromosomal p53 locus outside of the region corresponding to donor sequence. (**B**) Targeted donor plasmid recombination into the p53 z771 and z1166 loci in HEK293T cells. ZFN-induced recombination is shown by semi-nested PCR on purified external genomic PCR template, with forward primers that discriminate between wild-type and barcoded sequences. (**C**) Targeted donor recombination at the p53 z1166 locus in human SF268 glioblastoma cells. (**D**) Sequencing output demonstrating restoration of p53 wild-type sequence at codon 273 in ZFN-treated SF268 glioblastoma cells.

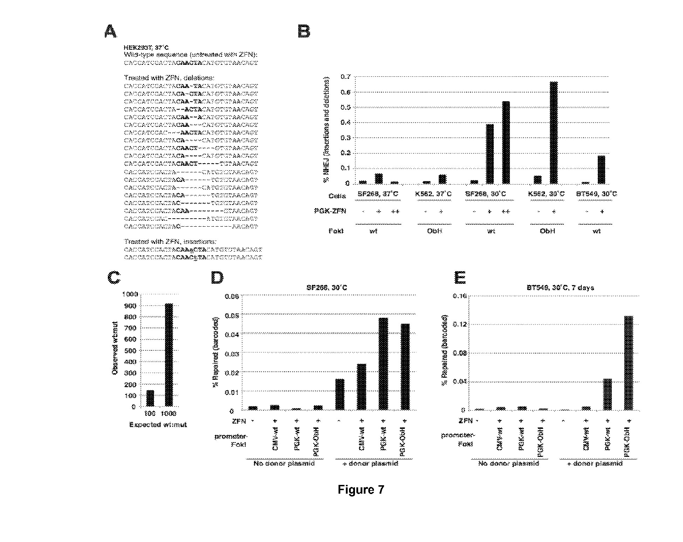

**Figure 7** Solexa-Illumina deep sequencing of ZFN-treated cells. (**A**) Short 31 bp reads detect ZFN-induced non-homologous end-joining (NHEJ) events in HEK293T cells. The *Fok*l cutting region (CAACTA) is indicated in bold, deletions are shown with "-" and insertions are underlined. (**B**) 104bp-read protocol: quantifying the rate of insertions and deletions induced by ZFN under a PGK promoter. SF268, K562 and BT549 cells were kept at 37°C or subjected to transient cold shock to increase NHEJ (30°C). ZFN plasmid amounts (0, 1 and 1.5 µg) are indicated by -, + and ++, respectively. Obligate heterodimer *Fok*l mutants (Miller et al. (2007), Nat. Biotechnol., 25: 778-785) are indicated where used (ObH). (**C**) Controls using wt to mutant plasmids at ratios of 100:1 and 1000:1. The proportion of wt:mutant sequence in the final samples is illustrated. (**D**) Quantifying the rate of insertion of a barcoded (wt coding sequence) donor plasmid into the genomic p53 locus in SF268 cells. CMV and PGK promoters were also tested in combination with wt or ObH *Fok*l nuclease domains. (**E**) A similar gene repair experiment in BT549 cells. Genomic DNA was collected 7 days after ZFN and donor plasmid transfection to reduce background.

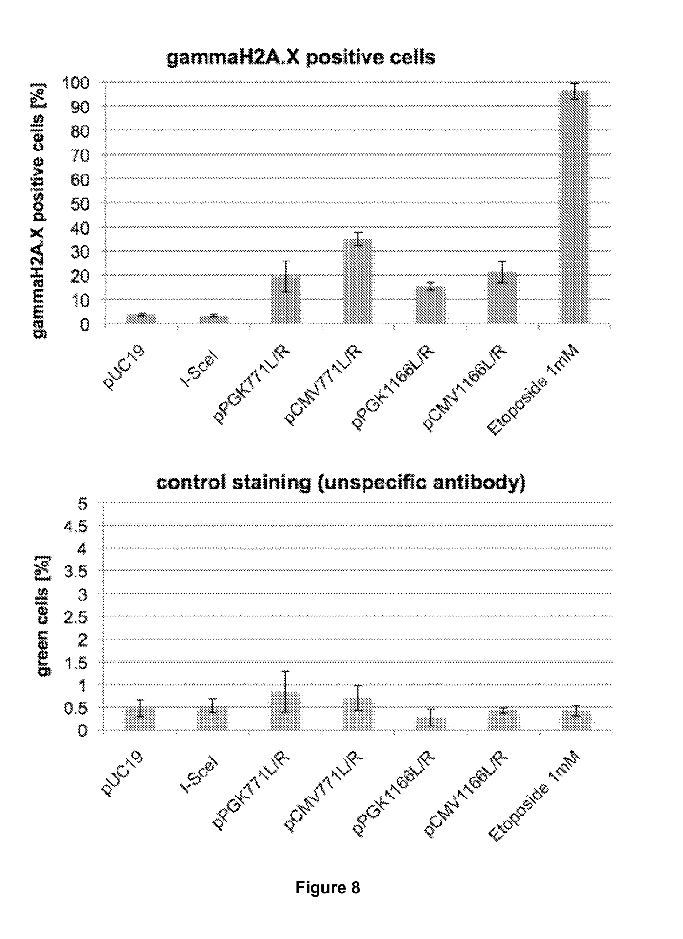

**Figure 8** ZFN-associated toxicity assay. Flow cytometry data for HEK293T cells transfected with the indicated constructs and stained with antibodies against gH2A.X (top) or unspecific staining-control antibodies (bottom). The columns show the percentage of gH2A.X-positive cells normalised for transfection efficiency. Etoposide is a toxic positive control.

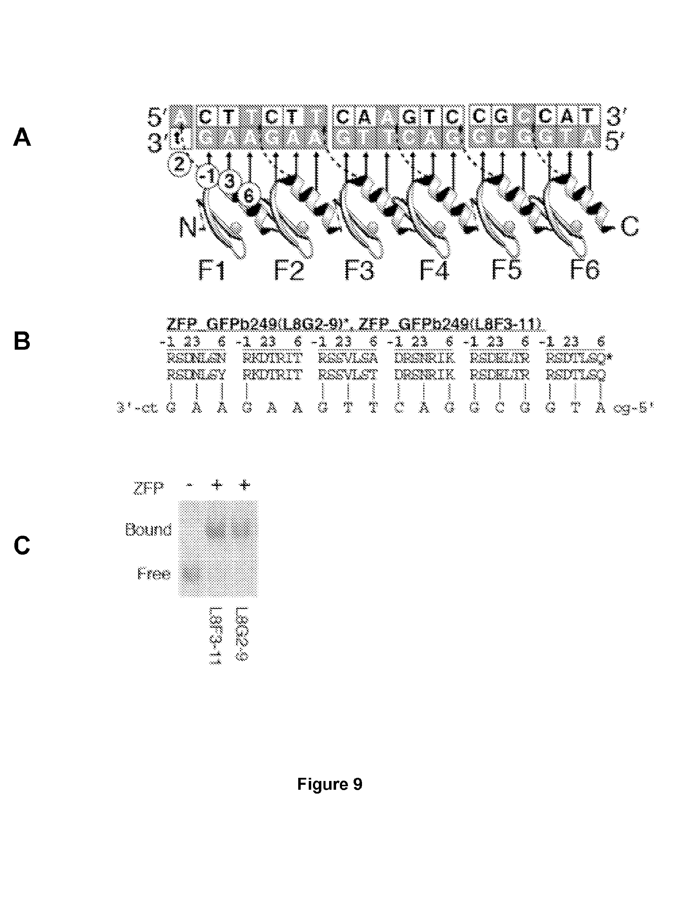

**Figure 9** Six-finger peptides engineered and selected to bind an EGFP gene sequence using the yeast one-hybrid assay of the invention. (**A**) Schematic illustration of a canonical binding model for a six-finger ZFP binding to a selected EGFP DNA target sequence. Primary DNA contacts (arrows) are from four positions on each zinc finger α-helix (circled, -1, 2, 3 and 6 in Finger 1). (**B**) Amino acid sequences of the zinc finger α-helices of two six-finger ZFPs selected against the EGFP target site (ZFP_GFPb249(L8G2-9)*, top row; and ZFP_GFPb249(L8F3-11), bottom row). The α-helices are shown aligned against the DNA bases they are expected to contact according to the canonical model. Note that ZFPs (N-C) bind antiparallel to their primary contacting DNA strand (3'-5'). (**C**) Gel shift assays on ZFPs expressed *in vitro* from T7-promoter PCR products show that the two ZFPs bind their DNA targets on an double-stranded oligonucleotide having the target sequence.

### Detailed Description of the Invention

Described herein are compositions (e.g. polypeptides, polynucleotides, therapeutics) and methods for the targeting of specific nucleic acid sequences. Beneficially, the compositions and methods are for the targeting and cleavage of nucleic acid sequences, e.g. gene sequences, especially in cellular chromatin. The methods and compositions of the invention may substitute or mutate the targeted sequences (and potentially hundreds of bases pairs around the targeted sequences) through homologous recombination. Alternatively, targeted nucleic acid sequences may be altered through non-homologous end joining (NHEJ).

The compositions useful in accordance with the invention are generally zinc finger peptides (ZFPs) and particularly zinc finger nucleases (ZFNs), which can be engineered to bind target nucleic acid sequences with good sequence specificity. A ZFN includes a ZFP and a nuclease (or cleavage) domain, which may be active as a single peptide, or may only be functional as a dimer. Each zinc finger domain binds approximately 3bp of nucleic acid sequence, and therefore, a ZFP having multiple zinc finger domains (sometimes termed a poly-ZFP) is used to target longer nucleic acid sequences. Suitable ZFPs for use in targeting genomic sequences typically comprise at least four zinc finger domains, although fewer domains may be used, for example, when dimers of ZFPs or ZFNs are used for targeting (and optionally cleaving) a gene sequence.

Also described are novel methods for selecting and/or maturing (optimising / improving) ZFP domains for binding to desired target sequences. The methods of the invention are particularly beneficial for selecting or maturing poly-ZFPs of 4 or more (e.g. 4, 5 or 6) zinc finger domains. Such long arrays of zinc finger domains can be particularly difficult to select or mature using previously known selection and maturation techniques. The novel method is suitably based on the yeast one-hybrid (or Y1H) technique; and this method may be useful for selecting and/or maturing a range of nucleic acid binding molecules in addition to ZFPs.

### General

Unless defined otherwise, all technical and scientific terms used herein have the same meaning as commonly understood by one of ordinary skill in the art (e.g. in cell culture, molecular genetics, nucleic acid chemistry and biochemistry).

Unless otherwise indicated, the practice of the present invention employs conventional techniques of chemistry, molecular biology, microbiology, recombinant DNA technology, chemical methods, pharmaceutical formulations and delivery and treatment of patients, which are within the capabilities of a person of ordinary skill in the art. Such techniques are also explained in the literature, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N. Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridisation: Principles and Practice, Oxford University Press; M. J. Gait (Editor), 1984, Oligonucleotide Synthesis: A Practical Approach, IRL Press; and D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

In order to assist with the understanding of the invention several terms are defined herein.

The term 'amino acid' in the context of the present invention is used in its broadest sense and is meant to include naturally occurring L α-amino acids or residues. The commonly used one and three letter abbreviations for naturally occurring amino acids are used herein: A=Ala; C=Cys; D=Asp; E=Glu; F=Phe; G=Gly; H=His; I=Ile; K=Lys; L=Leu; M=Met; N=Asn; P=Pro; Q=Gln; R=Arg; S=Ser; T=Thr; V=Val; W=Trp; and Y=Tyr (Lehninger, A. L., (1975) Biochemistry, 2d ed., pp. 71-92, Worth Publishers, New York). The general term 'amino acid' may further include D-amino acids, retro-inverso amino acids as well as chemically modified amino acids such as amino acid analogues, naturally occurring amino acids that are not usually incorporated into proteins such as norleucine, and chemically synthesised compounds having properties known in the art to be characteristic of an amino acid, such as β-amino acids. For example, analogues or mimetics of phenylalanine or proline, which allow the same conformational restriction of the peptide compounds as do natural Phe or Pro, are included within the broad definition of amino acid. Such analogues and mimetics are referred to herein as 'functional equivalents' of the respective amino acid. Other examples of amino acids are listed by Roberts and Vellaccio, The Peptides: Analysis, Synthesis, Biology, Gross and Meiehofer, eds., Vol. 5 p. 341, Academic Press, Inc., N.Y. 1983, which is incorporated herein by reference.

The term 'peptide' as used herein (e.g. in the context of a zinc finger peptide (ZFP) or zinc finger nuclease (ZFN) refers to a plurality of amino acids joined together in a linear or circular chain. The term oligopeptide is typically used to describe peptides having between 2 and about 50 or more amino acids. Peptides larger than about 50 amino acids are often referred to as polypeptides or proteins. For purposes of the present invention, however, the term 'peptide' is not limited to any particular number of amino acids, and is used interchangeably with the terms 'polypeptide' and 'protein'.

As used herein, the term 'zinc finger domain' refers to an individual 'finger', which comprises a ββα-fold stabilised by a zinc ion (as described elsewhere herein). Each zinc finger domain typically includes approximately 30 amino acids. The term 'domain' (or 'module'), according to its ordinary usage in the art, refers to a discrete continuous part of the amino acid sequence of a polypeptide that can be equated with a particular function. Zinc finger domains are largely structurally independent and may retain their structure and function in different environments. Typically, a zinc finger domain binds a triplet or (overlapping) quadruplet nucleotide sequence. Adjacent zinc finger domains arranged in tandem are joined together by linker sequences. A ZFP or ZFN of the invention is composed of a plurality of zinc finger domains, which in combination do not exist in nature. Therefore, they may be considered to be artificial, synthetic or engineered ZFPs or ZFNs.

A 'zinc finger peptide' (or ZFP) refers to a polypeptide that comprises one or more zinc finger domain. Generally, a ZFP of the invention is a peptide or portion of a larger polypeptide that performs the function of recognising / targeting / binding a desired nucleic acid sequence. Meanwhile, a 'zinc finger nuclease' (or ZFN) comprises a nuclease or cleavage domain (or portion) in addition to a ZFP domain (or portion). For expediency, when ZFP is referred to herein in connection with a particular aspect, embodiment or feature, the term should be taken to include ZFN, unless it is apparent that a ZFN in the context of that aspect, embodiment or feature would not be intended or appropriate. A single ZFN may or may not have nucleic acid cleaving activity in isolation from other peptides and particularly other ZFNs. Thus, the nuclease domain of a ZFN may be an active nuclease domain or an inactive portion or fragment of a nuclease domain. Nuclease domains that are only functional as dimers (or multimers) may be considered to be partial or 'half' nuclease or cleavage domains. Nuclease domains that are only functional as dimers may be engineered so as to function as homo- and heterodimers, homodimers only, or heterodimers only, depending on requirements. Preferred for use herein are nuclease domains that only function as heterodimers, i.e. in conjunction with another (perhaps closely related but) different nuclease domain. The skilled person understands how the peptide sequence of a zinc finger peptide or nuclease or effector may be modified by changing (e.g. substituting) one or more amino acid residues, for example, in backbone / framework positions, without substantially altering the function and activity of the zinc finger-containing peptide; and these modified zinc finger-containing peptides are encompassed in the scope of the invention. Such peptides may have at least 90%, at least 95%, at least 98% or at least 99% identity to the peptide sequences and the SEQ ID NOs disclosed herein.

The terms 'nucleic acid', 'polynucleotide', and 'oligonucleotide' are used interchangeably and refer to a deoxyribonucleotide (DNA) or ribonucleotide (RNA) polymer, in linear or circular conformation, and in either single- or double-stranded form. For the purposes of the present invention such DNA or RNA polymers may include natural nucleotides, non-natural or synthetic nucleotides, and mixtures thereof. Non-natural nucleotides may include analogues of natural nucleotides, as well as nucleotides that are modified in the base, sugar and/or phosphate moieties (e.g. phosphorothioate backbones). Examples of modified nucleic acids are PNAs and morpholino nucleic acids. Generally an analogue of a particular nucleotide has the same base-pairing specificity, i.e. an analogue of G will base-pair with C. For the purposes of the invention, these terms are not to be considered limiting with respect to the length of a polymer. The skilled person understands how the nucleic acid sequence of a polynucleotide may be modified by changing (e.g. substituting) one or more nucleic acid residues in the polynucleotide without substantially altering the function and activity of the encoded zinc finger-containing peptide (non-conservative mutations); and in some cases without even altering the encoded amino acid sequence (conservative mutations). Such polynucleotides are encompassed within the embodiments of the invention, and may have at least 80%, at least 99%, at least 95% or at least 98% identity to the nucleic acid sequences and the SEQ ID NOs disclosed herein.

A 'gene', as used herein, is the segment of nucleic acid (typically DNA) that is involved in producing a polypeptide or ribonucleic acid gene product. It includes regions preceding and following the coding region (leader and trailer) as well as intervening sequences (introns) between individual coding segments (exons). Conveniently, this term also includes the necessary control sequences for gene expression (e.g. enhancers, silencers, promoters, terminators etc.), which may be adjacent to or distant to the relevant coding sequence, as well as the coding and/or transcribed regions encoding the gene product. The phrase 'genomic sequence' therefore, includes genes and fragments or partial gene sequences. It may refer to non-coding and/or coding gene sequences. Such genomic sequences may be within a gene (e.g. in its natural or cellular context), or it may be isolated or extracted from its corresponding gene, such as an isolated nucleic acid molecule, vector or plasmid. Genomic sequences include those present in chromosomes whether natural or artificial, and whether in the nucleus or other organelles (e.g., mitochondria and chloroplasts). The genomic sequences may be animal (e.g. mammals, such as humans), or may be viral, parasitic, bacterial or fungal (e.g. yeast). Furthermore, the sequences that are to be targeted by the ZFPs or ZFNs of the invention can be normal (i.e., wild-type) or mutant (or abnormal). Mutant sequences may include point mutations, insertions, deletions, translocations and rearrangements. Any genomic sequence may be targeted by the ZFPs and ZFNs of the invention, although a particularly suitable genomic sequence is the p53 gene, or a portion or fragment (isolated or endogenous) thereof. Still more suitably the sequence is human (or derived from a human gene sequence).

A nucleic acid 'target', 'target site' or 'target sequence', as used herein, is a nucleic acid sequence to which a ZFP or ZFN of the invention will bind, provided that conditions of the binding interaction are not prohibitive. A target site may be a nucleic acid molecule or a portion of a larger polynucleotide. Particularly suitable target sites comprise mutated (i.e. abnormal) genetic sequences. In accordance with the invention, a target sequence for a ZFP of the invention may comprise a single contiguous nucleic acid sequence, or more than one non-contiguous nucleic acid sequence (e.g. two separate contiguous sequences, each representing a partial target site), which are interspersed by one or more intervening nucleotide or sequence of nucleotides. Where the target sequences are partial, they may be bound by the same or different ZFPs. Where the target sequence is bound by two ZFPs (e.g. as for dimeric ZFNs of the invention), the partial target sites may be on opposite strands of a double-stranded nucleic acid molecule. The above terms may also be substituted or supplemented with the terms 'binding site', 'binding sequence', 'recognition site' or 'recognition sequence', which are used interchangeably.

As used herein, 'binding' refers to a non-covalent interaction between macromolecules (e.g. between a ZFP and a nucleic acid target site). In some cases binding will be sequence-specific, such as between one or more specific nucleotides (or base pairs) and one or more specific amino acids. It will be appreciated, however, that not all components of a binding interaction need be sequence-specific (e.g. non-covalent interactions with phosphate residues in a DNA backbone). Binding interactions between a nucleic acid sequence and a ZFP of the invention may be characterised by binding affinity and/or dissociation constant (Kd). A suitable dissociation constant for a ZFP of the invention binding to its target site may be in the order of 1 µM or lower, 1 nM or lower, or 1 pM or lower. 'Affinity' refers to the strength of binding, such that increased binding affinity correlates with a lower Kd value. ZFPs of the invention may have DNA-binding activity, RNA-binding activity, and/or even protein-binding activity. Preferably ZFPs of the invention are designed or selected to have sequence specific dsDNA-binding activity. Preferably, the target site for a particular ZFP is a sequence to which the zinc finger concerned is capable of nucleotide-specific binding. It will be appreciated, however, that depending on the amino acid sequence of a ZFP it may bind to or recognise more than one target sequence (possibly with different binding strengths), although typically one sequence will be bound in preference to any other recognised sequences, depending on the relative specificity of the individual non-covalent interactions. Generally, specific binding is preferably achieved with a dissociation constant (Kd) of 100 nM or lower, 10 nM or lower, or 1 nM or lower. Preferably, a ZFP of the invention binds to a specific target sequence with a dissociation constant of 100 pM or lower; such as 10 pM or lower or 1 pM or lower.

By 'non-target' it is meant that the nucleic acid sequence concerned is not appreciably bound (and/or cleaved) by the relevant ZFP (and/or ZFN). In some cases it may be convenient to consider that where a ZFP of the invention has a known preferred or intended sequence-specific target sequence, all other nucleic acid sequences would then be 'non-target'. Preferably, and from a practical perspective, it can be convenient to define an interaction between a non-target sequence and a particular ZFP as being sub-physiological (i.e. the ZFP or ZFN is not capable of binding or creating a physiological response under physiological target sequence / ZFP concentrations). For example, if any binding can be measured between a ZFP and a non-target sequence, the dissociation constant (Kd) is typically weaker than 1 µM, such as 10 µM or weaker, 100 µM or weaker, or at least 1 mM.

While the ZFNs of the invention are particularly useful for cleaving target nucleic acid sequences and optionally promoting a change in the nucleic acid sequence (e.g. by non-homologous end joining (NHEJ) or homologous recombination), other engineered ZFPs of the invention may have other uses, which may be determined according on the modulation or effector domain that is associated with the ZFP (e.g. linked, fused, bound, coupled), in order to cause a desired effect once bound to the target nucleic acid. Thus, as used herein the term 'modulation', in relation to the expression of a gene refers to a change in the normal (or mutant) gene's previous activity. Modulation includes both activation (i.e. increase in activity or expression level) and repression or inhibition of gene activity.

Zinc finger domains can be 'engineered' to bind to a predetermined nucleotide sequence, i.e. a target sequence. Methods for engineering zinc finger domains of ZFPs include rational design as well as non-rational methods (e.g. selection from libraries). Engineered ZFPs of the invention do not occur in nature in exactly the same sequence; although an engineered ZFP may be designed to bind to the same target / recognition sequence as a natural ZFP.

The term 'library' is used according to its common usage in the art, to denote a collection of different polypeptides or a collection of nucleic acids encoding different polypeptides. The rationally designed or otherwise engineered ZFPs of the invention may be useful for generating libraries for the selection of further alternative or improved ZFPs for targeting the same or similar sequence. In some embodiments, therefore, rationally designed ZFPs are used as the basis for the generation of ZFP libraries, which are then screened for a desirable activity. Such a library is not 'naïve', because it is based on a ZFP framework having an assumed or predetermined preferred target / recognition sequence. As used herein, a 'naïve' library refers to a collection of different ZFPs or corresponding encoding nucleic acids that has not been predetermined or selected to have a particular preferred target binding site. The selection method of the invention may be suitable for the selection of a polypeptide with a preferred target sequence from naïve or non-naïve libraries.

### Zinc Finger Peptides

The present invention relates to non-naturally occurring (engineered) ZFPs for binding to any desired nucleic acid sequence, and their use as therapeutic molecules; for example, in the treatment of genetic disorders of humans or other mammals, and particularly cancers. The invention further provides for the use of ZFNs to bind and cleave target nucleic acid sequences, such as genomic sequence, and particularly p53 gene sequences. Suitably, the ZFNs of the invention bind and cleave mutated p53 gene sequences and promote repair or alteration of that genomic sequence. Most suitably, mutated gene sequences are replaced with wild-type genomic sequences, e.g. by allowing (in conjunction with a suitable donor nucleic acid) homologous recombination.

A zinc finger is a relatively small polypeptide domain comprising approximately 30 amino acids, which fold to form an α-helix adjacent an anti-parallel β-sheet (known as a ββα-fold). The fold is stabilised by the co-ordination of a zinc ion between four largely invariant (depending on zinc finger type) Cys and/or His residues, as described further below. Natural zinc finger domains have been well studied and described in the literature, see for example, Miller et al., (1985) EMBO J. 4: 1609-1614; Berg (1988) Proc. Natl. Acad. Sci. USA 85: 99-102; and Lee et al., (1989) Science 245: 635-637.

There are a number of natural zinc finger frameworks known in the art, and any of these frameworks may be suitable for the ZFPs of the invention. In general, a natural zinc finger framework has the sequence, Formula 1: X₀₋₂ C X₁₋₅ C X₉₋₁₄ H X₃₋₆ ^{H}/_{C}. The majority of the amino acid side chains in a zinc finger domain that are important for nucleic acid base recognition (e.g. in dsDNA) are located on or adjacent the α-helix of the finger. Conveniently, therefore, in order to readily identify the nucleic acid binding residues of a zinc finger domain, the amino acid positions in a zinc finger domain are numbered from the first residue in the α-helix, which is given the number (+)1. Thus, "-1" refers to the residue in the framework structure immediately preceding the first residue of the α-helix, and the helix is generally considered to end at the final zinc-coordinating Cys or His residue in Formula 1 above, which is typically position +11. The first invariant histidine residue that coordinates the zinc ion is position (+)7 of the zinc finger domain. As used herein, residues referred to as "++" are located in the immediately adjacent (C-terminal) zinc finger domain. Generally, nucleic acid (sequence-specific) recognition by a zinc finger module is achieved primarily by the amino acid side chains at positions -1, +2, +3 and +6; although other amino acid positions may sometimes contribute to binding between the zinc finger and the target molecule, and the contact from the +2 position is typically a cross-strand contact to a residue complementary to that recognised by the +6 position of the adjacent N-terminal domain, where present. Hence, the sequence of a zinc finger domain from -1 to (+)6 (i.e. residues -1, 1, 2, 3, 4, 5 and 6) is typically termed the 'recognition sequence'. Thus, a suitable zinc finger domain framework for use in accordance with the invention can be defined by Formula 2: X₀₋₂ C X₁₋₅ C X₂₋₇ X⁻¹ X⁺¹ X⁺² X⁺³ X⁺⁴ X⁺⁵ X⁺⁶ H X₃₋₆ ^{H}/_{C} where X is any amino acid, the numbers in subscript indicate the possible numbers of residues represented by X, and the numbers in superscript indicate the position of the amino acid in the α-helix.

A zinc finger domain recognises and binds to a nucleic acid triplet, or an overlapping quadruplet in a double-stranded DNA target sequence (as explained below). However, zinc fingers are also known to bind RNA and proteins (Clemens, K. R. et al. (1993) Science 260: 530-533; Bogenhagen, D. F. (1993) Mol. Cell. Biol. 13: 5149-5158; Searles, M. A. et al. (2000) J. Mol. Biol. 301: 47-60; Mackay, J. P. & Crossley, M. (1998) Trends Biochem. Sci. 23: 1-4).

Zinc finger proteins generally contain strings or chains of zinc finger domains (or modules). Thus, a natural zinc finger protein may include 2 or more zinc finger domains, which may be directly adjacent one another (i.e. separated by a short (canonical) linker sequence), or may be separated by longer, flexible or structured polypeptide sequences. Directly adjacent zinc finger domains are expected to bind to contiguous nucleic acid sequences, i.e. to adjacent trinucleotides / triplets. In some cases, cross-binding may also occur between adjacent zinc fingers and their respective target triplets, which helps to strengthen or enhance the recognition of the target sequence, and leads to the binding of overlapping quadruplet sequences (Isalan et al., (1997) Proc. Natl. Acad. Sci. USA, 94: 5617-5621). By comparison, distant zinc finger domains within the same protein may recognise (or bind to) non-contiguous nucleic acid sequences or even to different molecules (e.g. protein rather than nucleic acid).

When binding to a nucleic acid sequence, a zinc finger domain generally interacts mainly with one strand of a double stranded nucleic acid molecule (the primary strand or sequence). However, there can be subsidiary interactions between amino acids of a zinc finger domain and the complementary (or secondary) strand of the double-stranded nucleic acid molecule. On binding to dsDNA, the α-helix of the zinc finger domain almost invariably lies within the major groove and aligns anti-parallel to the target nucleic acid strand. Accordingly, the primary nucleic acid sequence is arranged 3' to 5' in order to correspond with the N-terminal to C-terminal sequence of the ZFP. Since nucleic acid sequences are conventionally written 5' to 3', and amino acid sequences N-terminus to C-terminus, when a target nucleic acid sequence and a ZFP sequence are aligned according to convention, the primary interaction of the ZFP is with the complementary (or minus) strand of the nucleic acid sequence, since it is this strand which is aligned 3' to 5' (see e.g. Figures 1C and 9A). These conventions are followed in the nomenclature used herein.

Zinc finger domains of ZFPs of the invention may be based on framework sequences according to Formula 1 or Formula 2 above. Such ZFPs are non-natural and suitably contain 3 or more, for example, 4, 5, 6, 7, 8, 9, 10, 11, 12 or more (e.g. up to approximately 18 or 24) zinc finger domains arranged adjacent one another in tandem. Such peptides may also be referred to as 'poly-ZFPs'. Particularly beneficial ZFPs of the invention include at least 4, at least 6, at least 8 or at least 12 zinc finger domains. Advantageously, the ZFPs and ZFNs of the invention comprise 4 zinc finger domains arranged in pairs of two-finger sub-domains; or 6 zinc finger domains arranged in pairs of three-finger sub-domains or in groups of three two-finger sub-domains. The individual ZFPs of the invention may bind to non-contiguous or contiguous nucleic acid binding sites. When targeted to non-contiguous binding sites, each sub-site (or half-site where there are only two non-contiguous sequences) is suitably approximately 6 bases long (e.g. in the case of pairs or triplets of two-finger domains), but may alternatively be approximately 9 bases long (when targeted by three-finger subdomains) or 12 bases long (when targeted by two- or four-finger subdomains).

In such (poly-)ZFPs of the present invention, adjacent zinc finger domains are joined to one another by 'linker sequences' that may be canonical, canonical-like, flexible or structured, as described, for example, in WO 01/53480 (Moore et al., (2001) Proc. Natl. Acad. Sci. USA 98: 1437-1441). In a tandem array of zinc finger domains, the linker sequence is the amino acid sequence that lies between the last residue of the α-helix in an N-terminal zinc finger and the first residue of the β-sheet in the next (i.e. C-terminal adjacent) zinc finger. For the purposes of the present invention, the last amino acid in the α-helix of a zinc finger domain is considered to be the final zinc-coordinating histidine (or cysteine) residue, while the first amino acid of the following finger is generally a tyrosine, phenylalanine or other hydrophobic residue. Generally, a natural zinc finger linker sequence lacks secondary structure in the free form of the peptide. However, when the protein is bound to its target site a canonical linker is typically in an extended, linear conformation, and amino acid side chains within the linker may form local interactions with DNA.

When multiple ZFPs or ZFNs (e.g. pairs) are used together (e.g. simultaneously or in conjunction) so as to specifically target a desired nucleic acid sequence (e.g. a genomic sequence), such as in the case of ZFN dimers, each individual ZFP or ZFN binds to its own target sequence, which may be in the vicinity of, but not necessarily directly adjacent to the binding site of the other ZFP of its pair. These target sites may be on the same, or more suitably on different strands of a double-stranded DNA molecule; and may be from approximately 1 to 100bp apart (e.g. from approximately 2 to 50, from approximately 3 to 30, from approximately 4 to 20, or from approximately 5 to 10bps apart.

Target sites of appropriate length for use in accordance with the invention may be recognised by a single ZFP (or ZFN) having a sufficiently long array of zinc finger domains, or (as in preferred embodiments of the invention) may be recognised by pairs of ZFPs (or ZFNs) that each recognise a portion (e.g. half) of the total target site.

The binding affinity of a selected ZFP for its selected target sequence can be measured using techniques known to the person of skill in the art, such as surface plasmon resonance or biolayer interferometry. Biosensor approaches are reviewed by Rich et al. (2009) Anal. Biochem., 386:194-216. Alternatively, real-time binding assays between a ZFP and target site may be performed using biolayer interferometry with an Octet Red system (Fortebio, Menlo Park, CA).

ZFPs, ZFNs or other polypeptides of the invention may have µM or higher binding affinity for a target nucleic acid sequence. Suitably, a ZFP of the invention has nM or even sub-nM binding affinity for its specific target sequence; for example, 10⁻⁹ M, 10⁻¹⁰ M, 10⁻¹¹ M, or 10⁻¹² M or less. In some particularly advantageous embodiments the affinity of a ZFP of the invention for its target sequence may be in the pM range or below, for example, in the range of 10⁻¹³ M, 10⁻¹⁴ M, or 10⁻¹⁵ M or less.

### Engineering of Zinc Finger Peptides

For specific biological functionality and therapeutic use, particularly *in vivo* (e.g. in gene therapy and transgenic animals), it is desirable that the poly-ZFPs of the invention are able to target unique or virtually unique sites within any genome. For complex genomes, such as in humans, it is generally considered that an address of at least 16 bps is required to specify a potentially unique DNA sequence. Shorter DNA sequences have a significant probability of appearing several times in a genome, which increases the possibility of causing undesirable non-specific gene targeting and biological effects. Since individual zinc fingers generally bind to 3 consecutive nucleotides, 6 zinc finger domains with an 18bp binding site could, in theory, be used for the specific recognition of a unique target sequence within any genome. Accordingly, a great deal of research has been carried out into so-called 'designer transcription factors' for targeted gene regulation, which typically involve 4 or 6 zinc finger domains that may be arranged in tandem or in dimerisable groups (e.g. of 3- or 4-finger units).

The utility and binding specificity of ZFPs and ZFNs of the invention thus depends on artificially engineered multi-zinc finger domain arrays. ZFPs have proven to be extremely versatile scaffolds for engineering novel DNA-binding domains (e.g. Rebar & Pabo (1994) Science 263: 671-673; Jamieson et al., (1994) Biochemistry 33: 5689-5695; Choo & Klug (1994) Proc. Natl. Acad. Sci. USA. 91: 11163-11167; Choo et al., (1994) Nature 372: 642-645; Isalan & Choo (2000) J. Mol. Biol. 295: 471-477; and many others). Thus, there exists a large body of work on zinc finger engineering (reviewed in Pabo et al., (2001) Annu. Rev. Biochem. 70: 313-340). Briefly, the established engineering methods (amongst others) range from rational design (Sera & Uranga (2002) Biochemistry 41: 7074-7081); modular assembly with pre-made fingers (Beerli & Barbas (2002) Nat. Biotechnol. 20: 135-141; Bae et al, (2003) Nat. Biotechnol. 21: 275-280; Mandell & Barbas (2006) Nucleic Acids Res. 34: W516-523); overlapping finger assembly (Greisman & Pabo (1997) Science 275: 657-661; Isalan et al., (2001) Nat. Biotechnol. 19: 656-660); and bacterial-two hybrid (B2H: Hurt et al., (2003) Proc. Natl. Acad. Sci. USA 100: 12271-12276). Any such methods may be used in order to generate nucleic acid-binding zinc finger domain arrays for use in the ZFPs and ZFNs of the invention.

In addition, a recent development in zinc finger engineering is the emergence of two publicly available sources of zinc fingers: the academic Zinc Finger Consortium (ZFC; www.zincfingers.org); and the commercially available CompoZr, from Sigma Aldrich (Pearson (2008) Nature 455: 160-164.). In particular, the ZFC has provided a variety of open source tools for the community to employ (Wright et al., (2006) Nat. Protoc. 1: 1637-1652; Sander et al., (2007) Nucleic Acids Res. 35: W599-605; Maeder et al., (2008) Mol. Cell 31: 294-301; Fu et al., (2009) Nucleic Acids Res. 37: D279-283), and these systems and methods may also be used for generating nucleic acid binding domains for use in accordance with the invention.

In the present invention, novel extended ZFPs comprising at least 4 zinc finger domains have been created. Preferred ZFPs of the invention comprise 4, 5, 6, 8, 9 or 12 zinc finger domains. Extended arrays of 8 or more zinc finger domains may be constructed in accordance with the methods and teaching of our co-pending European patent application (EP 10187818.9).

As already described, adjacent zinc finger domains are joined together by linker sequences. In a natural zinc finger protein, threonine is often the first residue in the linker and proline is often the last residue of the linker. On the basis of sequence homology, the canonical natural linker sequence is considered to be -TGEKP-. However, natural linkers can vary greatly in terms of amino acid sequence and length. Therefore, a common consensus sequence based on natural linker sequences may be represented by -TG^{E}/_{Q}^{K}/_{R}P- (SEQ ID NO: 107), and this sequence is preferred for use as a 'canonical' (or 'canonical-like') linker for use in ZFPs of the invention. Thus, the ZFPs of the invention may comprise directly adjacent zinc finger domains having canonical (or canonical-like) linker sequences between adjacent zinc finger domains. Such groups of zinc fingers preferentially bind to contiguous nucleic acid sequences, such that, for example, a ZFP with four zinc finger domains is particularly suitable for binding to contiguous stretches of approximately 12 nucleic acid bases.

However, in extended zinc finger arrays of e.g. 4 or more zinc finger domains, it has been shown that it can be beneficial (e.g. for improving binding affinity and specificity) to periodically disrupt the canonical linker sequence, when used between adjacent zinc fingers in an array, by adding one or more amino acid residue (e.g. Gly and/or Ser), so as to create sub-arrays of (e.g. groups of 2 or 3) zinc finger domains within the array (Moore et al., (2001) Proc. Natl. Acad. Sci. USA 98: 1437-1441; and WO 01/53480). In some embodiments, therefore, one or more pairs of adjacent zinc finger domains may be separated by short flexible (canonical-like) linker sequences, e.g. of 6 or 7 amino acids. For example, the zinc finger peptides of the invention may comprise nucleic acid binding domains having 4 or 6 zinc finger domains arranged in sub-arrays of 2-finger or 3-finger units arranged in tandem. In this case, again, the poly-ZFPs (or ZFNs) typically bind to contiguous DNA target sites, as they do when separated by canonical linkers. Canonical-like linkers for use in accordance with the invention may be based on the sequence, -TG^{G}/_{S}^{E}/_{Q}^{K}/_{R}P- (SEQ ID NO: 108), for example: TGGERP, TGSERP, TGGQRP, TGSQRP, TGGEKP, TGSEKP, TGGQKP, or TGSQKP (SEQ ID NOs: 109 to 116, respectively). A particularly suitable canonical-like linker is TGSERP (SEQ ID NO: 110). Accordingly, suitable linker sequences for use in accordance with the invention - particularly when targeting a contiguous binding site - include canonical linker sequences of 5 amino acids, and optionally related canonical-like linker sequences of between 5 and 7 (and particularly 6 or 7) amino acids.

In some other embodiments, one or more pairs of adjacent zinc finger domains may be separated by longer flexible linker sequences, for example, comprising 8 or more amino acids, such as between 8 and 50 amino acids. Adjacent zinc finger domains separated by long flexible linkers have the capacity to bind to non-contiguous binding sites in addition to the capacity to bind to contiguous binding sites. The length of the flexible linker may influence the length of DNA that may lie between such non-contiguous binding sub-sites. Linkers of 8 amino acids include the sequences -TG(^{G}/_{S})₃^{E}/_{Q}^{K}/_{R}P-(SEQ ID NO: 117) and -T(^{G}/_{S})₃G^{E}/_{Q}^{K}/_{R}P- (SEQ ID NO: 118).

According to alternative nomenclature sometimes used in the art, since the threonine of the linker sequence may also be considered as the last amino acid of the α-helix, the linker sequence can alternatively be considered not to include the first T residue indicated above, in which case the linker may be considered to be one residue shorter, and such alternative linkers are encompassed herein.

The ZFPs or ZFNs of the invention are particularly suitable for binding to genomic DNA sequences, such as p53 gene sequences. Suitably, the target site for the ZFPs of the invention is unique or virtually unique within the genome to be targeted (e.g. the human genome). To improve the likelihood of finding a suitable target sequence in a particular genome and to reduce the possibility of non-specific (or off-target binding), it may be beneficial to employ pairs of ZFPs that target neighbouring binding sites (i.e. close but not necessarily directly adjacent or contiguous), and require dimerisation for activity. The binding site for each peptide of the invention may therefore be considered a sub-site of the full target sequence or site. Exemplary sequences of the p53 gene that may be targeted by the ZFPs of the invention are indicated below (5' to 3'), in which sub-sites bound by each one from a pair of ZFPs are shown underlined: TCCCCTGCTTGGCTGGGCGCAGTGGCTCATGC (human p53 sequence, SEQ ID NO: 36); CCACCATCCACTACAACTACATGTGTAACAGT (human p53 sequence, SEQ ID NO: 37); CCACCATCCACTACAACT**t**CATGTGcAACAG**c** (porcine p53 sequence, SEQ ID NO: 38); CCACCATCCACTACAACT**t**CATGTGTAACAG**c** (ovine p53 sequence, SEQ ID NO: 39); and CCACCATCCACTACAAGTACATGTGTAAtAG**c** (mouse p53 sequence, SEQ ID NO: 40). Sequence differences between human target site SEQ ID NO: 37 and non-human target sites SEQ ID NOs: 38 to 40 are identified by bold, lowercase letters. The individual members of each pair of ZFPs may bind the same or opposite strands of a double-stranded nucleic acid molecule. Therefore, one of the pair may bind a sequence shown underlined above, while the other may bind the complement of the adjacent sequence shown underlined above.

There are a number of natural zinc finger domain frameworks known in the art, and any of these frameworks may be suitable for use in the extended ZFP frameworks of the invention. In general, a natural zinc finger domain framework has the sequence, Formula 1: X₀₋₂ C X₁₋₅ C X₉₋₁₄ H X₃₋₆ ^{H}/_{C}; or Formula 2: X₀₋₂ C X₁₋₅ C X₂₋₇ X⁻¹ X⁺¹ X⁺² X⁺³ X⁺⁴ X⁺⁵ X⁺⁶ H X₃₋₆ ^{H}/_{C} where X is any amino acid, the numbers in subscript indicate the possible numbers of residues represented by X, and the numbers in superscript indicate the position of the amino acid in the α-helix. In some embodiments, the zinc finger motif may be represented by the general sequence, Formula 3: X₂ C X₂,₄ C X₁₂ H X₃,₄,₅ ^{H}/_{C;} or Formula 4: X₂ C X_{2,4} C X₅ X⁻¹ X⁺¹ X⁺² X⁺³ X⁺⁴ X⁺⁵ X⁺⁶ H X₃,₄,₅ ^{H}/_{C}. Still more preferably the zinc finger motif may be represented by the general sequence, Formula 5: X₂ C X₂ C X₁₂ H X₃ H; or Formula 6: X₂ C X₂ C X₅ X⁻¹ X⁺¹ X⁺² X⁺³ X⁺⁴ X⁺⁵ X⁺⁶ H X₃ H. Accordingly, the zinc finger domains of the ZFPs or ZFNs of the invention may be based on zinc finger domains of Formulas 1 to 6, or combinations of Formulas 1 to 6, joined together in an array using linker sequences, such as the linker sequences described herein. In these formulas, the fixed C and H residues coordinate the zinc ion to stabilise the zinc finger structure: the first H residue is position +7 of the α-helix. As already described, preferred positions for diversification within the zinc finger domain so as to engineer a desired nucleic acid binding specificity are those within or adjacent the α-helix, for example, positions -1, 2, 3 and 6.

Preferred engineered ZFPs of the invention have 4 or 6 zinc finger domains, and may comprise the groups of amino acid sequences SEQ ID NOs: 16 to 19; SEQ ID NOs: 20 to 23; SEQ ID NOs: 24 to 27 and/or SEQ ID NOs: 28 to 31. Thus, particularly beneficial ZFPs of the invention may comprise the amino acid sequences of SEQ ID NOs: 11 to 15 (see Table 6 and Table 14). Preferred ZFNs of the invention comprise the amino acids sequences of SEQ ID NO: 2, SEQ ID NO: 4, SEQ ID NO: 6, SEQ ID NO: 8 or SEQ ID NO: 10 (see Table 7B and Table 14).

The invention also encompasses nucleic acid molecules that encode the peptide sequences of the invention. Preferred ZFN encoding sequences comprise SEQ ID NOs: 1, 3, 5, 7 and 9 which encode the zinc finger peptides of SEQ ID NOs: 2, 4, 6, 8 and 10, respectively.

It will be appreciated that the ZFP and ZFN sequences of the invention may further include optional (N-terminal) leader sequences, such as: amino acids to aid expression (e.g. N-terminal Met-Ala dipeptide); purification tags (e.g. FLAG-tags); and localisation / targeting sequences (e.g. nuclear localisation sequences (NLS), such as PKKKRKV). Any or all of these may be comprised in the polypeptides of the invention. Also, the peptides may optionally include additional C-terminal sequences, such as: linker sequences for fusing ZFPs and ZFNs to effector molecules; and effector molecules. Other sequences may be employed for cloning purposes. The sequences of any N- or C-terminal sequences may be varied, typically without altering the binding activity of the zinc finger domains, and such variants are encompassed within the scope of the invention.

The invention also encompasses derivatives of the ZFPs and ZFNs of the invention, particularly derivatives of SEQ ID NOs: 11 to 15, 2, 4, 6, 8 and 10. In this regard, it will be appreciated that modifications, such as amino acid substitutions may be made at one or more positions in the peptide without adversely affecting its physical properties (such as binding specificity or affinity). By 'derivative' of a ZFP it is meant a peptide sequence that has the selected desired activity (e.g. binding affinity for a specified target sequence), but that further includes one or more mutations or modifications to the primary amino acid sequence. Thus, a derivative of the invention may have one or more (e.g. 1, 2, 3, 4, 5 or more) chemically modified amino acid side chains, such as pegylation, sialylation and glycosylation modifications. In addition or alternatively, a derivative may contain one or more (e.g. 1, 2, 3, 4, 5 or more) amino acid mutations, substitutions or deletions to the primary sequence of a selected ZFP, but without altering its nucleic acid recognition sequence. Accordingly, the invention encompasses the results of maturation experiments conducted on a selected ZFP. It also encompasses ZFP libraries which are derived from a particular designed or selected ZFP that can be used in maturation experiments to improve or change one or more characteristics of the initially identified peptide. By way of example, one or more amino acid residues of a selected zinc finger domain (particularly within the recognition sequence) may be randomly or specifically mutated (or substituted) using procedures known in the art (e.g. by modifying the encoding DNA or RNA sequence). The resultant library or population of derivatised peptides may further be selected - by any known method in the art - according to predetermined requirements: such as improved specificity against particular target sites; or improved drug properties (e.g. solubility, bioavailability, immunogenicity etc.). Peptides that have been selected according to such additional or improved characteristics and that display the activity for which the peptide was initially selected are derivatives of the ZFPs of the invention and also fall within the scope of the invention. A particularly preferred maturation method is a yeast one-hybrid assay, as described further below.

### Zinc Finger Peptide Modulators and Effectors

While the ZFPs of the invention may have useful biological properties in isolation, they can also be given useful biological functions by the addition of effector domains. Therefore, in some cases it is desirable to conjugate a ZFP of the invention to one or more non-zinc finger domain, thus creating chimeric or fusion ZFPs. It may also be desirable, in some instances, to create a multimer (e.g. a dimer), of a ZFP of the invention -for example, to bind more than one target sequence simultaneously.

Thus, having identified a desirable ZFP, an appropriate effector or functional group may then be attached, conjugated or fused to the ZFP. The resultant polypeptide comprising at least a zinc finger portion (of more than one zinc finger domain) and a non-zinc finger effector domain, portion or moiety may be termed a 'fusion', 'chimeric' or 'composite' ZFP. Beneficially, the ZFP will be linked to the other moiety via sites that do not interfere with the activity of either moiety.

A 'non-zinc finger domain' (or moiety) as used herein, refers to an entity that does not contain a zinc finger (ββα-) fold. Thus, non-zinc finger moieties include nucleic acids and other polymers, peptides, proteins, peptide nucleic acids (PNAs), antibodies, antibody fragments, and small molecules, amongst others.

Chimeric ZFPs or fusion proteins of the invention may be used to up- or down-regulate the expression of desired target genes, *in vitro* or *in vivo.* Thus, potential effector domains include transcriptional repressor domains, transcriptional activator domains, transcriptional insulator domains, chromatin remodelling, condensation or decondensation domains, nucleic acid or protein cleavage domains, dimerisation domains, enzymatic domains, signalling / targeting sequences or domains, or any other appropriate biologically functional domain. Suitably, transcriptional modulation domains, as well as their functional fragments. can be directly derived from a basal or regulated transcription factor such as, for example, transactivators, repressors, and proteins that bind to insulator or silencer sequences (see Choo & Klug (1995) Curr. Opin. Biotech. 6: 431-436; Choo & Klug (1997) Curr. Opin. Str. Biol. 7:117-125; and Goodrich et al. (1996) Cell 84: 825-830); or from receptors such as nuclear hormone receptors (Kumar & Thompson (1999) Steroids 64: 310-319); or co-activators and co-repressors (Ugai et al. (1999) J. Mol. Med. 77: 481-494). Specific transcriptional / gene activation domains for fusing to ZFPs of the invention include the VP64 domain (see Seipel et al., (1996) EMBO J. 11: 4961-4968) and the herpes simplex virus (HSV) VP16 domain (Hagmann et al. (1997) J. Virol. 71: 5952-5962; Sadowski et al. (1988) Nature 335: 563-564); and transactivation domain 1 and/or 2 of the p65 subunit of nuclear factor-κB (NFκB; Schmitz et al. (1995) J. Biol. Chem. 270: 15576-15584). A suitable transcriptional repression domain is the Kruppel-associated box (KRAB) domain, which is a powerful repressor of gene activity. Therefore, ZFPs of the invention may be fused to the KRAB repressor domain from the human Kox-1 protein in order to repress a target gene activity (e.g. see Thiesen et al. (1990) New Biologist 2: 363-374). Fragments of the Kox-1 protein comprising the KRAB domain, up to and including full-length Kox protein may be used as transcriptional repression domains, as described in Abrink et al. (2001) Proc. Natl. Acad. Sci. USA 98: 1422-1426. Additional transcriptional repressor domains known in the art may alternatively be used, such as the *engrailed* domain, the *snag* domain, and the transcriptional repression domain of v-erbA.

Other useful functional domains for control of gene expression include, for example, protein-modifying domains such as histone acetyltransferases, kinases, methylases and phosphatases, which can silence or activate genes by modifying DNA structure or the proteins that associate with nucleic acids (Wolffe (1996) Science 272: 371-372; and Hassig et al., (1998) Proc. Natl. Acad. Sci. USA 95: 3519-3524). Additional useful effector domains include those that modify or rearrange nucleic acid molecules such as methyltransferases, endonucleases, ligases, recombinases, and nucleic acid cleavage domains (see for example, Smith et al. (2000) Nucleic Acids Res. 17: 3361-9; WO 2007/139982 and references cited therein).

Particularly preferred for use in fusion proteins of the present invention are nucleic acid cleavage domains, especially endonuclease domains or functional or non-functional fragments / portions thereof.

Other domains that may also be appended to ZFPs of the invention (and which have biological functionality) include peptide sequences involved in protein transport, localisation sequences (e.g. subcellular localisation sequences, nuclear localisation, protein targeting) or signal sequences. ZFPs can also be fused to epitope tags (e.g. for use to signal the presence or location of a target nucleotide sequence recognised by the ZFP. Functional fragments of any such domain may also be used.

The expression of many genes is also achieved by controlling the fate of the associated RNA transcript. RNA molecules often contain sites for RNA-binding proteins, which determine RNA half-life and hence, levels of protein expression. Therefore, ZFP modulators of the invention may also control gene expression by specifically targeting RNA transcripts to either increase or decrease their half-life within a cell.

All known methods of conjugating an effector domain to a peptide sequence are incorporated. Thus, the term 'conjugate' is used in its broadest sense to encompass all methods of attachment or joining that are known in the art, and is used interchangeably with the terms such as 'linked', 'bound', 'associated', 'coupled' or 'attached'. The effector domain(s) can be covalently or non-covalently attached to the binding domain: for example, where the effector domain is a polypeptide, it may be directly linked to a ZFP (e.g. at the C-terminus) by a flexible or structured amino acid (linker) sequence encoded by the corresponding nucleic acid molecule. One suitable linker sequence for joining an effector domain, such as an exonuclease domain to the C-terminus of a ZFP is illustrated in Table 7B and 14: i.e. the sequence QNKKQ, which is used within the polypeptides of SEQ ID NOs: 2, 4, 6, 8 and 10 to join the zinc finger portion to the nuclease portion. Alternatively, a synthetic non-amino acid or chemical linker may be used, such as polyethylene glycol, a maleimide-thiol linkage (useful for linking nucleic acids to amino acids), or a disulphide link. Synthetic linkers are commercially available, and methods of chemical conjugation are known in the art.

Suitably, the polypeptides of the invention are arranged such that the ZFP portion is towards the N-terminus (e.g. at the N-terminus) and the effector (e.g. nuclease domain) is towards the C-terminus (e.g. at the C-terminus).

Non-covalent linkages between a ZFP and an effector domain can be formed using, for example, leucine zipper / coiled coil domains, or other naturally occurring or synthetic dimerisation domains (Luscher & Larsson (1999) Oncogene 18: 2955-2966; and Gouldson et al. (2000) Neuropsychopharm. 23: S60-S77. Other non-covalent means of conjugation may include a biotin-(strept)avidin link or the like. In some cases, antibody (or antibody fragment)-antigen interactions may also be suitably employed, such as the fluorescein-antifluorescein interaction.

To cause a desired biological effect via modulation of gene expression, ZFPs (or their corresponding fusion peptides) are allowed to interact with, and bind to, one or more target nucleotide sequence associated with the target gene, either *in vivo* or *in vitro* depending to the application. Beneficially, therefore, a nuclear localisation domain is attached to the DNA binding domain to direct the protein to the nucleus.

When the target sequence is genomic DNA, preferred DNA regions for ZFPs and ZFNs of the invention include promoters, enhancers or locus control regions (LCRs). The target sequences may encompass intronic or exonic sequences. Other suitable regions within genomes, which may provide useful targets for ZFPs of the invention, include telomeres and centromeres. In one embodiment, the genomic DNA target sequence comprises a p53 gene sequence.

### Zinc Finger Nucleases (ZFNs)

A ZFN of the invention comprises at least one ZFP portion (comprising more than one zinc finger domain) and at least one nuclease (or cleavage) portion. The nuclease domain can be located N- or C-terminal to the ZFP portion, but preferably it is towards or at the C-terminus. ZFNs and nuclease domains have particularly useful in the polypeptides and methods of the invention.

The nuclease / cleavage domain portion or fragment (e.g. nuclease half-domains) may be obtained from any species and type of endo- or exonuclease. Exemplary endonucleases from which a cleavage domain can be derived include, but are not limited to restriction endonucleases and homing endonucleases (e.g. Belfort et al. (1997) Nucleic Acids Res. 25: 3379-3388). Additional enzymes which cleave DNA are known and may alternatively be used (e.g. S1 Nuclease; mung bean nuclease; pancreatic DNase I; micrococcal nuclease; yeast HO endonuclease: see Linn et al. (eds.) Nucleases, Cold Spring Harbor Laboratory Press, 1993). One or more of these enzymes (or functional fragments thereof) can be used as a source of cleavage domains and cleavage half-domains. In embodiments comprising pairs of nuclease half-domains, the domains may be derived from the same endonuclease (or functional fragments thereof), or from different endonucleases.

Natural endonucleases are generally capable of sequence-specific nucleic acid binding (at a defined recognition site), and then cleave the nucleic acid molecule at or near the site of binding. Certain restriction enzymes (e.g. type IIS restriction enzymes) cleave DNA at sites displaced from the recognition site (e.g. from 1 to 10 nucleotides away from the binding site), and thus tend to have binding and cleavage domains that are separable using biotechnology procedures. Hence, nuclease domains derived from type IIS restriction endonucleases (see e.g. Roberts et al. (2003) Nucleic Acids Res. 31: 418-420) are particularly useful in the ZFNs of the invention, wherein nucleic acid binding and cleavage functions are performed by separate domains of the polypeptide.

A particularly well-studied and suitable type IIS restriction endonuclease domain is derived from the enzyme *Fok*l, which is active as a dimer (Bitinaite et al. (1998) Proc. Natl. Acad. Sci. USA 95: 10,570-10,575). Wild-type *Fok*l catalyses double-stranded cleavage of DNA 9 nucleotides from its recognition sequence on one strand and 13 nucleotides from its recognition site on the complementary strand (see for example: Li et al. (1992) Proc. Natl. Acad. Sci. USA 89: 4275-4279; Li et al. (1993) Proc. Natl. Acad. Sci. USA 90: 2764-2768; and Kim et al. (1994) Proc. Natl. Acad. Sci. USA 91: 883-887). Since it is only active as a dimer, ZFNs of the invention comprising a *Fok*l nuclease domain are also only active as a dimer. Accordingly, the *Fok*l domain is considered for the purposes of the present invention to be a nuclease / cleavage 'half-domain', and two *Fok*l domains are required for the cleavage of double-stranded nucleic acids such as genomic DNA sequences.

Suitably, the ZFNs of the invention comprise a ZFP portion and a Fokl nuclease domain. Accordingly, for targeted double-stranded cleavage of genomic (or other) DNA, including targeted replacement of genomic sequences using ZFNs of the invention, compatible pairs of ZFNs are most suitable; although a polypeptide comprising a ZFP portion and two compatible Fokl domains may alternatively be used.

When it is desired to use pairs of ZFNs to target and cleave a particular bipartite binding site (i.e. comprising two binding subsites - one for each of a pair of ZFNs), it is preferable that the nuclease half-domains or portions are inactive in isolation and only form an active nuclease complex capable of cleaving double-stranded polynucleotides when they associate as a dimer (as per the Fokl domain). In some embodiments the nuclease half-domains may be capable of homodimerisation, which is useful where the bipartite binding site is palindromic, for example. However, more suitably, the nuclease half-domains are adapted for heterodimerisation.

The target sites for the ZFNs of the invention are preferably selected such that when the two ZFNs are correctly bound to the double-stranded target the nuclease half-domains are in suitable spatial orientation to allow dimerisation and the formation of a functional cleavage domain. Any suitable spacing and orientation of the target subsites may be selected according to preferences and the design of the ZFNs. However, typically there will be up to 100bp of intervening DNA between target subsites, such as between about 1 and 50bp, suitably between about 2 and 20bp, and more suitably between about 3 and 10bp between the edges of the target subsites.

The binding subsites may be on the same strand or on opposite strands; although preferably the subsites are arranged on opposite strands of dsDNA. Binding sites on opposite strands are preferred, because - assuming the nuclease domain is located at the same terminus of each ZFN (e.g. at the C-terminus) - the binding sites can be arranged such that the nuclease half-domains are brought together on binding of the zinc finger portions to their respective target sites. The nucleic acid cleavage point thus typically lies between the target sites, and can generally be determined by appropriate spacing of the subsites.

Another factor that determines the most suitable orientation and separation of target subsites for cleavage by dimerisable ZFNs of the invention is the manner in which the ZFP portion is attached to the nuclease portion (see e.g. Smith et al. (2000) Nucleic Acids Res. 28: 3361-3369; Bibikova et al. (2001) Mol. Cell. Biol. 21: 289-297). Suitably, the attachment means is covalent, and preferably it is a peptide linker sequence, which allows the ZFN to be produced in unitary fashion by protein expression from a single encoding nucleic acid molecule. When the nuclease domain is attached at the C-terminus of the ZFP portion, the linker sequence is generally defined as the sequence between the most C-terminal zinc-coordinating histidine residue of the final zinc finger domain and the N-terminal-most residue attributed to the nuclease domain. The peptide linker may be any suitable sequence and length, such as up to about 100 amino acids, or between about 3 and 50 amino acids. Preferred linker sequences for use between the a ZFP of the invention and a nuclease domain may be between about 4 and 30 amino acids, or between about 5 and 20 amino acids, e.g. QNKKQ (SEQ ID NO: 119 - see polypeptides of SEQ ID NOs: 2, 4, 6, 8 and 10).

ZFNs and other ZFP-fusion proteins can be designed and constructed using any suitable method known in the art. For example, generally polynucleotides encoding the desired fusion proteins (such as expression vectors) are constructed and the encoding polypeptide is then expressed from a suitable *in vitro* system or within a cell, e.g. for *in vivo* or *ex vivo* applications, as described below.

### Polynucleotides and Polypeptide Expression

The ZFPs of the invention and, where applicable, the ZFP fusion peptides, such as ZFNs of the invention may be produced by recombinant DNA technology and standard protein expression and purification procedures. Thus, the invention further provides nucleic acid molecules (polynucleotides) that encode the ZFPs and ZFNs of the invention as well as their derivatives; and nucleic acid constructs, such as expression vectors that comprise nucleic acids encoding peptides and derivatives according to the invention.

For instance, the DNA encoding the relevant peptide can be inserted into a suitable expression vector (e.g. pGEM^{®}, Promega Corp., USA), where it is operably linked to appropriate expression sequences, and transformed into a suitable host cell for protein expression according to conventional techniques (Sambrook J. et al., Molecular Cloning: a Laboratory Manual, Cold Spring Harbor Press, Cold Spring Harbor, NY). Suitable host cells are those that can be grown in culture and are amenable to transformation with exogenous DNA, including bacteria, fungal cells and cells of higher eukaryotic origin, preferably mammalian cells.

To aid in purification, the ZFPs (and corresponding nucleic acids) of the invention may include a purification sequence, such as a His-tag. In addition, or alternatively, the ZFPs may, for example, be grown in fusion with another protein and purified as insoluble inclusion bodies from bacterial cells. This is particularly convenient when the ZFP or ZFN (or other effector) may be toxic to the host cell in which it is to be expressed. Alternatively, peptides of the invention may be synthesised *in vitro* using a suitable *in vitro* (transcription and) translation system (e.g. the *E. coli* S30 extract system, Promega corp., USA).

The term 'operably linked', when applied to DNA sequences, e.g. in an expression vector or construct, indicates that the sequences are arranged so that they function cooperatively in order to achieve their intended purposes, i.e. a promoter sequence allows for initiation of transcription that proceeds through a linked coding sequence as far as the termination sequence.

It will be appreciated that, depending on the application, the ZFP or ZFN of the invention may comprise an additional peptide sequence or sequences at the N- and/or C-terminus for ease of protein expression, cloning, and/or peptide or RNA stability, without changing the sequence of any zinc finger domain. For example, suitable N-terminal leader peptide sequences are MA, MAERP (SEQ ID NO: 120) or MAEERP (SEQ ID NO: 121).

In some applications it may be desirable to control the expression of polypeptides of the invention by tissue specific promoter sequences or inducible promoters, which may provide the benefits of organ or tissue specific and/or inducible expression of polypeptides of the invention. These systems may be particularly advantageous for *in vivo* applications, making knockout cell-lines and organisms and gene therapy. Examples of tissue-specific promoters include the human CD2 promoter (for T-cells and thymocytes, Zhumabekov et al. (1995) J. Immunological Methods 185: 133-140); the alpha-calcium-calmodulin dependent kinase II promoter (for hippocampus and neocortex cells, Tsien et al. (1996) Cell 87: 1327-1338); the whey acidic protein promoter (mammary gland, Wagner et al. (1997) Nucleic Acids Res. 25: 4323-4330); the mouse myogenin promoter (skeletal muscle, Grieshammer et al. (1998) Dev. Biol. 197: 234-247); and many other tissue specific promoters that are known in the art.

Suitable inducible systems may use small molecule induction, such as the tetracycline-controlled systems (tet-on and tet-off), the radiation-inducible early growth response gene-1 (EGR1) promoter, and any other appropriate inducible system known in the art.

In some embodiments of the methods of the invention, i.e. where the ZFN comprises a nuclease half-domain (such as the nuclease half-domain from the *Fok*l restriction enzyme), it is desirable to express two ZFNs in the same cell. However, in embodiments where the genomic target site is palindromic, nuclease activity can be achieved using two identical ZFNs. Expression of two fusion proteins in a cell can be achieved by the simultaneous or sequential (preferably simultaneous) delivery and/or expression in the cell of: the two polypeptides; one polypeptide and one nucleic acid encoding the other polypeptide; two nucleic acid molecules encoding the polypeptides; or a single nucleic acid encoding both polypeptides

In additional embodiments, a fusion protein that comprises a single polypeptide chain which comprises two nuclease half-domains and a ZFP portion comprising more than one zinc finger binding domain. In this case, biological activity may require only a single fusion protein / polypeptide to be expressed in the target cell

### Selection and Characterisation of Zinc Finger Peptides from Libraries

Selection and screening methods used in accordance with the invention can be applied to the selection of ZFPs for binding to any desired target site or nucleic acid sequence; particularly suitable recognition sequences comprise at least 12, at least 18, at least 24, at least 30, or at least 36 nucleotides (bps). Binding sites may be contiguous or non-contiguous, depending on preference. In some advantageous embodiments polypeptides of the invention are adapted to function in binding pairs (e.g. as ZFNs comprising nuclease half-domains), and in these cases the combined target site may be longer, for example: it may be a non-contiguous target site comprising pairs of binding subsites of approximately 12 or approximately 18 nucleotides.

ZFPs having desirable binding activity may be selected by screening libraries of ZFPs in a suitable selection process. In accordance with one aspect of the invention, nucleic acid libraries encoding a plurality of ZFPs may be expressed, and the synthesised peptides may be initially selected for their ability to bind a desired target sequence using any library generation and selection system known to the person of skill in the art. Exemplary library generation and selection systems are identified below.

One approach is to produce a mixed population of candidate peptides by cloning a randomised oligonucleotide library into an Ff filamentous phage gene, which allows relatively large peptides to be expressed on the surface of the bacteriophage (Lowman (1997) Ann. Rev. Biophys. Biomol. Struct., 26: 401-424; and Smith et al. (1993) Meth. Enz., 217: 228-257). Randomised peptide libraries up to 38 amino acids in length have also been made, and longer peptides may be achievable using this system. The peptide libraries that are produced are then typically mixed with a pre-selected matrix-bound nucleic acid target sequence. Peptides that bind are isolated, and their sequences are determined. From this information new peptides are synthesised and their biological properties can be assessed.

Another approach may involve chemically synthesising libraries of candidate peptides that can be screened for binding to any desirable target nucleic acid sequences.

A potential disadvantage of the above procedures is that the size of the libraries that are typically generated with both phage display and chemical synthesis is limited to within the 10⁶-10⁹ range. This limitation can result in the isolation of peptides of relatively low binding affinity for the target ligand, unless a time-consuming maturation process is subsequently used. This library-size limitation has led to the development of techniques for the *in vitro* generation of peptide libraries including: mRNA display (Roberts, & Szostak (1997) Proc. Natl. Acad. Sci. USA 94, 12297-12302); ribosome display (Mattheakis et al., (1994) Proc. Natl. Acad. Sci. USA 91, 9022-9026); and CIS display (Odegrip et al., (2004) Proc. Natl. Acad. Sci USA 101 2806-2810) amongst others. These libraries can be superior to phage display libraries (and other in vivo-based procedures), in that the size of libraries generated may be 2 to 5 orders of magnitude larger than is possible with phage display.

After selecting or designing ZFPs it can be beneficial to carry out a ZFP maturation process to try to fine-tune or optimise the ZFP already obtained in order to improve particular desirable characteristics and/or properties, such as binding affinity, binding selectivity or binding specificity. Known maturation techniques that may be used in accordance with the invention include relatively low frequency random mutagenesis techniques (as described e.g. in Sambrook *et al*.), such as error prone PCR (Cadwell & Joyce (1995) in PCR Primer, A Laboratory Manual, Dieffenbach and Dveksler (Eds) Cold Spring Harbor Press, Cold Spring Harbor NY, pp. 583-590); DNA shuffling (Crameri et al. (1996) Nat. Biotechnol. 14: 315-19), random-priming recombination (RPR) (Shao et al. (1998) Nucleic Acids Res. 26: 681-683); or the staggered extension process (StEO) (Zhao et al. (1998) Nature Biotechnol. 16: 258-261). For these methods it is typically desirable to have a mutation rate of approximately 1 to 3 mutations per peptide, so as to avoid the generation of high levels of inactive or undesirable mutations, while providing sufficient library diversity to improve the chances of identifying useful new peptide sequences. Thus, error prone PCR is best suited to embodiments when it is thought likely that just a few mutations, close to the original polypeptide sequence, will give a significant benefit.

Another way to 'mature' ZFPs is by targeted randomisation / library production. Since ZFP randomisation and screening is a compromise between library size and binding potential, in some embodiments of the invention it was decided to use targeted (or intelligent) randomisation in order to optimise the ability of the selection protocol to identify useful ZFPs. For example, while using NNN codons in a DNA library (where N is any nucleotide) give very large, comprehensive libraries; these libraries suffer from unwanted stop codons and very sparse appropriate DNA-binding residues. Thus, NNS codons (where S represents C or G) may sometimes be used to remove the majority of the possible stop codons and reduce library sequence diversity, while still coding for all possible amino acids.

In practice, the inventors have found that such small, targeted libraries, with selectively-randomised codons, are a good compromise between library size and DNA-binding potential.

In the prior art, where library size was too large or peptide length too long for a particular library screening strategy, ZFP domains may have been screened and selected as part of smaller sub-domains (e.g. of 1.5, 2 or possibly 3 adjacent zinc finger domains), and then joined together using appropriate linkers between adjacent zinc finger domains. Suitable linker sequences have already been described in relation to the peptides of the invention. Alternatively, nucleic acid sequences encoding the nucleic acid binding residues (e.g. the recognition sequence) of each zinc finger domain can be synthesised and grafted or cloned into the genetic sequence encoding each zinc finger domain of a poly-ZFP.

Although the above-mentioned engineering methods and information sources (e.g. the publicly available resources) facilitate obtaining and in some cases maturating ZFNs, the resultant zinc finger arrays have to be tested on a case-by-case basis for *in vivo* functionality and, previously, suboptimal candidates would often have to be abandoned because of a lack of straightforward optimisation protocols. For example, whereas screening systems exist for 1- to 2-finger libraries (e.g. phage display) and 3-finger mini-libraries of pre-selected modules (e.g. bacterial two-hybrid: Hurt *et al.* (2003)), no straightforward system exists to optimise the 4- to 6-finger type scaffolds (as provided by Sigma Aldrich, for example), which are preferred for targeting genomic sequences. Furthermore, since inter-finger interactions or other unknown interactions may have some influence over nucleic acid binding and sequence recognition, the selection and optimisation / maturation of complete binding blocks, such as these 4- and 6-finger arrays, may be particularly beneficial.

### Yeast one-hybrid assays

As noted, the above procedures do not generally allow the selection and/or optimisation of whole arrays of zinc finger domains for particular target sites.

Another type of selection or maturation system that may be used in accordance with the invention to identify DNA-binding peptides is the one-hybrid system, such as yeast one-hybrid systems. In these assays, polypeptides that bind to a 'bait element' (e.g. a *cis-*acting regulatory element or any other short DNA sequence), are identified by detecting the activation or repression of a reporter gene (see for example, Wei et al. (1999) Mol. Cell. Biol. 19: 1271-1278). In a similar fashion to two-hybrid systems, in the one-hybrid system the open-reading frame of the polypeptide of interest is fused to a transcriptional repressor or activator domain (generally an activator) of a transcription factor. When the fusion protein binds to a promoter of interest (i.e. the bait element) through its cognate DNA binding domain, reporter gene expression is activated by the activation domain part of the fusion protein and these active proteins can be selected for in resultant colonies (e.g. growing yeast cells).

A commercial yeast one-hybrid assay is the Matchmaker^{™} yeast one-hybrid system (Clontech, US). Briefly, as described in the User Manual (PT 1031-1; Catalog No. K1603-1 (1997), Clontech), to screen a library for a cDNA encoding a DNA-binding protein that interacts with a particular bait element, such as a promoter, yeast containing one or more bait-reporter constructs including a plurality of bait elements, are transformed with an activation domain fusion library, and the transformants are plated on a selective medium at an appropriate dilution to generate a masterplate. The activation domain fusion protein library is typically generated by co-transforming a nucleic acid library encoding variants of the DNA-binding peptide to be screened and having appropriate 5' and 3' sequences for homologous recombination, with a 'prey plasmid' containing the Gal4 transcriptional activation domain and compatible sequences for homologous recombination. Once transformed into yeast, the nucleic acid library sequence is inserted in-frame at the 3' end of the nucleic acid sequence encoding the Gal4 activation domain. This bypasses an additional cloning step and makes use of the high recombination rate in yeast cells to form a library of potential transcriptional repression fusion proteins. After incubating the plates for two days at 30°C, colonies are replica-plated onto different plates to test the activation of different bait-reporter constructs. To evaluate the activation of the His3 bait-reporter construct, the yeast cells are grown on plates lacking histidine with increasing concentrations of the transcriptional repressor 3-AT (e.g. 20 to 100 mM, suitably 25 to 75 mM). If the test fusion protein interacts with the bait element, the His3 reporter gene is expressed and colonies are able to grow on minimal medium lacking histidine and containing 3-AT. Therefore, a colony growing on the media is expected to contain a candidate DNA-binding fusion protein for the bait element concerned. Furthermore, since 3-AT is a competitive inhibitor of the His3 enzyme, higher amounts of His3 need to be expressed (due to transcriptional activation by the fusion protein activator) to confer growth in the presence of this compound. This provides a means to increase selectivity and reduce false positives that may otherwise be identified when background levels of His3 expression are relatively high.

However, the inventors have found that the existing one-hybrid assays, such as the yeast one-hybrid assay described above, are not optimal for the selection and maturation of some DNA-binding domains, such as ZFPs. Therefore, in accordance with another aspect of the invention, the invention is directed to an improved one-hybrid assay for the selection of DNA-binding peptides. The system of the invention facilitates and improves the selection of high-affinity DNA-binding peptides for any desired nucleic acid bait element sequence. In particular, the selection system of the invention reduces background reporter expression levels and false positives, which make the identification of active library members more difficult. The one-hybrid assay of the invention is preferably performed in yeast cells.

In one embodiment, the one-hybrid assay of the invention includes only a single copy of the bait element nucleic acid sequence in the bait plasmid: preferably at the 5' end of the reporter gene. Advantageously, therefore, it is more likely that only one fusion protein library member is able to bind to and activate (or repress as the case may be) the relevant reporter gene. Amongst other things, this may reduce mass action effects or variations due to fusion protein expression / concentration effects; and may help to select for the most tightly binding activator proteins.

Furthermore, in some embodiments of the invention, the DNA-binding polypeptide library is cloned in-frame at the 5' end (i.e. the N-terminus) of the relevant transcriptional effector domain (e.g. an activator domain such as Gal4). Without being bound by theory, this modification to the known protocol may have the beneficial effect of reducing reporter gene activation by non-desirable fusion proteins, such as peptides that are truncated in the library portion. According to the methods of the invention, it is likely that a truncated peptide (e.g. due to a STOP codon within the library sequence) will not possess a fully formed and/or functional transcriptional effector domain (contrary to the 3'-fusions of the prior art), and so any such truncated proteins should not be capable of activating expression of the reporter gene and should not then be selected.

In some embodiments, the invention provides methods for identifying proteins that associate with / bind to a bait element. The bait element may contain any desirable length of nucleotides, for example: at least about 9bp or up to about 200bp. Suitably, the bait element contains between about 9 and 100bp, between about 9 and 80bp or between about 9 and 60bp. The bait element may comprising a potential binding site for one or more polypeptides, for example, it may contain a pair of binding sites for use in selecting DNA-binding protein pairs; or it may comprise a non-contiguous binding sequence for recognition by a single DNA-binding protein having more than one nucleic acid binding domain. In such cases, the binding sequences (and the bait element when it consists only of the target binding sequence), may contain between about 9 and 54bp. More specifically, in some embodiments, the binding sequence may contain at least about 9bp, at least about 12bp, at least about 15bp, at least about 18bp, or at least about 24bp. Such bait element sequences are particularly preferred for use with zinc finger DNA-binding domains. However, for some applications, such as when it is desirable to find a DNA-binding domain that associates with a natural gene promoter region, the bait elements may be substantially longer and may comprise DNA sequences that lie upstream or downstream of endogenous genes, such as enhancer regions and introns. In such cases, the bait element may include over 1000bp derived from a target genome.

The bait element may be formed by any convenient method, such as annealing of complementary single-stranded oligonucleotides, primer fill-in, or PCR. Suitably, in accordance with the methods of the invention, the bait element is used as a single copy in the bait-reporter construct, which may be a plasmid or vector. The bait element may be flanked by nucleic acid sequences for homologous recombination (e.g. lambda recombination sites), for use in assembly of the bait-reporter construct. Alternatively or additionally, the bait element may be flanked by restriction sites for cloning of the bait element into the bait-reporter construct in a suitable orientation and location. Preferably, the bait element is located 5' to the reporter gene and in or near (e.g. upstream of) a minimal promoter region (Pₘᵢₙ) for the reporter gene in the bait construct. The reporter gene can be any gene whose expression can be detected. Therefore, any detectable peptide-encoding gene may be used as the reporter gene in the bait construct; for example, a gene whose expression is essential for survival of the cell in which it is expressed, such as His3 and/or LacZ (when the cells are grown on selective media); or a gene whose product can be visibly detected, such as a fluorescent protein, e.g. EGFP.

The methods of the invention may further include providing or obtaining a cell containing a bait-reporter construct. Any suitable cell may be used in the one-hybrid assay of the invention; although suitably the cell is a mammalian or yeast cell; preferably a yeast cell, such as yeast strain Y187.

More than one bait-reporter construct may be used in a target cell. The method of the invention may comprise transforming the cell with one or more bait-reporter constructs. In some embodiments, the bait-reporter construct (vector etc.) may be co-transformed with other nucleic acid sequences, such as the prey plasmid, an expression vector encoding a fusion protein comprising an activation domain and a library peptide; and/or nucleic acids encoding polypeptides of the library to be screened. The one or more bait-reporter constructs may be integrated into the genome of the cell.

The methods of the invention may also involve providing an expression vector encoding a fusion protein comprising a potential DNA-binding polypeptide and a fused transcription effector domain. Any appropriate effector domain may be used, such as a transcriptional activator or a transcriptional repressor domain. Suitably, the effector domain is an activator for up-regulating the expression of the reporter gene in the selected target cell. A beneficial activator domain is the Gal4 transcriptional activation domain. The expression vector may be constructed by inserting a nucleic acid sequence encoding the potential DNA-binding polypeptide into a prey plasmid comprising the appropriate effector domain. In some embodiments, the nucleic acid encoding the potential DNA-binding polypeptide may be formed by PCR. It can be convenient for the 5' and 3' ends of the nucleic acid encoding the DNA-binding peptide to include appropriate restriction enzyme sites and/or 5' and 3' homology arms to allow for cloning of the nucleic acid sequence into the prey plasmid. The appropriate 5' and 3' sequences can conveniently be introduced during PCR using appropriate primer sequences.

Next, the expression vector may be introduced into cells containing the bait construct. A convenient method is to simultaneously transform the cells (particularly yeast cells) with linear polynucleotides encoding the DNA-binding polypeptide or polypeptide library and linear prey plasmid, both of which include appropriate homology arms for homologous recombination. This saves an additional cloning step. However, alternatively the complete expression vector can be constructed, e.g. using appropriate restriction enzymes and ligation prior to transformation. The cell may previously have been transformed with the appropriate bait construct, or this may be performed simultaneously with the transformation of the expression construct(s) or shortly afterwards.

Transformed cells may then be assessed for activation (or repression) of the reporter gene. Activation of the reporter gene generally indicates that the activation domain fusion protein has associated with the bait element. In accordance with the invention, the effector (typically an activator) is suitably located at the C-terminus of the fusion protein so that any cells expressing truncated fusion proteins do not possess a complete effector domain and should not, therefore, be able to grow on the selective media.

In some embodiments, reporter gene expression is assessed by plating transformed (yeast) cells onto an appropriate selective media, such as nutrient deficient plates, and under suitable growth conditions, and observing cell growth. In some embodiments, activation may be assessed by determining the colour of the yeast cells, for example, by using genes encoding fluorescent reporter protein, such as luciferase, green fluorescent protein, yellow fluorescent protein, red fluorescent protein, blue fluorescent protein, or any other suitable gene or variant.

The method of the invention may further comprise selecting a cell (or cells) in which a reporter gene is activated; and isolating the cDNA encoding at least the DNA-binding domain of the activation domain fusion protein that is associating with the bait element in the cell. In some embodiments, the methods include determining the sequence of the isolated cDNA. The methods may also include contacting the cell with a test compound, and evaluating the effect of the test compound on the association (e.g. binding) between the bait element and the DNA-binding domain or DNA-binding domain-activation domain fusion protein.

The one-hybrid screening system of the invention can, therefore, be used to screen any potential DNA-binding domains for association with any DNA sequences (bait elements). The DNA-binding domains may be part of a library, which may include random libraries or selectively randomised libraries, such as those described herein. The bait elements may be derived from any organism, including mammals, and particularly human, rat, mouse, pig, sheep or cows.

A preferred DNA-binding domain for use in the one-hybrid assays of the invention is a zinc finger DNA-binding domain. Libraries of zinc finger domains can be synthesised and screened in the assay method for binding to a desired target sequence (i.e. the bait element). Since ZFP randomisation and screening is a compromise between library size and binding potential, it can be an advantage to use targeted (or intelligent) randomisation in order to optimise the ability of the selection protocol to identify useful ZFPs, as previously noted.

In accordance with some embodiments of the invention, therefore, small, targeted ZFP libraries, with selectively-randomised codons, are used. Thus, zinc finger libraries are suitably created by selective randomisation of an otherwise invariant framework. The selective randomisation may be selected positions within one or more zinc finger domains, and/or selected amino acid variations in each position to be diversified. In this way, zinc finger libraries may be used to design or select for ZFP domain arrays that target any desired nucleic acid sequence. In these embodiments, the zinc finger frameworks may be diversified at one or more positions in one or more zinc finger domain. In some embodiments the framework is diversified at one or more of amino acids positions -1, 1, 2, 3, 4, 5 and 6 of one or more domains. The polypeptide sequence changes may conveniently be achieved by diversifying the nucleic acid sequence encoding the zinc finger peptide frameworks at the codons for at least one of those positions, so as to encode more than one polypeptide variant. Beneficially, at least 2, at least 3, at least 4, at least 5, or at least 6 of the positions in the recognition sequence of each zinc finger domain of the library are diversified. For example, in one embodiment, the framework nucleic acid or peptide is varied specifically or randomly at one or more (e.g. 2, 3 or 4) of positions -1, 2, 3 and 6. All such nucleic acid and polypeptide variants are encompassed within the scope of the invention. Some exemplary zinc finger libraries of the invention are illustrated in the Examples (see Table 3 for general selective libraries according to α-helix position and target nucleic acid residue; and Table 4 for specific libraries for binding to p53 target sequences). Suitably, the ZFP libraries of the invention comprise at least 3 zinc finger domains, and preferably 4 or 6 zinc finger domains. Advantageously, the libraries include diversity in at least one position of each zinc finger domain in the ZFP library. In some embodiments, the methods of the invention encompass screening such ZFP libraries for binding to desired target sequences (e.g. for affinity maturation), particularly mammalian genomic sequences and especially p53 gene (or associated) sequences.

Hence, in one aspect the invention relates to a library of nucleic acids or polypeptides comprising more than one zinc finger peptide or encoding nucleic acid sequence, which allow selection of ZFPs having desirable properties (such as binding affinity for a chosen target nucleic acid sequence), from a suitable library screening / selection method. The library may be a 'naïve' library, i.e. comprising a mixture of peptides or nucleic acids that has not been optimised or selected to have a particular functionality; or a library based on one or more candidate molecule that has already been selected or partially selected towards a particular target. In a particularly advantageous embodiment, the library and methods of the invention are suitable for maturation of ZFPs. Therefore, the ZFPs may have previously been designed or selected to bind to a desired target site. The methods of the invention may, therefore, include providing a bait element comprising the desired target site, along with a ZFP library containing diversifications in one or more zinc finger domain of the ZFP already selected or designed, and performing a one-hybrid assay as already described in order to identify improved target site-binding ZFPs.

The amino acid residues at each of the diversified positions may be non-selectively randomised, i.e. by allowing the amino acid at the position concerned to be any of the 20 common naturally occurring amino acids; or may be selectively randomised, i.e. by allowing the specified amino acid to be any one from a defined sub-group of the 20 naturally occurring amino acids. It will be appreciated that one convenient way of creating a library of mutant peptides with randomised amino acids at each selected location, is to randomise the nucleic acid codon of the corresponding nucleic acid sequence that encodes the selected amino acid. On the other hand, given the knowledge that has now accumulated in relation to the sequence specific binding of zinc finger domains to nucleic acids, in some embodiments it may be convenient to select a specific amino acid (or small sub-group of amino acids) at one or more chosen positions in the zinc finger domain, for example, where it is known that a specific amino acid provides optimal binding to a particular nucleotide residue in a specific target sequence. Such libraries are the result of 'intelligent' design, as described in the Examples below. Conveniently the whole of the zinc finger recognition sequence may be selected by intelligent design and inserted / incorporated into an appropriate zinc finger framework. The person of skill in the art is well aware of the codon sequences that may be used in order to specify one, or more than one particular amino acid residue within a library. Non-randomised amino acid positions in each zinc finger domain may be chosen from known wild-type or artificial zinc finger structures.

The library for use in accordance with the invention thus comprises a plurality of nucleic acid sequences (e.g. at least 10³, 10⁴, 10⁵, 10⁶, 10⁷ or more different coding sequences), which can be expressed and screened to identify ZFPs having the desired or improved binding characteristics. Suitably, the diversity in the library is manageable in a yeast-one hybrid assay as described herein and, therefore, library sizes in the order of 10⁴ to 10⁷ are preferred as a compromise between diversity and screening capacity.

Yet another aspect of the present invention is directed towards the selection, identification and/or characterisation of ZFPs having a desired property, from a naïve zinc finger framework.

Also encompassed within the scope of the invention are nucleic acid-binding domains, such as ZFPs that are identified and/or isolated using the one-hybrid assay of the invention.

### Gene Therapy

One aspect of the invention relates to gene therapy treatments utilising polypeptides comprising ZFPs of the invention and/or polynucleotides encoding such polypeptides for treating diseases.

Gene therapy is the insertion of exogenous genomic / DNA sequences, such as whole genes or fragments thereof, into an individual's cell (e.g. animal or human) or biological tissues to treat a disease. For example, deleterious mutant gene alleles may be replaced with functional / corrected versions. The most promising target diseases to date are those that are caused by single-gene defects, such as cystic fibrosis, haemophilia, muscular dystrophy, sickle cell anaemia, and HD. Other common gene therapy targets are aimed at cancer and hereditary diseases linked to a genetic defect, such as p53 gene mutations.

Gene therapy is classified into two types: germ line gene therapy, in which germ cells, (i.e. sperm or eggs), are modified by the introduction of therapeutic genes, which are typically integrated into the genome and have the capacity to be heritable (i.e. passed on to later generations); and somatic gene therapy, in which the therapeutic genes are transferred into somatic cells of a patient, meaning that they may be localised and are not inherited by future generations.

Gene therapy treatments require delivery of the therapeutic gene or genes (or DNA or RNA molecule) into target cells. The therapeutic gene or genes may be the one or more ZFP-encoding polynucleotide of the invention alone, or in conjunction with another therapeutic polynucleotide. The other therapeutic polynucleotide may be a gene or gene fragment for replacing or repairing a mutated, damaged or missing endogenous gene, or may be an exogenous gene providing a useful therapeutic function.

There are two categories of delivery systems, either viral-based delivery mechanisms or non-viral mechanisms, and both mechanisms are envisaged for use with the present invention.

Viral systems may be based on any suitable virus, such as: retroviruses, which carry RNA (e.g. influenza, SIV, HIV, lentivirus, and Moloney murine leukaemia); adenoviruses, which carry dsDNA; adeno-associated viruses (AAV), which carry ssDNA; herpes simplex virus (HSV), which carries dsDNA; and chimeric viruses (e.g. where the envelop of the virus has been modified using envelop proteins from another virus).

One particularly suitable viral delivery system for some gene therapy systems is AAV. AAV is a small virus of the parvovirus family with a genome of single stranded DNA. A key characteristic of wild-type AAV is that it almost invariably inserts its genetic material at a specific site on human chromosome 19. However, recombinant AAV, which contains a therapeutic gene in place of its normal viral genes, may not integrate into the animal genome, and instead may form circular episomal DNA, which is likely to be the primary cause of long-term gene expression. Advantages of AAV-based gene therapy vectors include: that the virus is non-pathogenic to humans (and is already carried by most people); most people treated with AAV will not build an immune response to remove either the virus or the cells that have been successfully infected with it; it will infect dividing as well as non-dividing (quiescent) cells; and it shows particular promise for gene therapy treatments of muscle, eye, and brain. Thus, for use in gene therapy, a polypeptide encoding nucleic acid construct of the invention may be inserted into an AAV vector, particularly AAV2/1 subtype (see e.g. Molecular Therapy (2004) 10: 302-317).

Non-viral based approaches for gene therapy may alternatively be used, according to preferences. Where non-viral systems are possible, they can provide advantages, for example, in view of the simple large-scale production and low host immunogenicity. Types of non-viral mechanism include: naked DNA (e.g. plasmids); oligonucleotides (e.g. mRNA, double-stranded oligodeoxynucleotides, and ssDNA oligonucleotides); lipoplexes (complexes of nucleic acids and liposomes); polyplexes (complexes of nucleic acids and polymers); and dendrimers (highly branched, roughly spherical macromolecules).

Accordingly, the zinc finger-encoding nucleic acids of the invention may be used in methods of treating diseases by gene therapy or generating model animals for studying those diseases. Suitable genomic target sequences are those associated with genetic disorders, particularly neoplastic disorders and cancers, and more suitably disorders associated with mutation / substitution, deletion, insertion or expansion of genomic sequences. Preferred diseases for treatment with the therapeutic compositions and methods of the invention are those associated with alteration of the p53 gene, such as p53-associated cancers or other disorders in which p53 function or expression is altered.

In particular, the gene therapy therapeutics and regimes of the invention may provide for the expression of therapeutic ZFPs, ZFNs and other polypeptides in target cells for repressing the expression of target genes, such as those having either wild-type or non-wild-type sequences, and especially the *p53* gene. ZFPs and ZFP-effector fusion proteins of the invention are particularly useful for therapeutic applications in which it is desired to alter (e.g. up or down-regulate) the expression level of a target endogenous gene. ZFNs of the invention (e.g. as fusion proteins with the *Fok*1 nuclease domain or partial domain) may also be useful in gene therapy treatments for gene cutting or for directing the site of integration of therapeutic genes to specific chromosomal sites, as previously reported by Durai et al. (2005) Nucleic Acids Res. 33, 18: 5978-5990.

In one particular use or method of the invention, the ZFN is used to create a double-stranded break (cleavage) in a target DNA sequence, which in *in vitro,* ex *vivo* or *in vivo* applications within a cell (particularly mammalian cells such as human cells), may be 'repaired' by non-homologous end-joining (NHEJ; Lees-Miller & Meek (2003) Biochimie 85(11): 1161-73).

NHEJ is the primary pathway by which double-stranded DNA breaks are repaired in mammalian cells. It is called 'non-homologous' because no homology is required to direct the repair mechanism at the ends of the DNA strands to be joined. In non-mammalian cells and organisms such as yeast, homologous recombination tends to predominate. NHEJ is error-prone, and incorrect repair can result in mutations, such as DNA insertions and deletions, and even connection of the broken strand to the end of an unrelated DNA section.

NHEJ can therefore be useful for deleting or inactivating a target gene: for example, to create knock-out animal models for studying disease states, or for treating a disease by knocking out an aberrant gene. The inventors have determined (as described in the Examples) that treatment of target genes with ZFNs of the invention can cause detectable NHEJ events (i.e. in which the target DNA sequence is altered) in more than 10% of treated cells. Therefore, this method of the invention may be particularly beneficial for uses in which treated cells can be screened for NHEJ events prior to further use: such as in the creation of non-human transgenic animals. A preferred gene target for NHEJ is the p53 gene.

The invention also relates to transgenic knock-out animals, such as pigs, that are produced by the ZFNs of the invention.

### Diseases associated with the p53 gene or protein

The ZFPs, ZFNs and other therapeutic polypeptides and polynucleotides of the invention can be used to study and/or treat any p53 or p53-associated disease. These diseases and disorders include those in which the p53 gene itself is mutated or deleted (which may cause malexpression or malfunctioning of the p53 protein; and those in which associated or related genes or gene-products are over- or under-expressed so as to adversely affect the functioning of p53.

In this regard, the p53 gene is mutated in over 50% of all human cancers (Hollstein et al. (1991), Science 253: 49-53), but mutations associated with p53 or the p53 pathway (e.g. MDM2, AKT) are found in virtually 100% of all cancers. By way of example, *mdm2* amplifications occur in approximately 10% of tumours, especially sarcomas; whereas p14ARF function is lost in around 20 to 30% tumours (either by locus deletion, methylation or point mutations). These genetic alterations also, therefore, contribute to deregulation of p53 function. Moreover, AKT activation is necessary to shuttle MDM2 across the nuclear membrane, which is a necessary movement to initiate p53 degradation. AKT is activated in many tumours either by PTEN mutations (approx. 40%) or P13K amplifications (approximately 10%). Constitutive activation of AKT also contributes to p53 deregulation. In addition, Li-Fraumeni syndrome is an autosomal dominant hereditary disorder, where germline mutations in the p53 tumour suppressor gene have been shown to lead to a variety of cancers (Varley (2003) Hum. Mutat. 21 (3): 313-320).

Accordingly, the polypeptides and polynucleotides of the invention are useful for studying, detecting, diagnosing or treating various proliferative diseases; and particularly those associated with the p53 gene and/or protein.

### Therapeutic Compositions

A ZFP, ZFN or other ZFP-effector of the invention may be incorporated into a pharmaceutical composition for use in treating an animal, such as a human. A therapeutic peptide of the invention (or derivative thereof) may be used to treat one or more diseases or infections, depending on which genomic sequence the ZFP was selected or designed to recognise. Alternatively, a nucleic acid encoding the therapeutic peptide may be inserted into an expression construct / vector and incorporated into pharmaceutical formulations or medicaments for the same purpose.

ZFPs, ZFNs and other polypeptides of the invention typically contain naturally occurring amino acid residues, but in some cases non-naturally occurring amino acid residues may also be present. Therefore, so-called 'peptide mimetics' and 'peptide analogues', which may include non-amino acid chemical structures that mimic the structure of a particular amino acid or peptide, may also be used within the context of the invention. Such mimetics or analogues are characterised generally as exhibiting similar physical characteristics such as size, charge or hydrophobicity, and the appropriate spatial orientation that is found in their natural peptide counterparts. A specific example of a peptide mimetic compound is a compound in which the amide bond between one or more of the amino acids is replaced by, for example, a carbon-carbon bond or other non-amide bond, as is well known in the art (see, for example Sawyer, in Peptide Based Drug Design, pp. 378-422, ACS, Washington D.C. 1995). Such modifications may be particularly advantageous for increasing the stability of ZFP therapeutics and/or for improving or modifying solubility, bioavailability and delivery characteristics (e.g. for *in vivo* applications).

The therapeutic peptides and nucleic acids of the invention may be particularly suitable for the treatment of diseases, conditions and/or infections that can be targeted (and treated) intracellularly, for example, by targeting genetic sequences within an animal cell; and also for *in vitro,* ex *vivo* and *in vivo* applications. As used herein, the terms 'therapeutic agent' and 'active agent' encompass both peptides and the nucleic acids that encode a therapeutic polypeptide of the invention. Therapeutic nucleic acids include vectors, viral genomes and modified viruses, such as AAV, which comprise nucleic acid sequences encoding ZFPs and fusion proteins of the invention.

Therapeutic uses and applications for the polypeptides and nucleic acids of the invention include: the treatment of various neoplastic and non-neoplastic diseases and disorders; cancers / neoplastic diseases and related conditions; non-neoplastic conditions, such as neurological disorders, including head injury, spinal cord injury, acute hypertension, meningitis, encephalitis, cerebral malaria, multiple sclerosis, and encephalopathy; diabetic and other proliferative retinopathies; inflammation and inflammatory-related conditions. Other therapeutic uses for the molecules and compositions of the invention include the treatment of microbial infections and associated conditions, for example, bacterial, viral, fungal or parasitic infection. Diseases, particularly cancer, associated with aberrant p53 protein or aberrant expression of p53 are preferred and are amenable to the therapies of the present invention.

One or more additional pharmaceutical acceptable carrier (such as diluents, adjuvants, excipients or vehicles) may be combined with the therapeutic peptide of the invention in a pharmaceutical composition. Suitable pharmaceutical carriers are described in "*Remington*'*s Pharmaceutical Sciences*" by E. W. Martin. Pharmaceutical formulations and compositions of the invention are formulated to conform to regulatory standards and can be administered orally, intravenously, topically, or via other standard routes.

In accordance with the invention, the therapeutic peptide or nucleic acid may be manufactured into medicaments or may be formulated into pharmaceutical compositions. When administered to a subject, a therapeutic agent is suitably administered as a component of a composition that comprises a pharmaceutically acceptable vehicle. The molecules, compounds and compositions of the invention may be administered by any convenient route, for example, intradermal, intramuscular, intraperitoneal, intravenous, subcutaneous, intranasal, epidural, oral, sublingual, intranasal, intravaginal, transdermal, rectally, by inhalation, or topically to the skin. Administration can be systemic or local. Delivery systems that are known also include, for example, encapsulation in microgels, liposomes, microparticles, microcapsules, capsules, etc., and can be used to administer the compounds of the invention. Any other suitable delivery systems known in the art are also envisioned in use of the present invention.

Acceptable pharmaceutical vehicles can be liquids, such as water and oils, including those of petroleum, animal, vegetable or synthetic origin, such as peanut oil, soybean oil, mineral oil, sesame oil and the like. The pharmaceutical vehicles can be saline, gum acacia, gelatin, starch paste, talc, keratin, colloidal silica, urea, and the like. In addition, auxiliary, stabilising, thickening, lubricating and colouring agents may be used. When administered to a subject, the pharmaceutically acceptable vehicles are preferably sterile. Water is a suitable vehicle when the compound of the invention is administered intravenously. Saline solutions and aqueous dextrose and glycerol solutions can also be employed as liquid vehicles, particularly for injectable solutions. Suitable pharmaceutical vehicles also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or buffering agents.

The medicaments and pharmaceutical compositions of the invention can take the form of liquids, solutions, suspensions, lotions, gels, tablets, pills, pellets, powders, modified-release formulations (such as slow or sustained-release), suppositories, emulsions, aerosols, sprays, capsules (for example, capsules containing liquids or powders), liposomes, microparticles or any other suitable formulations known in the art. Other examples of suitable pharmaceutical vehicles are described in *Remington's* Pharmaceutical Sciences, Alfonso R. Gennaro ed., Mack Publishing Co. Easton, Pa., 19th ed., 1995, see for example pages 1447-1676.

Suitably, the therapeutic compositions or medicaments of the invention are formulated in accordance with routine procedures as a pharmaceutical composition adapted for oral administration (more suitably for humans). Compositions for oral delivery may be in the form of tablets, lozenges, aqueous or oily suspensions, granules, powders, emulsions, capsules, syrups, or elixirs, for example. Thus, in one embodiment, the pharmaceutically acceptable vehicle is a capsule, tablet or pill.

Orally administered compositions may contain one or more agents, for example, sweetening agents such as fructose, aspartame or saccharin; flavouring agents such as peppermint, oil of wintergreen, or cherry; colouring agents; and preserving agents, to provide a pharmaceutically palatable preparation. When the composition is in the form of a tablet or pill, the compositions may be coated to delay disintegration and absorption in the gastrointestinal tract, so as to provide a sustained release of active agent over an extended period of time. Selectively permeable membranes surrounding an osmotically active driving compound are also suitable for orally administered compositions. In these dosage forms, fluid from the environment surrounding the capsule is imbibed by the driving compound, which swells to displace the agent or agent composition through an aperture. These dosage forms can provide an essentially zero order delivery profile as opposed to the spiked profiles of immediate release formulations. A time delay material such as glycerol monostearate or glycerol stearate may also be used. Oral compositions can include standard vehicles such as mannitol, lactose, starch, magnesium stearate, sodium saccharine, cellulose, magnesium carbonate, etc. Such vehicles are preferably of pharmaceutical grade. For oral formulations, the location of release may be the stomach, the small intestine (the duodenum, the jejunem, or the ileum), or the large intestine. One skilled in the art is able to prepare formulations that will not dissolve in the stomach, yet will release the material in the duodenum or elsewhere in the intestine. Suitably, the release will avoid the deleterious effects of the stomach environment, either by protection of the peptide (or derivative) or by release of the peptide (or derivative) beyond the stomach environment, such as in the intestine. To ensure full gastric resistance a coating impermeable to at least pH 5.0 would be essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and Shellac, which may be used as mixed films.

To aid dissolution of the therapeutic agent or nucleic acid (or derivative) into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. Potential nonionic detergents that could be included in the formulation as surfactants include: lauromacrogol 400, polyoxyl 40 stearate, polyoxyethylene hydrogenated castor oil 10, 50 and 60, glycerol monostearate, polysorbate 20, 40, 60, 65 and 80, sucrose fatty acid ester, methyl cellulose and carboxymethyl cellulose. These surfactants, when used, could be present in the formulation of the peptide or nucleic acid or derivative either alone or as a mixture in different ratios.

Typically, compositions for intravenous administration comprise sterile isotonic aqueous buffer. Where necessary, the compositions may also include a solubilising agent.

Another suitable route of administration for the therapeutic compositions of the invention is via pulmonary or nasal delivery.

Additives may be included to enhance cellular uptake of the therapeutic peptide (or derivative) or nucleic acid of the invention, such as the fatty acids oleic acid, linoleic acid and linolenic acid.

In one pharmaceutical composition, a polypeptide or nucleic acid of the invention may be mixed with a population of liposomes (i.e. a lipid vesicle or other artificial membrane-encapsulated compartment), to create a therapeutic population of liposomes that contain the therapeutic agent and optionally the modulator or effector moiety. The therapeutic population of liposomes can then be administered to a patient by any suitable means, such as by intra-venous injection. Where it is necessary for the therapeutic liposome composition to target specifically a particular cell-type, such as a particular microbial species or an infected or abnormal cell, the liposome composition may additionally be formulated with an appropriate antibody domain or the like (e.g. Fab, F(ab)₂, scFv) or any alternative targeting moiety that recognises a particular target cell to tissue type. Such methods are known to the person of skill in the art.

The therapeutic peptides or nucleic acids of the invention may also be formulated into compositions for topical application to the skin of a subject.

The polypeptides and nucleic acids of the invention may also be useful in non-pharmaceutical applications, such as in diagnostic tests, *in vitro* assays, imaging, as affinity reagents for purification and as delivery vehicles.

### Examples

The invention will now be further illustrated by way of the following non-limiting examples.

Unless otherwise indicated, commercially available reagents and standard techniques in molecular biological and biochemistry were used. Many of the procedures used by the Applicant are described in Sambrook, J. *et al.,* 1989 *supra.:* analysis of restriction enzyme digestion products on agarose gels and preparation of phosphate buffered saline. General-purpose reagents, oligonucleotides, chemicals and solvents were purchased from Sigma-Aldrich Quimica SA (Madrid, Spain). Enzymes and polymerases were obtained from New England Biolabs (NEB Inc.; c/o IZASA, S.A. Barcelona, Spain).

### Example 1

### Design of zinc finger peptides against p53 target sites

### 1.1 Target site selection

To target a complex genome, such as a human genome, for gene therapy and other applications, it is generally beneficial for the target DNA sequence(s) to be unique or virtually unique within the genome. The chances of finding a unique target site are improved by systems that require there to be pairs (or dimers) of target sites in close proximity for activity. Where both of the individual sequences making up one half of a pair are the same (e.g. the sequence is palindromic), the combined target site may conveniently be considered to be homodimeric; on the other hand, when each sequence forming half of the target site is different, the target site can be considered heterodimeric.

In these Examples, four-finger ZFNs were used, each of which recognises an approximately 12bp sequence of contiguous DNA. As already described, the ZFNs of the invention operate in pairs, binding target sites on opposite DNA strands in close proximity, in order to form functional dimeric nuclease domains when bound to their intended paired (dimeric) target sequences. Therefore, the DNA sequence of the human p53 gene in the vicinity of the p53 mutation hotspot (see Figure 1A) was scanned to identify paired 12bp sequences that might form potentially unique dimeric target sites within the human genome. Two potential heterodimeric target sites each comprising two 12bp target sequences on opposite DNA strands were identified, termed z771 and z1166 according to their location in the p53 gene (See black-boxed sequences in Figure 1 B), and novel functional four-finger ZFPs were designed and selected to bind these target sequences. The ZFPs constructed to bind the 12bp binding sites making up each heterodimeric target site are identified as z771 L, z771 R, z1166L and z1166R, where L and R represent left and right, respectively (see Figure 1 C). The binding sites selected for each ZFP were: 3'- GGGGACGAACCG -5' (z771 R; corresponding to SEQ ID NO: 41); 3'- GTACTCGGTGAC -5' (z771L; corresponding to SEQ ID NO: 42); 3'-GTGGTAGGTGAT -5' (z1166R; corresponding to SEQ ID NO: 43); and 3'-GACAATGTGTAC -5' (z1166L; corresponding to SEQ ID NO: 44); and in the genomic sequence of p53 there were 6bp intervening sequence (or a gap) separating the binding sites of each heterodimeric target site (i.e. between z771 R and z771 L, and between z1166R and z1166L).

The uniqueness of the target sites in the entire human genome was verified by a genome scanning algorithm developed by the inventors (see Table 1). This showed that the z771L/R and z1166L/R target sites are in fact unique. More detailed analysis showed that the z1166 target site had very few related targets in the human genome (i.e. only one 2bp mismatch and eight 3bp mismatches for the full heterodimeric site); while the z771 target site had slightly more related targets (i.e. 35 sequences with 2bp mismatches for the full z771 zinc finger binding site), but these were identified as being mostly in duplicated intronic sequences. In the event of non-specific (i.e. unintended) binding to any related non-target sites, it is thought that intron sites are likely to be more tolerant of indels from NHEJ, because no coding sequence is disrupted. Furthermore, intron sites can still be used for exonic gene repair, because homologous recombination can extend for hundreds of bases beyond the double-stranded break. Interestingly, both target binding sequences had virtually no related homodimer matches (i.e. z771 L/L, z771 R/R, z1166L/L and z1166R/R) in the human genome.

**Table 1: Putative off-target sites. The number of occurrences in the human genome of sequences related to the target site were counted with a computer script written in C. bs=binding site; pts=palindromic target site. Pts771L (SEQ ID NO: 122), pts771R (SEQ ID NO: 123), bs771 (SEQ ID NO: 124), pts1166L (SEQ ID NO: 125), pts1166R (SEQ ID NO: 126), bs1166 (SEQ ID NO: 127).**

| **Target sequence** | | **0bp** | **1bp** | **2bp** | **3bp** | **4bp** |
|---|---|---|---|---|---|---|
| pts771L | cctCATGAGCCACTGtgggcgCAGTGGCTCATGcct | 0 | 0 | 0 | 2 | 25 |
| pts771R | cctCCCCTGCTTGGCtgggcgGCCAAGCAGGGGcct | 0 | 0 | 0 | 0 | 14 |
| bs771 | cctCCCCTGCTTGGCtgggcgCAGTGGCTCATGcct | 0 | 7 | 35 | 457 | 1956 |
| pts1166L | tacCTGTTACACATGcaactaCATGTGTAACAGttc | 0 | 0 | 0 | 1 | 14 |
| pts1166R | tacCACCATCCACTAcaactaTAGTGGATGGTGttc | 0 | 0 | 0 | 0 | 3 |
| bs1166 | tacCACCATCCACTAcaactaCATGTGTAACAGttc | 0 | 0 | 1 | 8 | 20 |

### 1.2 Construction and design of four-finger ZFPs

A ZFP having rationally designed (DNA) recognition helices was constructed by 'intelligent' design using available data on ZFP-DNA binding interactions. After, the recognition helices were selectively randomised in order to determine whether improved ZFP DNA-binding domains could be created (see Example 1.2.4 and Tables 2, 3, 4 and 5).

### 1.2.1 Rational design of ZFP to bind p53 target sequences

ZFPs for binding to target DNA sequences in the p53 gene were constructed from a backbone ZFP sequence based on the wild-type ZFP Zif268 in which the sequences of the DNA-recognition helices had been specifically mutated with a view to binding the selected DNA target sequence. The amino acid sequences for each DNA-recognition helix of the ZFP were chosen by rational design based on the published ZFP literature (e.g. Isalan (2004) Zinc Fingers. Encyclopedia of Biological Chemistry. Elsevier/Academic Press, ISBN: 0124437109, 435-439). Two-finger subunits were used, based on the second and third fingers (F2-F3) of the Zif268 sequence (Pavletich & Pabo (1991) Science 252: 809-817). Each pair of two-finger subunits was then separated by a longer TGSERP linker (Moore et al., (2001) Proc. Natl. Acad. Sci. USA 98: 1437-1441), as previously described; although other suitable 6 amino acid linker peptides could alternatively be used.

Four-finger library cassettes were constructed from oligonucleotides using a PCR based construction approach. Two-finger units (F1-F2 and F3-F4) making up the four-finger ZFPs of the invention were built from two oligonucleotides by overlapping primer extension (Isalan M. (2006) Nat. Protoc. 1: 468-475). After amplification by PCR, introducing a *BamH*I site at the 3'-end of F1-F2 and a *Bgl*II site at the 5'-end of F3-F4, the two-finger units were mixed, cut with *BamH*I and *Bgl*II and conditionally ligated. The resulting four-finger library cassettes (F1-F2-F3-F4) were amplified by PCR using oligonucleotides which added 5'- and 3'-homology arms for a prey plasmid for yeast-one hybrid selections, as described below.

Rationally designed ZFPs were then tested for binding to their target binding sequences and cloned into a suitable vector for yeast one-hybrid assays for comparison with library-selected ZFPs as described below.

### 1.2.2 ZFP cloning for use in a yeast one-hybrid assay

The design and construction of a vector for the expression of ZFPs in a yeast one-hybrid assay as schematically illustrated in Figure 2, is described here with reference to one of the ZFPs of the invention, z1166L (see Table 2). Any in-frame zinc finger sequence can be inserted into the Matchmaker Y1H system using the template that has been developed here. As already noted, the template is based on 2 x two-finger subunits of F2-F3 of the Zif268 sequence (Pavletich & Pabo (1991) Science 252: 809-817). Each pair of two-finger subunits is separated by a TGSERP linker, which is one amino acid longer than the wild-type canonical linker (Moore et al., (2001) Proc. Natl. Acad. Sci. USA 98: 1437-1441). The final C-terminal linker, QNKKQLVKSEL (SEQ ID NO: 128) suitable for linking the ZFP to the *Fok*I nuclease domain to create a ZFN is a mixture of the Sp1 ZFP C-terminal linker QNKK (SEQ ID NO: 129) and the *Fok*I leader sequence QLVKSEL (SEQ ID NO: 130) and is adapted from Bibikova et al. (2001) Mol. Cell. Biol. 21: 289-297).

Four-finger library cassettes (F1-F2-F3-F4) are amplified by PCR using oligonucleotides to add 5'- and 3'-homology arms as shown in Table 2 and subsequently introduced into the *Hind*III-linearised modified prey plasmid pGADT7-Rec2 (Clontech's Matchmaker One-Hybrid Library Construction and Screening Kit; Ref 630304) in which the second *Hind*III site at 2351 bp had been removed by site directed mutagenesis, for in-frame recombination in yeast strain Y187. Plasmid template was removed by Dpnl digestion prior to bacterial transformation.

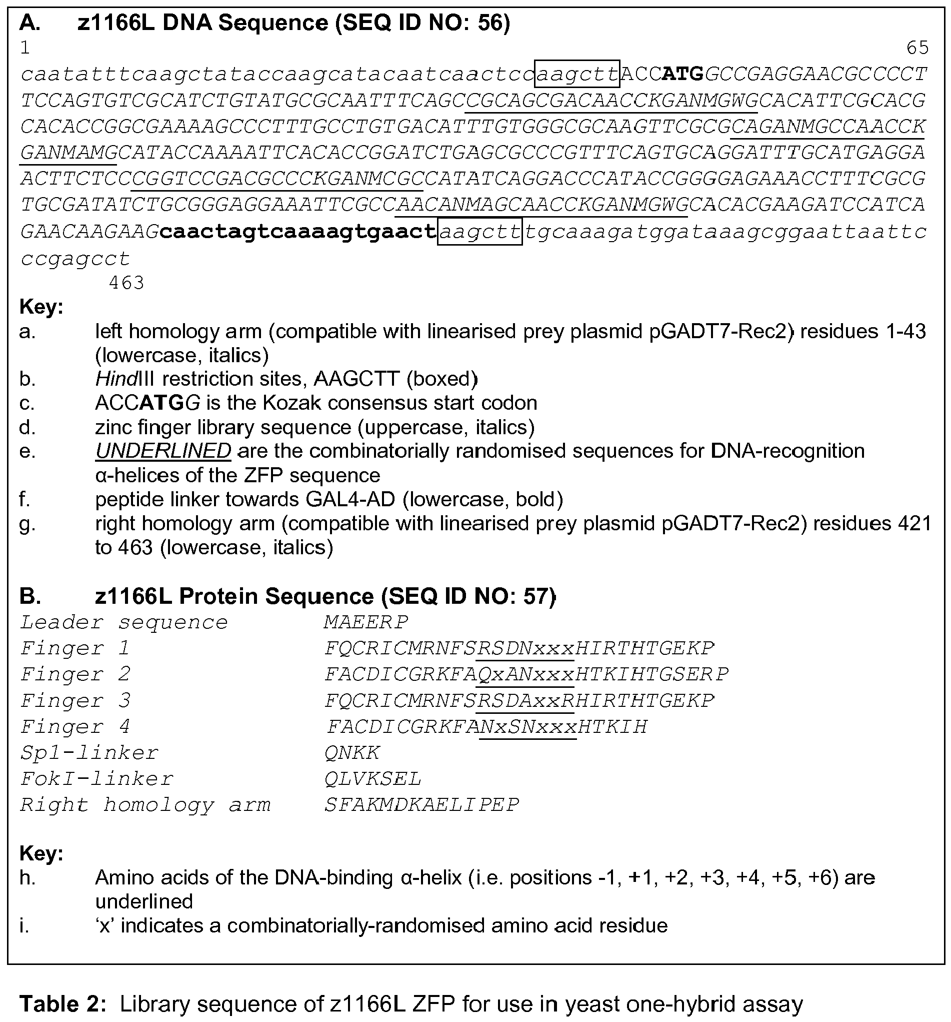

### 1.2.3 ZFP library design

To generate ZFP libraries one or more of the amino acid positions in the DNA-binding α-helix of each finger (see residues underlined in Table 2 above, e.g. RSDNxxx) was randomised. In order to keep the library size to a manageable diversity, however, selective randomisations (rather than full randomisations) were carried out, i.e. at selected positions and using a select group of amino acid residues, depending on the position in the α-helix and the corresponding nucleic acid of the target sequence.

The selection of amino acids used in each of the chosen positions was based on the amino acids that were predicted to work best at certain positions, having regard to the publicly available information on ZFP-DNA binding interactions - primarily derived from previously reported phage display experiments, for example: position -1, see Proc. Natl. Acad. Sci. USA. (1994), 91(23): 11168-72 and others; position +2, see Proc. Natl. Acad. Sci. USA. (1997), 94: 5617-21 (1997) and Biochemistry (1998), 37: 12026-33; position +3, see Proc. Natl. Acad. Sci. USA. (1994), 91 (23): 11168-72 and others; and position +6, see Proc. Natl. Acad. Sci. USA. (1994), 91 (23): 11168-72 and others.

The potential selective randomisations used to construct ZFP libraries in accordance with the invention are shown In Table 3. As illustrated, randomisations were selected according to the relative position in the α-helix of the residue to be varied, the base of the target sequence that is contacted by the amino acid in that position, and the degeneracy that is desired for the library concerned. Codons highlighted in bold represent the preferred options for a particular position, but whether or not the preferred options alone were used was determined by a balance between the desired library size and the desired library binding potential.

**Table 3: Library generation of DNA-binding residues in ZFP according to α-helix position and target base.**

| **Library codon** | **Amino acids encoded** | **Helix position** | **Potential DNA bases bound** | **DNA sequence degeneracy** |
|---|---|---|---|---|
| **ARG** | **KR** | **-1,+6** | **G,T** | **2** |
| **AMC** | **NT** | **-1,+3** | **A,T** | **2** |
| **RMC** | **NTAD** | **-1,+3** | **A,T,C** | **4** |
| **CAS** | **QH** | **1** | **A,T,C** | **2** |
| MAC | NH | -1 | A,T,C | 2 |
| GHC | VDA | -1,+3 | T,C,(N) | 3 |
| RAC | DN | -1 | C,A,T | 2 |
| GWC | DV | -1,+3 | C,T | 2 |
| GMC | AD | -1,+3 / +2 | T,C / (N) | 2 |
| SAC | HD | -1 / +3 | T,C / C,G | 2 |
| GMS | ADE | -1,+3 | T,C,(N) | 4 |
| GVS | GADE | -1,+3 | C,(N) | 6 |
| GHS | VADE | -1,+3,+6 | T,C,(N) | 6 |
| RRK | NDESRKG | -1,+6 | A,C,G,T,(N) | 8 |
| **AGC** | **S** | **+1** | | **1** |
| **ANM** | **RSKNTI** | **+1,+4,+5** | | **8** |
| **GMC** | **AD** | **+2** | **(N)** | **2** |
| **RRC** | **DGNS** | **+2 / +3** | **(N) / A,T,C** | **4** |
| ARC | SN | +2 | (N) | 2 |
| KMC | YSAD | +2 | (N) | 4 |
| MRC | RHNS | +2 | (N) | 4 |
| VRC | RHSNGD | +2 / -1,+3 | (N) / (N) | 6 |
| SDG | RQLGEV | +2 | (N) | 6 |
| **RMC** | **TNAD** | **+3,-1** | **A,T,C** | **4** |
| **CAC** | **H** | **+3** | **(G)** | **1** |
| GYC | AV | +3 | T,C) | 2 |
| RCC | AT | +3 | T,C(N) | 2 |
| ASC | ST | +3 | T,C | 2 |
| ASS | STR | -1,+3,+6 | G,T,C | 4 |
| ARS | SNKR | -1,+3,+6 | (N) | 4 |
| AVS | SNKRT | -1,+3,+6 | (N) | 6 |
| ANS | SNKRTMI | -1,+3,+6 | (N) | 8 |
| **CKG** | **LR** | **+4** | **(N)** | **2** |
| CDC | LRH | +4 | (N) | 3 |
| **AWA** | **IK** | **+5** | **(N)** | **2** |
| AHA | KIT | +5 | (N) | 3 |
| AHM | KITN | +5 | (N) | 6 |
| **ANM** | **RSKNTI** | **+1,+4,+5** | | **8** |
| ABS | MIRST | +1,+4,+5 | | 6 |
| **AMG** | **KT** | **+6 / +5** | **G,T / (N)** | **2** |
| **GWG** | **EV** | **+6** | **C,T** | **2** |
| **TAC** | **Y** | **+6** | **A** | **1** |
| **CAG** | **Q** | **+6** | **A** | **1** |
| **CRG** | **QR** | **+6** | **A,G** | **2** |
| **SAG** | **QE** | **+6** | **A,C** | **2** |
| **DAC** | **YND** | **+6** | **A,C** | **3** |
| MAG | QK | +6 | A,G | 2 |
| RYA | ATVI | +6 | T,C,(N) | 4 |
| GHG | EAV | +6,+3 | T,C,(N) | 3 |
| MAM | NQKH | +6 | A,G,T | 4 |
| RMG | TAKE | +6 | T,G,C,(N) | 4 |
| RMK | TKAEDN | +6 | T,G,C,A,(N) | 8 |
| **KEY: IUPAC Base code** | | | | |
| Letter = DNA bases coded | | | | |
| R=AG; Y=CT; M=AC; K=GT; S=CG; W=AT; B=CGT; D=AGT; H=ACT; V=ACG | | | | |

The intelligent or selective randomisations given in Table 3 above are based on an assumed canonical binding mode. However, there are many known examples where unexpected combinations of amino acids give high-affinity, specific DNA-binding interactions, which may not be expected. Therefore, larger libraries in which some non-expected amino acid residues are included at certain positions may sometimes be advantageous, if library size permits.

Appropriate positions in each finger of the four-finger ZFPs were selectively randomised to try to improve the binding specificity of the fingers for each of their target binding triplets using the yeast one-hybrid assay. By way of example, the rational design of finger 3 (F3) of z1166L, which is intended to bind the triplet 3'- AAT -5' is N- QKANRTK - C, and is shown aligned to its DNA recognition site below (aligned with ZFP sequence N-C and DNA sequence 3'-5'). Rationally designed F3 of z1166L was then selectively randomised at positions +1, +4, +5, and +6 to try to improve its binding specificity and affinity for its target site using the variants shown below.

3'-a  a  t-5'                                    QKANRTK
   |  |  |                                       R  LKT
 N-QKANRTK-C                                     S   R
                                                 N   S
                                                 T   N
                                                 I   I

The required intelligent library can be created using the following nucleic acid codons to encode the α-helix: 5'- CAG ANM GCC AAC CKG ANM AMG -3' (SEQ ID NO: 58).

### 1.2.4 Four-finger ZFP library design and construction for p53 gene targeting

First, the desired selective randomisations for each finger of each of the four-finger ZFPs for targeting the p53 gene were chosen. The libraries were selected on the basis of library size (degeneracy) and the desire to optimise particular regions of the DNA-recognition α-helices (see Table 4). In Table 4, the ZFP recognition helices (e.g. N-RSSHLSR -C) are illustrated in the N-terminal to C-terminal orientation, and are shown aligned to its target DNA recognition sequences in the 3' to 5' orientation. The randomised amino acid positions are indicated, as well as the amino acid variants forming the library at each of the positions chosen for selective randomisation. The library size (n) is also given directly below each varied position (synonymous codons are counted separately), and the total library size is given on the right.

Each of the four-finger ZFP library cassettes were constructed from oligonucleotides using a PCR based construction approach (i.e. oligonucleotide-overlap and primer fill-in), using the oligonucleotides shown in Table 4. Two-finger units (F1-F2 and F3-F4) were built from two oligonucleotides by overlapping primer extension (Isalan (2006), Nat. Protoc. 1: 468-475). After amplification by PCR, introducing a *BamHI* site at the 3'-end of F1-F2 and a *Bgl*II site at the 5'-end of F3-F4, the two-finger units were mixed, cut with *BamHI* and *Bgl*II and conditionally ligated.

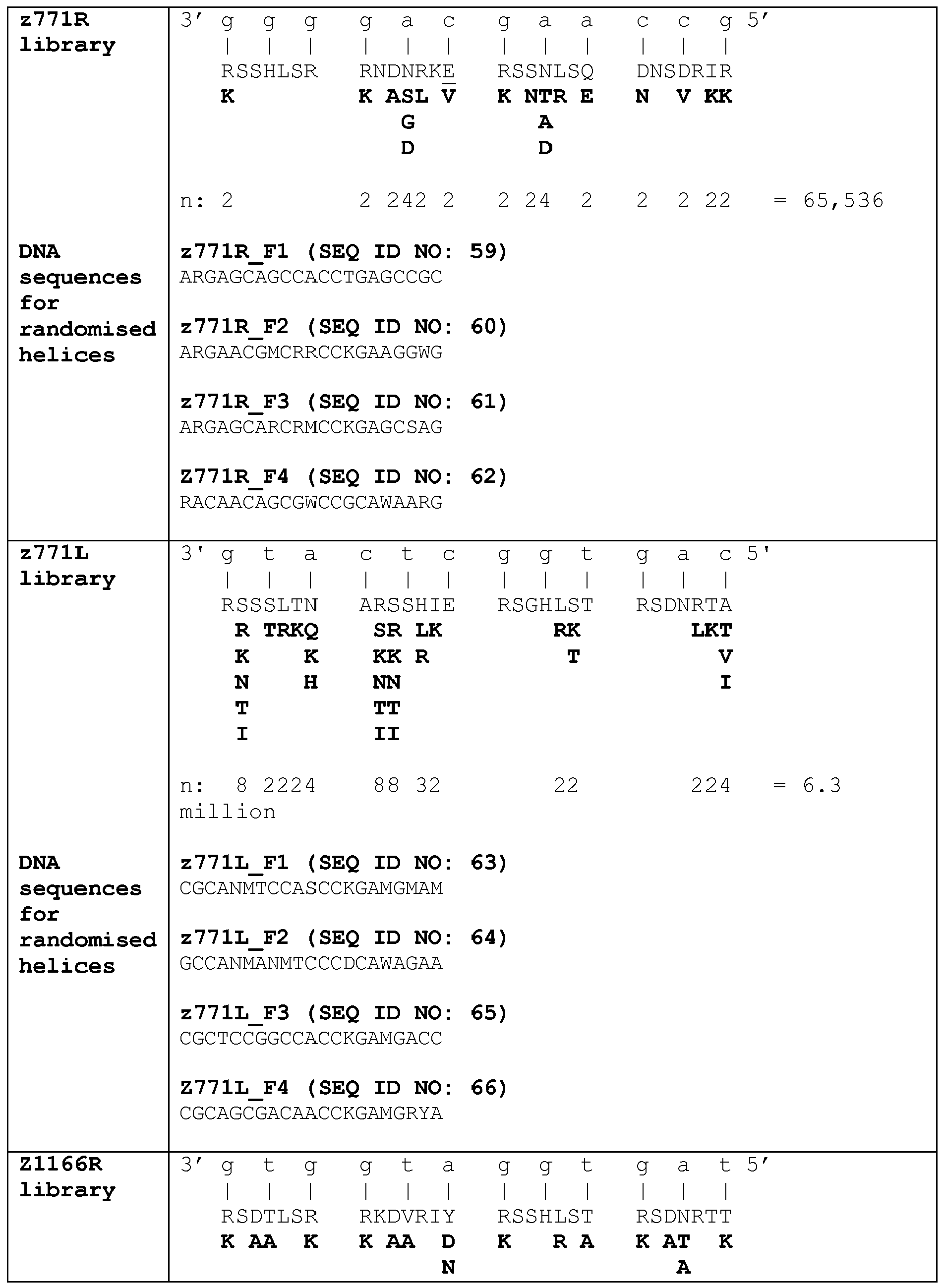

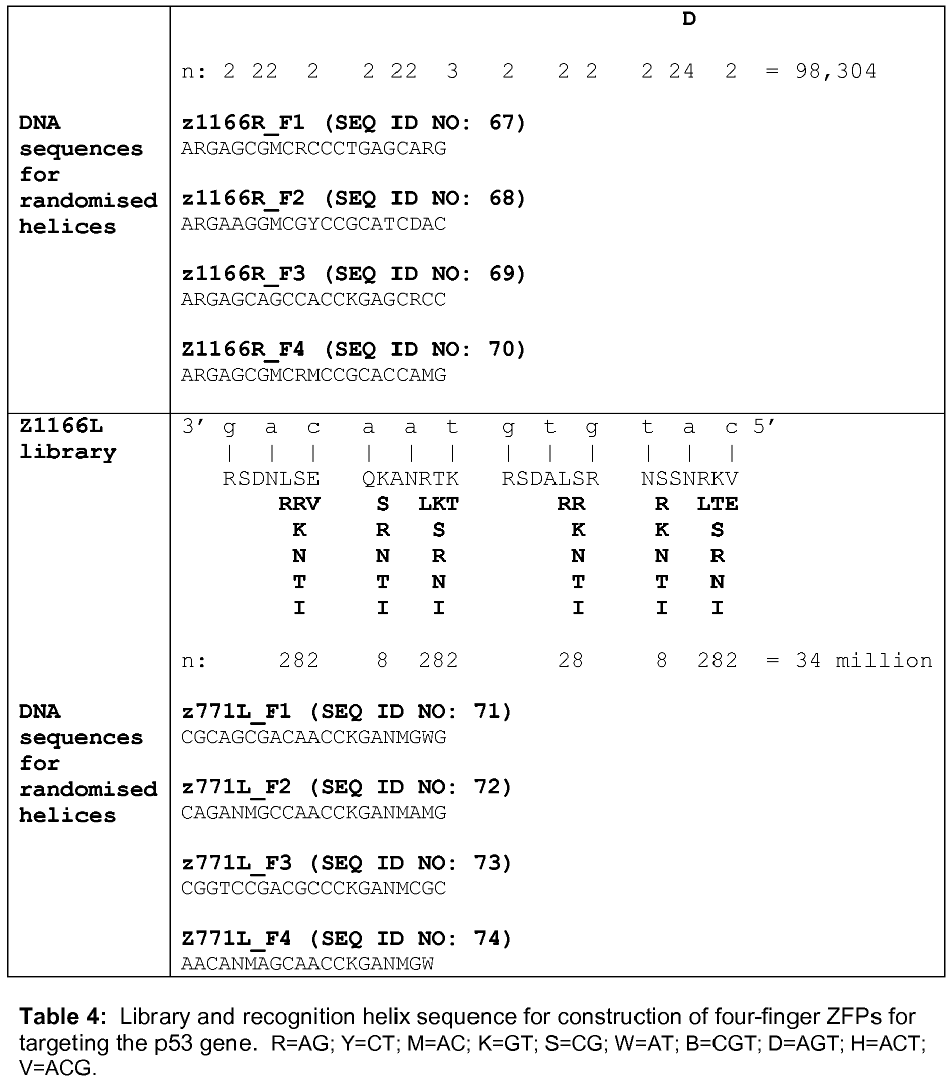

The resulting four-finger library cassettes (F1-F2-F3-F4) were amplified by PCR using oligonucleotides which added 5'- and 3'-homology arms for inserting and expressing in the yeast one-hybrid prey plasmid. In contrast to the conventional yeast one-hybrid assay system and that described in the Matchmaker kit (Clontech), the ZFP library cassettes and homology arms were designed and constructed for insertion 5' (i.e. N-terminal) to the Gal4-AD sequence in the yeast one-hybrid prey plasmid (rather than 3'). The sequences of the four ZFP libraries used in the yeast one-hybrid assays are given in Table 5.

| | |
|---|---|
| **Library z771 R (SEQ ID NO: 46)** | |
| caatatttcaagctataccaagcatacaatcaactccaagcttACCATGGCCGAGGAACGCCCC TTCCAGTGTCGCATCTGTATGCGCAATTTCAGCARGAGCAGCCACCTGAGCCGCCACATTCGCA CGCACACCGGCGAAAAGCCCTTTGCCTGTGACATTTGTGGGCGCAAGTTCGCGARGAACGMCRR CCKGAAGGWGCATACCAAAATTCACACCGGATCTGAGCGCCCGTTTCAGTGCAGGATTTGCATG AGGAACTTCTCCARGAGCARCRMCCKGAGCSAGCATATCAGGACCCATACCGGGGAGAAACCTT TCGCGTGCGATATCTGCGGGAGGAAATTCGCCRACAACAGCGWCCGCAWAARGCACACGAAGAT CCATCAGAACAAGAAGcaactagtcaaaagtgaactaagcttgcaaagatggataaagcggaa ttaattcccgagcct | |
| **Library z771 L (SEQ ID NO: 47)** | |
| caatatttcaagctataccaagcatacaatcaactccaagcttACCATGGCCGAGGAACGCCCC TTCCAGTGTCGCATCTGTATGCGCAATTTCAGCCGCANMTCCASCCKGAMGMAMCACATTCGCA CGCACACCGGCGAAAAGCCCTTTGCCTGTGACATTTGTGGGCGCAAGTTCGCGGCCANMANMTC CCDCAWAGAACATACCAAAATTCACACCGGATCTGAGCGCCCGTTTCAGTGCAGGATTTGCATG AGGAACTTCTCCCGCTCCGGCCACCKGAMGACCCATATCAGGACCCATACCGGGGAGAAACCTT TCGCGTGCGATATCTGCGGGAGGAAATTCGCCCGCAGCGACAACCKGAMGRYACACACGAAGAT CCATCAGAACAAGAAGcaactagtcaaaagtgaactaagcttgcaaagatggataaagcggaa ttaattcccgagcct | |
| **Library z1166R (SEQ ID NO: 48)** | |
| caatatttcaagctataccaagcatacaatcaactccaagcttACCATGGCCGAGGAACGCCCC TTCCAGTGTCGCATCTGTATGCGCAATTTCAGCARGAGCGMCRCCCTGAGCARGCACATTCGCA CGCACACCGGCGAAAAGCCCTTTGCCTGTGACATTTGTGGGCGCAAGTTCGCGARGAAGGMCGY CCGCATCDACCATACCAAAATTCACACCGGATCTGAGCGCCCGTTTCAGTGCAGGATTTGCATG AGGAACTTCTCCARGAGCAGCCACCKGAGCRCCCATATCAGGACCCATACCGGGGAGAAACCTT TCGCGTGCGATATCTGCGGGAGGAAATTCGCCARGAGCGMCRMCCGCACCAMGCACACGAAGAT CCATCAGAACAAGAAGcaactagtcaaaagtgaactaagctttgcaaagatggataaagcggaa ttaattcccgagcct | |
| **Library z1166L (SEQ ID NO: 49)** | |
| caatatttcaagctataccaagcatacaatcaactccaagcttACCATGGCCGAGGAACGCCCC TTCCAGTGTCGCATCTGTATGCGCAATTTCAGCCGCAGCGACAACCKGANMGWGCACATTCGCA CGCACACCGGCGAAAAGCCCTTTGCCTGTGACATTTGTGGGCGCAAGTTCGCGCAGANMGCCAA CCKGANMAMGCATACCAAAATTCACACCGGATCTGAGCGCCCGTTTCAGTGCAGGATTTGCATG AGGAACTTCTCCCGGTCCGACGCCCKGANMCGCCATATCAGGACCCATACCGGGGAGAAACCTT TCGCGTGCGATATCTGCGGGAGGAAATTCGCCAACANMAGCAACCKGANMGWGCACACGAAGAT CCATCAGAACAAGAAGcaactagtcaaaagtgaactaagctttgcaaagatggataaagcggaa ttaattcccgagcct | |
| **Key:** | |
| a. | left homology arm (compatible with linearised prey plasmid pGADT7-Rec2) residues 1-43 (lowercase, italics) |
| b. | *Hind*III restriction sites, AAGCTT (boxed) |
| c. | ACC**ATG***G* is the Kozak consensus start codon |
| d. | zinc finger library sequence (uppercase, italics) |
| e. | *UNDERLINED* are the combinatorially randomised sequences for DNA-recognition α-helices of the ZFP sequence |
| f. | peptide linker towards GAL4-AD (lowercase, bold) |
| g. | right homology arm (compatible with linearised prey plasmid pGADT7-Rec2) residues 421 to 463 (lowercase, italics) |

**Table 5:** Nucleic acid sequences of the four ZFP libraries used in the yeast one-hybrid assays.

### Example 2

### Yeast one-hybrid selection of p53 zinc-fingers

### 2.1 Yeast one-hybrid selections and construction of zinc finger nucleases (ZFNs)

To create the 'bait plasmids', the target DNA sequences for the four ZFPs (z771 L, z771R, z1166L and z1166R) were cloned in single copies into pHis2.1 plasmid (Clontech), using 22bp duplex DNA oligomers. Each pair of oligonucleotides was annealed to form duplex DNA with *EcoR*I / *Spe*I compatible overhangs, and ligated into *EcoR*I / *Spe*I-cut vector pHis2.1 upstream of a minimal promoter controlling the HIS3 reporter gene (see Figure 2). This created four different bait plasmids - one for each ZFP selection. This is a departure from the conventional selection system that recommends using a plurality of target sequences. Successful application of the yeast one-hybrid system is constrained by requiring low recognition of the target sequence by endogenous transcription factors. However, the basal expression of the His3 protein, in the absence of an activating prey protein, can be repressed using 3-AT: a competitive inhibitor of the His3 enzyme (Durfee et al. (1993), Genes Dev., 7: 555-569). Thus, in the presence of increasing amounts of 3-AT, more His3 needs to be expressed to confer growth, and so the selection pressure can be fine-tuned. Before screening the libraries, we tested each target site for basal histidine expression in the absence of activating ZFPs. The amount of 3-AT needed to fully suppress basal expression varied between 25 mM for z1166L, and 75 mM for each of z771 L, z771 R, and z1166R. Consequently, 100 to 150 mM 3-AT was used for screening the libraries for DNA-binding ZFP.

pGADT7-Rec2 prey plasmid (Clontech Matchmaker One-Hybrid Library Construction and Screening Kit; Ref. 630304) was modified by removing a second *Hind*III site at 2351 bp by site directed mutagenesis (PCR primers: HIND3_Frw TACGAGGGCTTATTCAGAAcCTTTGGACTTCTTCGCCAG (SEQ ID NO: 75); HIND3_Rev CTGGCGAAGAAGTCCAAAGgTTCTGAATAAGCCCTCGTA (SEQ ID NO: 76). PCR Settings: [(97°C (30s), 46°C (30s), 68°C (5 min)] x 5; [97°C (30s), 68°C (5 min)] x 15). The PCR libraries were then introduced into *Hind*III-linearised modified prey plasmid by in-frame recombination in yeast strain Y187.

### 2.2 Yeast transformation and library interrogation

The transformation process was adapted from Clontech's Matchmaker One-Hybrid Library Construction and Screening Kit (Ref 630304; see the Manual for quantities and scales).

To interrogate the ZFP libraries, the ZFP library PCR (with the appropriate homology arms) was mixed with *Hind*III-linearised modified prey plasmid pGADT7-Rec2 (with the *Hind*III site at 2351bp removed), and the bait plasmid (pHis2.1, including one cloned ZFP-DNA-binding site in the *EcoR*I / *Spe*I sites), and simultaneously heat transformed into yeast (strain Y187), as described in the manual of the Matchmaker Kit (see also Figure 2B). This system exploits the high rate of recombination in yeast to bypass standard cloning and to allow the PCR library to fuse in frame with the prey vector in a single step. In-frame recombination in yeast strain Y187 results in a complete ZFP prey library (see Figure 2).

ZFPs with specific DNA-binding activities were recovered by plating the yeast transformations onto selective (SD) medium lacking histidine, tryptophan and leucine but supplemented with 100 mM or 150 mM 3-amino-1,2,4-triazole (3-AT). Colonies growing on this medium were re-plated on the same medium. Potential positive clones (i.e. active ZFPs) were recovered by PCR as described in Example 3.

The number of screened clones per library was calculated according to the manual of the Matchmaker Kit (Clontech) and was typically between 100,000 and 160,000 for each experiment. After incubation for 3 to 5 days at 30°C, library screening revealed a large number of positive colonies of various sizes (200 to 2000 colonies). Ultimately, 96 colonies were picked for each library, and these were replated in a 96-well pattern on selection media containing 3-AT. These colonies express ZFPs that bind to their target p53 gene sequences. The expressed ZFPs isolated, and the amino acid sequences of fingers 1 to 4 (including inter-finger linker sequences) that target the p53 genomic sequences are disclosed in Table 6. SEQ ID NO: 15 is a rationally designed variant of z1166L, which is for targeting of p53 genes in some non-human mammals. Z771 R, z771 L and z1166R ZFP sequences are suitable for targeting the p53 gene in humans and some other mammals, such as pigs, sheep and mouse.

**Table 6: Amino acid sequences of zinc finger domains 1 to 4 including linker sequences of z771 R, z771 L, z1166L and z1166R for targeting of human p53 genomic sequences (SEQ ID NOs: 11 to 14); and z1166Lp for targeting porcine p53 genomic sequences (SEQ ID NO: 15; see also Table 14). α-helices underlined from position -1 to position +6.**

| **z771R ZFP (SEQ ID NO: 11)** |
|---|
| |
| FQCRICMRNFSRSSHLSRHIRTHTGEKP FACDICGRKFARNDNRKTHTKIHTGGQRP FQCRICMRNFSRSSNLSQHIRTHTGEKP |
| |
| FACDICGRKFADNSSRIRHTKIH |
| **z771 L ZFP (SEQ ID NO: 12)** |
| FQCRICMRNFSRNSSLTNHIRTHTGEKP FACDICGRKFAATNSLIEHTKIHTGSERP FQCRICMRNFSRSCHLKTHIRTHTGEKP FACDICGRKFARSDNLKTHTKIH |
| **z1166R ZFP (SEQ ID NO: 13)** |
| FQCRICMRNFSRSDTLSRHIRTHTGEKP FACDICGRKFARKDARINHTKIHTGSERP FQCRICMRNFSRSSHLSTHIRTHTGEKP FACDICGRKFAKSDNRTTHTKIH |
| **z1166L ZFP (SEQ ID NO: 14)** |
| FQCRICMRNFSRSDNLIVHIRTHTGEKP FACDICGRKFAQNANRNTHTKIHTGSERP FQCRICMRNFSRSDALSRHIRTHTGEKP FACDICGRKFANSSNRTVHTKIH |
| **z1166Lp ZFP (SEQ ID NO: 15)** |
| FQCRICMRNFSRSSNLIVHIRTHTGEKP FACDICGRKFAQNANRNTHTKIHTGSERP FQCRICMRNFSRSSALSRHIRTHTGEKP FACDICGRKFANSSNRTVHTKIH |

The yeast one-hybrid assay of the invention was also used to select for six-finger ZFPs that were able to target the EGFP gene (see Example 10).

### Example 3

### Zinc finger nucleases

### 3.1 Zinc finger nuclease constructs and plasmids

To create ZFNs, ZFP genes were extracted from selected yeast by colony PCR (KOD polymerase; 2 mM Mg; initial lysis at 98°C, 8 min) followed by a nested PCR to introduce a T7 promoter for protein expression in a TnT T7 Quick for PCR DNA kit (Promega). The coding sequence for the *Fok*I-nuclease domain was fused in frame, via PCR, to create functional ZFNs (see Figure 3A). All primers and PCR conditions used to construct the ZFNs are given in Table 7A. The nucleic acid and amino acid sequences of the resultant ZFNs of the invention (SEQ ID NOs: 1 to 8) are displayed in Table 7B. The activity of the resulting ZFNs was verified by an *in vitro* DNA cleavage assay (described in Example 4).

ZFN coding sequences were cloned into *BamH*I / *Sal*I cut plasmid pPGK.GZF1-N (gift from Toni Cathomen; see Szczepek et al. (2007), Nat. Biotechnol. 25: 786-793). To create ZFN of the high-fidelity obligate heterodimer type (Miller et al. (2007), Nat. Biotechnol. 25: 778-785), we introduced point mutations in *Fok*I using the QuickChange mutagenesis kit (Invitrogen). The nuclease expression vector pRK5.LHA-Scel, the repair plasmid pUC.Zgfp/Rex and the target plasmid pCMV.IacZsdGFP "0-0" have been described before (Alwin et al. (2005), Mol. Ther. 12: 610-617).

The plasmids pTARGET-771 and pTARGET-1166 containing the full heterodimeric p53 target sequences at locations 771 and 1166, respectively; and plasmids pTARGET-pts771L, pTARGET-pts771R, pTARGET-pts1166L, pTARGET-pts1166R containing homodimeric palindromic target sequences were created by adding additional binding sites for the respective ZFNs. Two complementary synthetic 39-mer oligonucleotides were annealed and cloned into the *Pac*I site of pCMV.IacZsdGFP "0-0" to generate each of the target sequences indicated in Figures 1 B and 1C and Figure 4A. The ZFN target sites in each construct were verified by DNA sequencing.

**Table 7A: Primer sequences and PCR conditions for extraction of ZFPs from yeast one-hybrid assays and construction of ZFNs.**

| **Yeast colony PCR:** |
|---|
| |
| **pADH1_HA_Frw (SEQ ID NO: 77):** |
| caatatttcaagctataccaagcatacaatcaactccaagcttACCATGGCCGAGGAACGCCCCTTCCAGTGTC GCATC |
| |
| **GAL4_HA_Rev (SEQ ID NO: 78):** |
| AGGCTCGGGAATTAATTCCGCTTTATCCATCTTTGCAaagcttAGTTCACTTTTGACTAGTTGC TTCTTGTTCTGATGGATC |
| |
| **Amplification conditions:** 98 °C for 8 min, then 25 cycles of 95 °C for 30 sec, 55°C for 30 sec and 72 °C for 1 min, followed by extension at 72 °C for 3 min. |

| **Nested PCR to introduce T7 promoter:** |
|---|
| |
| **T7 Kozak Fwd_phage (SEQ ID NO: 79):** |
| TCGAGTAATACGACTCACTATAGGGAGAAACACCATAGATTGCCATGGCCGAGGAACGCCC CTTC |
| |
| **ZFP_LVKSEL_Rev fusion (SEQ ID NO: 80):** |
| CAGTTCACTTTTGACTAG |
| |
| **Amplification conditions:** 95 °C for 3 min, then 25 cycles of 95 °C for 30 sec, 55°C for 30 sec and 72 °C for 1 min, followed by extension at 72 °C for 3 min. |

| ***Fokl* PCR**: |
|---|
| |
| **Fokl_LVKSEL_Fwd_fusion (SEQ ID NO: 81):** |
| CTAGTCAAAAGTGAACTG |
| |
| **2xFLAG_REV (SEQ ID NO: 82):** |
| CTCACTACTTGTCATCGTCATCCTTGTAGTCACCGTCATGGTCTTTGTAGTCTGCGGCAAAG TTTATCTCGCCGTTATTAAATTTCCG |
| |
| **Amplification conditions:** 95 °C for 3 min, then 30 cycles of 95 °C for 30 sec, 58 °C for 30 sec and 72 °C for 1 min, followed by extension at 72 °C for 3 min. |

| **Fusion PCR of ZFP and *Fok*I:** |
|---|
| |
| **T7 Kozak Fwd_phage (SEQ ID NO: 79):** |
| TCGAGTAATACGACTCACTATAGGGAGAAACACCATAGATTGCCATGGCCGAGGAACGCCC CTTC |
| |
| **2xFLAG_REV (SEQ ID NO: 82):** |
| CTCACTACTTGTCATCGTCATCCTTGTAGTCACCGTCATGGTCTTTGTAGTCTGCGGCAAAG TTTATCTCGCCGTTATTAAATTTCCG |
| |
| **Amplification conditions:** 95 °C for 5 min, then 25 cycles of 95 °C for 20 sec, 55°C for 20 sec and 72 °C for 1 min, followed by extension at 72 °C for 3 min. |

**Table 7B: SEQ ID NOs: 1 to 8 are nucleic acid and corresponding protein ZFN sequences of z771 R, z771 L, z1166L and z1166R including the standard FokI domain for targeting human p53 gene sequences. The nucleic acid and amino acid sequences of the modified positive and negative FokI obligate dimer sequences are also shown (SEQ ID NOs: 50 to 53; Miller et al. (2007) Nat. Biotech. PMID 17603475). These sequences can be used in conjunction with the ZFPs of the invention (in place of the standard FokI sequences) to create ZFNs that are not able to form homodimers.**

| |
|---|
| **SEQ ID NO: 1 (z771 R ZFN - nucleic acid sequence 5'-3')** |
| ATGGCCCCCAAAAAGAAGCGCAAGGTCGCCGAGCGCCCCTTCCAGTGTCGCATCTGTATGCGCAATTTCAGC CGCAGCAGCCACCTGAGCCGCCACATTCGCACGCACACCGGCGAAAAGCCCTTTGCCTGTGACATTTGTGGG CGCAAGTTCGCGCGCAACGACAACCGCAAGACCCATACCAAAATTCACACCGGCGGACAGCGGCCGTTTCAG TGCAGGATTTGCATGAGGAACTTCTCCCGCAGCAGCAACCTGAGCCAGCATATCAGGACCCATACCGGGGAG AAACCTTTCGCGTGCGATATCTGCGGGAGGAAATTCGCCGACAACAGCAGCCGCATCCGCCACACGAAGATC CATCAGAACAAGAAGCAACTAGTcaaaagtgaactggaggagaagaaatctgaacttcgtcataaattgaaa tatgtgcctcatgaatatattgaattaattgaaattgccagaaattccactcaggatagaattcttgaaatg aaggtaatggaatttttttatgaaagtttatggatatagaggtaaacatttgggtggatcaaggaaaccggac ggagcaatttatactgtcggatctcctattgattacggtgtgatcgtggatactaaagcttatagcggaggt tataatctgccaattggccaagcagatgaaatgcaacgatatgtcgaagaaaatcaaacacgaaacaaacat atcaaccctaatgaatggtggaaagtctatccatcttctgtaacggaatttaagtttttatttgtgagtggt cactttaaaggaaactacaaagctcagcttacacgattaaatcatatcactaattgtaatggagctgttctt agtgtagaagagcttttaattggtggagaaatgattaaagccggcacattaaccttagaggaagtgagacgg aaatttaataacggcgagataaactttTAA |
| |
| **SEQ ID NO: 2 (z771 R ZFN - amino acid sequence N-C)** |
| MAPKKKRKVAERP FQCRICMRNFSRSSHLSRHIRTHTGEKP FACDICGRKFARNDNRKTHTKIHTGGQRP FQCRICMRNFSRSSNLSQHIRTHTGEKP FACDICGRKFADNSSRIRHTKIHQNKKQ lvkseleekkselrhklkyvpheyielieiarnstqdrilemkvmeffmkvygyrgkhlggsrkpdgaiytv gspidygvivdtkaysggynlpigqademqryveenqtrnkhinpnewwkvypssvtefkflfvsghfkgny kaqltrlnhitncngavlsveelliggemikagtltleevrrkfnngeinf* |
| **SEQ ID NO: 3 (z771 L ZFN - nucleic acid sequence 5'-3')** ATGGCCCCCAAAAAGAAGCGCAAGGTCGCCGAGGAACGCCCCTTCCAGTGTCGCATCTGTATGCGCAATTTC AGCCGCAACTCCAGCCTGACGAACCACATTCGCACGCACACCGGCGAAAAGCCCTTTGCCTGTGACATTTGT GGGCGCAAGTTCGCGGCCACCAACTCCCTCATAGAACATACCAAAATTCACACCGGATCCGAGCGCCCGTTT CAGTGCAGGATTTGCATGAGGAACTTCTCCCGCTCCTGCCACCTGAAGACCCATATCAGGACCCATACCGGG |
| GAGAAACCTTTCGCGTGCGATATCTGCGGAGGAAATTCGCCCGCAGCGACAACCTGAAGACACACACGAAG ATCCATCAGAACAAGAAGCAACTAGTcaaaagtgaactggaggagaagaaatctgaacttcgtcataaattg aaatatgtgcctcatgaatatattgaattaattgaaattgccagaaattccactcaggatagaattcttgaa atgaaggtaatggaatttttatgaaagtttatggatatagaggtaaacatttgggtggatcaaggaaaccg gacggagcaatttatactgtcggatctcctattgattacggtgtgatcgtggatactaaagcttatagcgga ggttataatctgccaattggccaagcagatgaaatgcaacgatatgtcgaagaaaatcaaacacgaaacaaa catatcaaccctaatgaatggtggaaagtctatccatcttctgtaacggaatttaagtttttatttgtgagt ggtcactttaaaggaaactacaaagctcagcttacacgattaaatcatatcactaattgtaatggagctgtt cttagtgtagaagagcttttaattggtggagaaatgattaaagccggcacattaaccttagaggaagtgaga cggaaatttaataacggcgagataaactttTAA |
| **SEQ ID NO: 4 (z771 L ZFN - amino acid sequence N-C)** |
| MAPKKKRKVAEERP FQCRICMRNFSRNSSLTNHIRTHTGEKP FACDICGRKFAATNSLIEHTKIHTGSERP FQCRICMRNFSRSCHLKTHIRTHTGEKP FACDICGRKFARSDNLKTHTKIHQNKKQ lvkseleekkselrhklkyvpheyielieiarnstqdrilemkvmeffmkvygyrgkhlggsrkpdgaiytv gspidygvivdtkaysggynlpigqademqryveenqtrnkhinpnewwkvypssvtefkflfvsghfkgny kaqltrlnhitncngavlsveelliggemikagtltleevrrkfnngeinf* |
| **SEQ ID NO: 5 (z1166R ZFN - nucleic acid sequence 5'-3')** |
| ATGGCCCCCAAAAAGAAGCGCAAGGTCGCCGAGGAACGCCCCTTCCAGTGTCGCATCTGTATGCGCAATTTC AGCAGGAGCGACACCCTGAGCAGGCACATTCGCACGCACACCGGCGAAAAGCCCTTTGCCTGTGACATTTGT GGGCGCAAGTTCGCGAGGAAGGACGCCCGCATCAACCATACCAAAATTCACACCGGATCTGAGCGCCCGTTT CAGTGCAGGATTTGCATGAGGAACTTCTCCAGGAGCAGCCACCTGAGCACCCATATCAGGACCCATACCGGG GAGAAACCTTTCGCGTGCGATATCTGCGGGAGGAAATTCGCCAAGAGCGACAACCGCACCACGCACACGAAG ATCCATCAGAACAAGAAGCAACTAGTcaaaagtgaactggaggagaagaaatctgaacttcgtcataaattg aaatatgtgcctcatgaatatattgaattaattgaaattgccagaaattccactcaggatagaattcttgaa atgaaggtaatggaatttttttatgaaagtttatggatatagaggtaaacatttgggtggatcaaggaaaccg gacggagcaatttatactgtcggatctcctattgattacggtgtgatcgtggatactaaagcttatagcgga ggttataatctgccaattggccaagcagatgaaatgcaacgatatgtcgaagaaaatcaaacacgaaacaaa catatcaaccctaatgaatggtggaaagtctatccatcttctgtaacggaatttaagtttttatttgtgagt ggtcactttaaaggaaactacaaagctcagcttacacgattaaatcatatcactaattgtaatggagctgtt cttagtgtagaagagcttttaattggtggagaaatgattaaagccggcacattaaccttagaggaagtgaga cggaaatttaataacggcgagataaactttTAA |
| **SEQ ID NO: 6 (z1166R ZFN - amino acid sequence N-C)** |
| MAPKKKRKVAEERP FQCRICMRNFSRSDTLSRHIRTHTGEKP FACDICGRKFARKDARINHTKIHTGSERP FQCRICMRNFSRSSHLSTHIRTHTGEKP FACDICGRKFAKSDNRTTHTKIHQNKKQ lvkseleekkselrhklkyvpheyielieiarnstqdrilemkvmeffmkvygyrgkhlggsrkpdgaiytv gspidygvivdtkaysggynlpigqademqryveenqtrnkhinpnewwkvypssvtefkflfvsghfkgny kaqltrlnhitncngavlsveelliggemikagtltleevrrkfnngeinf* |
| **SEQ ID NO: 7 (z1166L ZFN - nucleic acid sequence 5'-3')** |
| ATGGCCCCCAAAAAGAAGCGCAAGGTCGCCGAGGAACGCCCCTTCCAGTGTCGCATCTGTATGCGCAATTTC AGCCGCAGCGACAACCTGATAGTGCACATTCGCACGCACACCGGCGAAAAGCCCTTTGCCTGTGACATTTGT GGGCGCAAGTTCGCGCAGAACGCCAACCGGAACACGCATACCAAAATTCACACCGGATCTGAGCGCCCGTTT CAGTGCAGGATTTGCATGAGGAACTTCTCCCGGTCCGACGCCCTGAGCCGCCATATCAGGACCCATACCGGG GAGAAACCTTTCGCGTGCGATATCTGCGGGAGGAAATTCGCCAACAGCAGCAACCGGACAGTGCACACGAAG ATCCATCAGAACAAGAAGCAACTAGTcaaaagtgaactggaggagaagaaatctgaacttcgtcataaattg aaatatgtgcctcatgaatatattgaattaattgaaattgccagaaattccactcaggatagaattcttgaa atgaaggtaatggaatttttttatgaaagtttatggatatagaggtaaacatttgggtggatcaaggaaaccg gacggagcaatttatactgtcggatctcctattgattacggtgtgatcgtggatactaaagcttatagcgga ggttataatctgccaattggccaagcagatgaaatgcaacgatatgtcgaagaaaatcaaacacgaaacaaa catatcaaccctaatgaatggtggaaagtctatccatcttctgtaacggaatttaagtttttatttgtgagt |
| ggtcactttaaaggaaactacaaagctcagcttacacgattaaatcatatcactaattgtaatggagctgtt cttagtgtagaagagcttttaattggtggagaaatgattaaagccggcacattaaccttagaggaagtgaga cggaaatttaataacggcgagataaactttTAA |
| **SEQ ID NO: 8 (z1166L ZFN - amino acid sequence N-C)** |
| MAPKKKRKVAEERP FQCRICMRNFSRSDNLIVHIRTHTGEKP FACDICGRKFAQNANRNTHTKIHTGSERP FQCRICMRNFSRSDALSRHIRTHTGEKP FACDICGRKFANSSNRTVHTKIHQNKKQ lvkseleekkselrhklkyvpheyielieiarnstqdrilemkvmeffmkvygyrgkhlggsrkpdgaiytv gspidygvivdtkaysggynlpigqademqryveenqtrnkhinpnewwkvypssvtefkflfvsghfkgny kaqltrlnhitncngavlsveelligemikagtltleevrrkfnngeinf* |
| **Obligate heterodimer Fokl nucleic acid sequence (SEQ ID NO: 50) Negatively-charged monomer nucleic acid sequence (5'-3')** |
| ctagtcaaaagtgaactggaggagaagaaatctgaacttcgtcataaattgaaatatgtgcctcatgaatat attgaattaattgaaattgccagaaattccactcaggatagaattcttgaaatgaaggtaatggaatttttt atgaaagtttatggatatagaggtaaacatttgggtggatcaaggaaaccggacggagcaatttatactgtc ggatctcctattgattacggtgtgatcgtggatactaaagcttatagcggaggttataatctgccaattggc caagcagatgaaatggagcgatatgtcgaagaaaatcaaacacgaaacaaacatctcaaccctaatgaatgg tggaaagtctatccatcttctgtaacggaatttaagtttttatttgtgagtggtcactttaaaggaaactac aaagctcagcttacacgattaaatcatatcactaattgtaatggagctgttcttagtgtagaagagctttta attggtggagaaatgattaaagccggcacattaaccttagaggaagtgagacggaaatttaataacggcgag ataaacttt*TAA* |
| **Obligate heterodimer Fokl amino acid sequence (SEQ ID NO: 51) Negatively-charged monomer amino acid sequence (N-C)** |
| lvkseleekkselrhklkyvpheyielieiarnstqdrilemkvmeffmkvygyrgkhlggsrkpdgaiytv gspidygvivdtkaysggynlpigqademeryveenqtrnkhlnpnewwkvypssvtefkflfvsghfkgny kaqltrlnhitncngavlsveelliggemikagtltleevrrkfnngeinf* |
| **Obligate heterodimer Fokl nucleic acid sequence (SEQ ID NO: 52) Positively-charged monomer nucleic acid sequence (5'-3')** |
| ctagtcaaaagtgaactggaggagaagaaatctgaacttcgtcataaattgaaatatgtgcctcatgaatat attgaattaattgaaattgccagaaattccactcaggatagaattcttgaaatgaaggtaatggaattttt atgaaagtttatggatatagaggtaaacatttgggtggatcaaggaaaccggacggagcaatttatactgtc ggatctcctattgattacggtgtgatcgtggatactaaagcttatagcggaggttataatctgccaattggc caagcagatgaaatgcaacgatatgtcaaagaaaatcaaacacgaaacaaacatatcaaccctaatgaatgg tggaaagtctatccatcttctgtaacggaatttaagtttttatttgtgagtggtcactttaaaggaaactac aaagctcagcttacacgattaaatcataagactaattgtaatggagctgttcttagtgtagaagagcttta attggtggagaaatgattaaagccggcacattaaccttagaggaagtgagacggaaatttaataacggcgag ataaacttt***TAA*** |
| **Obligate heterodimer Fokl amino acid sequence (SEQ ID NO: 53) Positively-charged monomer amino acid sequence (N-C)** |
| Lvkseleekkselrhklkyvpheyielieiarnstqdrilemkvmeffmkvygyrgkhlggsrkpdgaiytv Gspidygvivdtkaysggynlpigqademqryvkenqtrnkhinpnewwkvypssvtefkflfvsghfkgny kaqltrlnhktncngavlsveelliggemikagtltleevrrkfnngeinf* |
| **Key:** |
| a. ***ATG*** (nucleic acid sequences) = start codon |
| b. **PKKKRKV** (peptide sequences) = SV40 nuclear localisation signal |
| c. ZFP peptide sequence α-helices underlined from position -1 to position +6 |
| d. ACTAGT = *Spe*I site |
| e. *Fok*I coding region (lower case in nucleic acid and amino acid sequences): normal (wild-type) *Fok*I illustrated in ZFN sequences; and *Fok*I obligate dimer sequences shown separately - can be used interchangeably according to preference |
| f. ***TAA*** = stop codon (not part of SEQ ID NOs) |

### Example 4

### In vitro DNA cleavage of p53 target sites

To validate the yeast one-hybrid assay-based protein-DNA interactions, ZFP genes were recovered from yeast colonies by PCR, while introducing a T7 promoter for subsequent expression. After a PCR-based fusion to the *Fok*I domain (described in Example 3), the full-length ZFN candidates were expressed *in vitro* and tested for specific cutting activity in an *in vitro* cleavage assay (Figure 3B and Figure 4).

### 4.1 In vitro DNA cleavage assay

A 154bp linear target DNA was amplified by PCR from pTARGET plasmids with primers Target_seqL1 (5'-accagttggtctggtgtcaa-3'; SEQ ID NO: 83) and Target_seqR1 (5'-ctgaacttgtggccgtttac-3'; SEQ ID NO: 84). DNA templates for *in vitro* expression of ZFNs were amplified by PCR from positive yeast colonies or from ZFN expression vectors using primers T7kozak_FWD (5'-tcgagtaatacgactcactatagggagaaacaccatagattgccatggccgagcgccccttc-3'; SEQ ID NO: 85) and UnivQQR_R_NotI (5'-aaggaaaaaagcggccgcaaaaggaaaaggatcctcattaaaagtttatc-3'; SEQ ID NO: 86). All PCR reactions were purified with the QiaQuick PCR Purification Kit (Qiagen).

The ZFNs were expressed from 100 ng T7-PCR templates, using the TnT-Quick coupled transcription-translation system (Promega), according to the manufacturer's instructions, except that ZnCl₂ was added to a final concentration of 4 mM.

To analyse ZFN activities, TnT reactions were mixed with 200 ng of target DNA and diluted with *Fok*I cleavage buffer to a final concentration of: 20 mM Bis-Tris-propane pH7.0, 100 mM NaCl, 5 mM MgCl₂, 0.1 mM ZnCl₂, 5 mM DTT, 1.8% (vol/vol) glycerol, 20 µg/ml poly-d(I-C), 0.1 mg/ml BSA. After incubation for 5 to 6 hrs at 30°C, the reactions were cleaned with a PCR Purification Kit (Qiagen) or, alternatively, were treated with 1 ml RNase A (10 mg/ml, Qiagen) for 30 minutes at 37°C and 1 ml Proteinase K (20 mg/ml, Qiagen) for 1 hour at 37°C. Samples were then analysed on a 1.8% agarose gel, with ethidium bromide staining.

From the results several four-finger anti-p53 ZFNs that bind and cleave their palindromic target sites efficiently *in vitro* were identified (z771 L, 4 clones; z771 R, 1 clone; z1166R, 3 clones; and z1166L, 1 clone), and the α-helix sequences of fingers 1 to 4 of the most active ZFNs are given in Table 8. The sequences of the z771 L, z1166R and z1166L ZFNs selected from the libraries are each different to their corresponding rationally designed ZFP, and have markedly improved cutting activity. In fact, whereas the rationally designed ZFN sequences for the 771 L and 1166L target sites were inactive in the *in vitro* DNA cleavage assay (and 1166R was poorly active), all of the library-selected clones were active. Only z771 R, whose rational design was already very active, was not improved further after the yeast one-hybrid screen; therefore the original z771 R design was subsequently used. The italicised 'C' residue in finger 3 of z771 L indicates a spontaneous random mutation. ZFPs with and without this unintended mutation can be used.

From the selected clones, the most active ZFNs were similarly tested in homo- and heterodimer pairs, for cleaving either palindromic test sites or full DNA target sites (in the configuration shown in Figure 1C; L=left; R=right) shown in Figure 4A. The L/L and R/R-homodimers only cut their respective palindromic target sites but not the unspecific targets (see Figure 4B; left panel). Conversely, the full heterodimer targets (ts771 and ts1166) were cleaved only by the correct combination of z771 L/R or z1166L/R ZFNs, respectively, showing that a full pair of ZFNs was required for cutting the desired heterodimeric target site (see Figure 4B; right panel).

**Table 8: ZFN DNA-binding α-helix sequences of the most active ZFNs selected using yeast one-hybrid assays.**

| **Zinc** | **Target Binding Site (3' to 5')** | α**-helix amino acids sequences (F1, F2, F3, F4)** |
|---|---|---|
| **Finger** | | |
| **Nuclease** | | |
| z771R | GGG GAC GAA CCG | RSSHLSR, RNDNRKT, RSSNLSQ, DNSSRIR |
| Z771L | GTA CTC GGT GAC | RNSSLTN, ATNSLIE, RSCHLKT, RSDNLKT |
| Z1166R | GTG GTA GGT GAT | RSDTLSR, RKDARIN, RSSHLST, KSDNRTT |
| Z1166L | GAC AAT GTG TAC | RSDNLIV, QNANRNT, RSDALSR, NSSNRTV |

### Example 5

### Episomal gene repair assay

To evaluate whether the selected potential p53-specific ZFNs (z771 and z1166, whose sequences are indicated above) could promote homologous recombination or gene repair *in vivo,* they were first tested in a plasmid-based EGFP repair assay, developed by the Cathomen lab (Alwin et al. (2005), Mol. Ther. 12: 610-617), as depicted in Figure 5A.

### 5.1 Cell lines and culturing

The wt p53 cell line HEK293T was maintained in DMEM and the mutant p53 cell line SF268 (kindly provided by A. Carnero) was cultured in RPM11640 at 37°C, in 5% CO₂. All media were supplemented with 10% FCS, 100 units/ml penicillin and 100 mg/ml Streptomycin.

### 5.2 Episomal gene repair

The protocol described in Alwin *et al.* (2005), was generally followed for the episomal gene repair assays.

Briefly, HEK293T cells in 24-well plates were transfected by Lipofectamine 2000 (Invitrogen) with 100 ng of target plasmid, 300 ng of repair plasmid pUC.Zgfp/REx or repair control pUC.REx, and 100 ng of PGK-driven ZFN expression vectors, I-Scel (pRK5.LHA-Scel) or control vector pUC19. The amount of DNA was kept constant by adding pUC19 to 500 ng. After 48 hrs, 5x10⁵ cells were analysed by flow cytometry (FACSCanto, BD Bioscience) to determine the percentage of EGFP- and REx-positive cells, and the results are shown in Figure 5B. Statistical significance was determined using the Student t-test, and error bars are one standard error based on three biological replicates.

In this assay, a promoterless EGFP sequence, with lacZ gene homology arm, is used to repair a 5'-truncated (non-fluorescent) EGFP gene, and the process is stimulated by cleavage with the appropriate nuclease (see Figure 5A). The target plasmid harbours an 18bp recognition binding site for the meganuclease I-Scel, which serves as a positive control, in combination with a target site for the anti-p53 ZFNs, z771 or z1166. The system is thus designed to restore EGFP expression by generating a lacZ-EGFP fusion protein upon nuclease-induced homologous recombination. The expression of the red-fluorescent protein DsRed-Express (REx), from a gene cassette located on the repair plasmid labels transfected cells (Alwin *et al.* 2005).

To test the activity and efficiency of the ZFNs, HEK293T cells were transfected either with target plasmid "ts771" or "ts1166", the repair plasmid and the respective PGK-driven ZFN pairs z771 L/R and z1166L/R. 48 hours after transfection, the percentage of green and red cells was assessed by flow cytometry and the results are shown in Figure 5B. The GFP repair assay revealed that the ZFNs showed the strongest activity when expressed in appropriate pairs to form heterodimers specific for the heterodimeric target sites. The repair efficiencies were comparable with that of the benchmark control, the meganuclease I-Scel: i.e. z771L/R, cleavage efficiency 20.9%; z1166L/R, cleavage efficiency 24.5%; and I-Scel, cleavage efficiency 20% to 25%.

As shown in Figure 5B, some gene repair was observed when z771 L (12% efficiency) and z771 R (15% efficiency) were expressed alone. This non-specific cleavage may be due to DNA-binding by one ZFN monomer, followed by non-specific *Fok*I dimerisation. This was only seen for the nucleases with stronger activity; for example, z1166L alone did not generate appreciable activation of the EGFP gene. Recent advances in generating obligate heterodimer ZFN have demonstrated that it is possible to remove this non-specific nuclease activity (Miller et al. (2007), Nat. Biotechnol. 25: 778-785; and Szczepek et al. (2007), Nat. Biotechnol. 25: 786-793), and so obligate heterodimer mutants were used in the downstream assays in order to reduce any non-specific activity of the ZFNs.

Notably, the EGFP-background level in the absence of any nuclease was relatively high, representing spontaneous homologous recombination events in this episomal system (8.2% for target plasmid ts771 and 6.4% for ts1166). Nonetheless, the nuclease-induced signals were highly reproducible and statistically significant (z771 L/R, p<0.002; z1166L/R, p<0.001). Therefore this assay is a good way of validating ZFN for cellular use.

Thus, by optimising the promoter for the ZFNs (as discussed further in Example 8 below) and adding a nuclear localisation signal to the ZFN, good induction of EGFP repair was achieved by the anti-p53 ZFNs.

### Example 6

### Chromosomal targeting of p53 gene by ZFNs.

To determine whether the custom-built ZFNs would also work on a genomic level, HEK293T cells were transfected with PGK-driven ZFN expression vectors against the target sites z771 and z1166, together with homology repair plasmids for a region of the p53 gene. Because p53 cancer mutations are localised to one region of hotspots (see Figure 1A), repair plasmids covering the majority of hotspots could be synthesised, containing either 1.35 kb DNA homologous to the genomic p53 locus between exons 6 and 8, or 1.78 kb DNA homologous to the genomic p53 locus between exons 5 and 8 (repair plasmid sequences are shown in Table 9).

### 6.1 Construction of donor plasmids

To avoid cutting of the donor plasmids by the respective ZFNs, the p53 gene repair sequences within the donor plasmids were mutated within the z771 and z1166 target sites. These modifications further constitute a 'barcode', which allows detection of genomic recombination of the plasmid by PCR analysis (see Figure 6). The z771 site is within an intron sequence, whereas the z1166 site is within an exon. Therefore, the modified barcodes in the z1166 sequence were carefully chosen silent mutations that do not alter the p53 amino acid sequence.

**Table 9: Donor plasmid sequences for p53 gene repair**

| |
|---|
| Homo sapiens (human) Build 36.3 (Current)The Human Genome NW_001838403 Homo sapiens chromosome 17 genomic contig, alternate assembly (based on HuRef SCAF_1103279188371) |
| **p53 repair sequence encompassing exons 6 to 8 of p53 (SEQ ID NO: 54) pUC57_truncated_p53_repair matrix (used in 293T, SF268)** |
| 1                                                                      72 TTGCGTGTGGAGTATTTGGATGACAGAAACACTTTTCGACATAGTGTGGTGGTGCCCTATGAGCCGCCTGAG GTCTGGTTTGCAACTGGGGTCTCTGGGAGGAGGGGTTAAGGGTGGTTGTCAGTGGCCCTCCGGGTGAGCAGT AGGGGGGCTTTCTCCTGCTGCTTATTTGACCTCCCTATAACCCCATGAGATGTGCAAAGTAAATGGGTTTAA CTATTGCACAGTTGAAAAAACTGAAGCTTACAGAGGCTAAGGGCCTccccctgcttggcTGGCTGCTCGAGCT AGCGcagtggctcatgCCTGTAATCCCAGCACTTTGGGAGGCCAAGGCAGGCGGATCACGAGGTTGGGAGAT CGAGACCATCCTGGCTAACGGTGAAACCCCGTCTCTACTGAAAAATACAAAAAAAAATTAGCCGGGCGTGGT GCTGGGCACCTGTAGTCCCAGCTACTCGGGAGGCTGAGGAAGGAGAATGGCGTGAACCTGGGCGGTGGAGCT TGCAGTGAGCTGAGATCACGCCACTGCACTCCAGCCTGGGCGACAGAGCGAGATTCCATCTCAAAAAAAAAA AAAAAAGGCCTCCCCTGCTTGCCACAGGTCTCCCCAAGGCGCACTGGCCTCATCTTGGGCCTGTGTTATCTC CTAGGTTGGCTCTGACTGTACGacGatTcaTtacaactaTatgtgCaaTTCcTCCTGCATGGGCGGCATGAA CCGGAGGCCCATCCTCACCATCATCACACTGGAAGACTCCAGGTCAGGAGCCACTTGCCACCCTGCACACTG GCCTGCTGTGCCCCAGCCTCTGCTTGCCTCTGACCCCTGGGCCCACCTCTTACCGATTTCTTCCATACTACT ACCCATCCACCTCTCATCACATCCCCGGCGGGGAATCTCCTTACTGCTCCCACTCAGTTTTCTTTTCTCTGG CTTTGGGACCTCTTAACCTGTGGCTTCTCCTCCACCTACCTGGAGCTGGAGCTTAGGCTCCAGAAAGGACAA GGGTGGTTGGGAGTAGATGGAGCCTGGTTTTTAAATGGGACAGGTAGGACCTGATTTCCTTACTGCCTCTT GCTTCTCTTTTCCTATCCTGAGTAGTGGTAATCTACTGGGACGGAACAGCTTTGAGGTGCGTGTTTGTGCCT GTCCTGGGAGAGACCGGCGCACAGAGGAAGAGAATCTCCGCAAGAAAGGGGAGCCTCACCACGAGCTGCCCC CAGGGAGCACTAAGCGAGGTAAGCAAGCAGGACAAGAAGCGGTGGAGGAGACCAAGGGTGCAGTTATGCCTC AGATTCACTTTTATCACCTTTCCTTGCCTCTTTCCTAGCACTGCCCAACAAC 1348 |
| **p53 repair sequence encompassing exons 5 to 8 of p53 (SEQ ID NO: 55) pUC57_p53_repair matrix_771(used in 293T)** |
| 1                                                                      72 AAGAAAAGCTCCTGAGGTGTAGACGCCAACTCTCTCTAGCTCGCTAGTGGGTTGCAGGAGGTGCTTACACAT GTTTGTTTCTTTGCTGCCGTCTTCCAGTTGCTTTATCTGTTCACTTGTGCCCTGACTTTCAACTCTGTCTCC TTCCTCTTCCTACAGTACTCCCCTGCCCTCAACAAGATGTTTTGCCAACTGGCCAAGACCTGCCCTGTGCAG CTGTGGGTTGATTCCACACCCCCGCCCGGCACCCGCGTCCGCGCCATGGCCATCTACAAGCAGTCACAGCAC ATGACGGAGGTTGTGAGGCGCTGCCCCCACCATGAGCGCTGCTCAGATAGCGATGGTGAGCAGCTGGGGCTG GAGAGACGACAGGGCTGGTTGCCCAGGGTCCCCAGGCCTCTGATTCCTCACTGATTGCTCTTAGGTCTGGCC CCTCCTCAGCATCTTATCCGAGTGGAAGGAAATTTGCGTGTGGAGTATTTGGATGACAGAAACACTTTTCGA CATAGTGTGGTGGTGCCCTATGAGCCGCCTGAGGTCTGGTTTGCAACTGGGGTCTCTGGGAGGAGGGGTTAA GGGTGGTTGTCAGTGGCCCTCCGGGTGAGCAGTAGGGGGGCTTTCTCCTGCTGCTTATTTGACCTCCCTATA ACCCCATGAGATGTGCAAAGTAAATGGGTTTAACTATTGCACAGTTGAAAAAACTGAAGCTTACAGAGGCTA AGGGCCTccGTtgTttggTtgggcgcaCAggAtcTACCCTGTAATCCCAGCACTTTGGGAGGCCAAGGCAGG CGGATCACGAGGTTGGGAGATCGAGACCATCCTGGCTAACGGTGAAACCCCGTCTCTACTGAAAAATACAAA AAAAATTAGCCGGGCGTGGTGCTGGGCACCTGTAGTCCCAGCTACTCGGGAGGCTGAGGAAGGAGAATGGC GTGAACCTGGGCGGTGGAGCTTGCAGTGAGCTGAGATCACGCCACTGCACTCCAGCCTGGGCGACAGAGCGA GATTCCATCTCAAAAAAAAAAAAAAAAGGCCTCCCCTGCTTGCCACAGGTCTCCCCAAGGCGCACTGGCCTC ATCTTGGGCCTGTGTTATCTCCTAGGTTGGCTCTGACTGTACcaccatccactaCAACTAcatgtgtaacag TTCCTGCATGGGCGGCATGAACCGGAGGCCCATCCTCACCATCATCACACTGGAAGACTCCAGGTCAGGAGC CACTTGCCACCCTGCACACTGGCCTGCTGTGCCCCAGCCTCTGCTTGCCTCTGACCCCTGGGCCCACCTCTT ACCGATTTCTTCCATACTACTACCCATCCACCTCTCATCACATCCCCGGCGGGAATCTCCTTACTGCTCCC ACTCAGTTTTCTTTTCTCTGGCTTTGGGACCTCTTAACCTGTGGCTTCTCCTCCACCTACCTGGAGCTGGAG CTTAGGCTCCAGAAAGGACAAGGGTGGTTGGGAGTAGATGGAGCCTGGTTTTTTAAATGGGACAGGTAGGAC CTGATTTCCTTACTGCCTCTTGCTTCTCTTTTCCTATCCTGAGTAGTGGTAATCTACTGGGACGGAACAGCT TTGAGGTGCGTGTTTGTGCCTGTCCTGGGAGAGACCGGCGCACAGAGGAAGAGAATCTCCGCAAGAAAGGGG AGCCTCACCACGAGCTGCCCCCAGGGAGCACTAAGCGAGGTAAGCAAGCAGGACAAGAAGCGGTGGAGGAGA CCAAGGGTGCAGTTATGCCTCAGATTCACTTTTATCACCTTTCCTTGCCTCTT 1781 |
| **Key:** |
| a. 771 target sites shown in bold, underlined, lowercase, underlined (with silent "barcode" mutations within the 771 binding site highlighted in bold, underlined, uppercase) |
| b. 1166 target sites in bold, lowercase (with silent "barcode" mutations within the 1166 binding site highlighted in bold, uppercase) |
| c. exons shown in grey (the 3' 20 bases of SEQ ID NO: 54 represent the start of p53 exon 9) |
| d. mutation hot spots in p53 gene shown in boxes Hotspots: ATG=133; CCG=152; CGC=175*; CGA=213; AAC=235; GGC=245*; CGG=248*; AGG=249*; CGT=273*; CGG=282 |
| e. *Xho*I sites in uppercase, italics |

### 6.2 PCR analysis of genomic recombination of p53-donor plasmid

2x10⁶ K562 cells were transfected with 5 µg of each ZFN expression construct, and 10 µg of the donor plasmid, using the single-cuvette format of the Nucleofector Kit V (Lonza), according to the manufacturer's protocol. HEK293T, SF268, and BT-549 cell lines were transfected in 6-well plates by Lipofectamine 2000 (Invitrogen), with 5 mg repair matrix donor plasmid, and 2 µg of ZFN expression vectors, or empty control vector. An EGFP expression vector (0.5 µg) was co-transfected in all the samples (except K5652 cells) to identify transfected cells. Six days after transfection, 1x10⁵ GFP-positive cells per sample were collected by fluorescence activated cell sorting (FACS), and genomic DNA was isolated from cells with the DNA Blood and Tissue Kit (Qiagen).

Genomic recombination is detected using external PCR primers to amplify the p53 genomic regions, followed by semi-nested PCR, with a forward primer specific for the modified DNA sequence / barcode, and an external genomic reverse primer. First, 30 to 300 ng genomic DNA was subjected to PCR with high fidelity enzymes KOD Hot Start (Novagen) or Accuprime Taq DNA Polymerase (Invitrogen). PCR products were column purified with the PCR purification kit (Qiagen) and eluted in 30 µl H₂0. A fraction of the resulting amplicons was amplified by nested PCR with Taq polymerase using primers designed to discriminate between wild-type and integrated modified p53 sequence. Nested PCR amplicons were resolved on a 1.5% agarose gel and visualised by ethidium bromide staining. All primers and conditions are given in Table 10.

**Table 10: PCR primers and conditions for assessing genomic integration of p53 donor sequence**

| |
|---|
| **Genomic PCR (HEK293T, SF268, and BT-549 cell lines)** |
| **p53_diagnostic_fwd (SEQ ID NO: 87):** |
| GTCTGGCCCCTCCTCAGCATCTTATCCGAG |
| **p53_diagnostic_external_reverse (SEQ ID NO: 88):** |
| CAAGACTTAGTACCTGAAGGGTG |
| **Amplification conditions**: 95 °C for 3 min, then 25 cycles of 95 °C for 30 sec, 56°C for 30 sec and 68 °C for 1 min, followed by extension at 68 °C for 3 min. The expected amplicon length is 1448 bp. |
| **Genomic PCR (K562 cell line)** |
| **771F_wt (SEQ ID NO: 89):** |
| CCCCTGCTTGGCTGGGCGCAGTGGCTCATG |
| **Diagnostic_external_R (SEQ ID NO: 90):** |
| GGTTTCTTCTTTGGCTGGGGAGAGGAGC |
| **Amplification conditions:** 95 °C for 5 min, then 10 cycles of 95 °C for 30 sec (decrease 0.5 °C every cycle), 68 °C for 1 min 30 sec then 10 cycles of 95 °C for 30 sec, 58°C for 30 sec and 72 °C for 1 min 20 sec, followed by extension at 72 °C for 5 min. The expected amplicon length is 1105 bp (wt) and 1117 bp (modified). |
| **Discrimination between wild type and integrated modified p53 sequence (Nested "barcode" PCR)** |
| *A. z1166 site in K562 cells:* |
| **1166_Xho_I_diag_fwd_alternate (K562) (SEQ ID NO: 91):** |
| ACCACCATCCACTACAACTGCTCGAGC |
| **Diagnostic_external_R (SEQ ID NO: 92):** |
| GGTTTCTTCTTTGGCTGGGGAGAGGAGC |
| *B. z1166 site in HEK293T and SF268 cells:* |
| **1166F_repaired_short1 (SEQ ID NO: 93):** |
| GacGatTcaTtacaactaTatgtgCaaTTC |
| **p53_diagnostic_external_reverse (SEQ ID NO: 88):** |
| CAAGACTTAGTACCTGAAGGGTG |
| *C. z771 site in HEK293T cells:* |
| **771 F_repair (SEQ ID NO: 94):** |
| ccGTtgTttggTtgggcgcaCAggAtcTAC |
| **p53_diagnostic_external_reverse (SEQ ID NO: 88):** |
| CAAGACTTAGTACCTGAAGGGTG |
| **Amplification conditions:** 95 °C for 3 min, then 35 (wt control: 22) cycles of 95 °C for 30 sec, 60 °C for 25 sec and 72 °C for 1 min, followed by extension at 72 °C for 3 min. |

### 6.3 Genome editing in a model cell line - HEK293T

For analysis of genome editing, genomic DNA was prepared from a pool of treated HEK293T cells (cultured as previously described), 3 to 6 days after transfection. Targeted donor recombination at the p53 locus was demonstrated by semi-nested PCR, as described above, and the results are shown in Figure 6B. When measuring wild-type alleles, as expected, there was no major difference with or without ZFN treatment because these templates were in large excess. However, when looking for mutant barcoded alleles, there was no detectable genomic insertion in the absence of ZFN, whereas ZFN treatment clearly induced the presence of modified cells

The results demonstrated that both ZFN pairs were able to induce recombination of the donor plasmid with the chromosomal p53 gene, whereas control cells, transfected only with donor plasmid (and an empty PGK expression vector), did not show any sign of donor plasmid recombination. Although ZFN-specific recombination was seen with both repair matrices, the shorter repair construct of the exon 6 to 8 donor plasmid gave the clearest results. This may have been because the external genomic primer sequence used for this assay was particularly specific for the target sequence at the start of exon 6. Therefore, this donor plasmid was mainly used in subsequent assays.

### 6.4 Restoration of wt-p53 status in human glioblastoma cancer cell line SF268

Next, the z1166 ZFNs (i.e. z1166L and z1166R) were tested to determine whether they could induce the restoration of wt-p53 status in the human glioblastoma cancer cell line SF268. This cell line harbours a single missense mutation (cgt to cat) at codon 273 in the core domain of p53 (Chen et al. (1995), Cancer Genet. Cytogenet. 82: 106-115). The SF268 cells were also transfected with PGK-driven z1166-ZFN expression vectors and a donor plasmid with wild type p53 sequence at codon 273.

The treated cells were analysed by PCR, as previously described for the HEK293T cells, and were found to also show site-specific recombination of the donor plasmid with the p53 gene, only when co-transfected with functional ZFNs (see Figure 6C).

As the point mutation in SF268 cells is located in exon 8, approximately 450bp downstream of the z1166 target site, PCR amplicons obtained with recombination-specific primers were subcloned by Topo-TA cloning; 10 clones were sequenced to check for downstream modification at the mutated R273H codon. All the clones showed a restoration of the p53 wild-type sequence at codon position 273, as demonstrated by the boxed residue in the sequence given in Figure 6D. These results thus indicated that homology-directed repair occurred over 450bp downstream of the ZFN-induced double-stranded break.

### Example 7

### Measuring gene repair and non-homologous end-joining by deep sequencing

Next generation sequencing is an ideal tool to quantify the effects of ZFNs on cells. DNA sequence read lengths from genomic PCR products can routinely give over 20 million sequences (approximately 100 bp length) in a single run; and primer barcoding can be used to mix different samples together, allowing subsequent data deconvolution. We therefore developed a Solexa-Illumina method to sequence p53 locus genomic PCRs at the site targeted by z1166. The short insertions and deletions caused by non-homologous end-joining (NHEJ) could, therefore, be measured after a nuclease-induced double-stranded break (Meng et al. (2008), Nat. Biotechnol. 26: 695-701). The rate of wild-type sequence insertion from a 'barcoded' donor plasmid (with wild-type protein-coding sequence) was also measured.

### 7.1 Illumina sequencing and basecalling - sample Preparation

The frequency of targeted gene modification in ZFN-treated pools of cells was determined by Illumina's Solexa deep sequencing platform. Transfections, genomic DNA preparation and genomic PCR reactions were performed as previously described. There were two protocols depending on the sequencing read length required.

### 7.1.1 Preparation of DNA products for short read length - Mme1 digestion

The method involved 2 rounds of PCR: one external genomic PCR and one internal PCR to introduce 3bp sequencing barcodes and an Mmel cleavage site.

PCR products were excised from agarose gels, to avoid contamination with transfected donor plasmid, were column purified with a PCR purification kit (Qiagen) and eluted in 30 µl H₂0. 10 ng of the PCR products were amplified by nested PCR with KOD HiFi polymerase, with Solexa primers flanking the 1166 target region, approximately 90bp up-or down-stream. The primers contained 3bp barcodes in order to distinguish between individual samples in a pooled Solexa lane containing all eight PCRs (see Table 11 for primer sequences and conditions). PCR amplicons were column purified for sequencing adapter ligation with Illumina protocols.

PCR products were digested with *Mme*I (0.5 µl BSA, 5 µl NEB4, 20 µl DNA (1.5 µg), 1 µl SAM 50X (dilute 32 mM stock: 1 in 12.8 µl H₂O to make 50X), 0.75 µl *Mme*I, 22.75 µl H₂O: incubate 37°C / 15 mins) and the digested products purified. For Solexa sequencing single-end reads using the manufacturer's protocol were carried out on a Genome Analyser II machine.

### 7.1.2 Preparation of DNA products for long read length

PCR amplicons from 7.1.1 before *Mme*I digestion are subjected to a second nested PCR reaction. 50 ng PCR template was amplified using Soiexa1_F and Solexa_R (forward and reverse) primers (see Table 11). PCR amplicons were column purified for sequencing adapter ligation with Illumina protocols. To avoid PCR cross-over artefacts, barcoded samples were amplified and prepared for Solexa sequencing separately, mixing was carried out just prior to loading onto flow cells (see below).

**Table 11: Deep sequencing barcode primer sequences and PCR conditions**

| *A. Forward primers:* |
|---|
| **new1166_solexa_F_ATC (SEQ ID NO: 95):** |
| TCTTGGtccgacGGCGCACTGGCCTCATCTTAGGCATCTGTTATCTCCTAGGTTGGCTCTG |
| **new1166_solexa_F_TTG (SEQ ID NO: 96):** |
| TCTTGGtccgacGGCGCACTGGCCTCATCTTAGGCTTGTGTTATCTCCTAGGTTGGCTCTG |
| **newl166_solexa_F_CCT (SEQ ID NO: 97):** |
| TCTTGGtccgacGGCGCACTGGCCTCATCTTAGGCCCTTGTTATCTCCTAGGTTGGCTCTG |
| **new1166_solexa_F_GGA (SEQ ID NO: 98):** |
| TCTTGGtccgacGGCGCACTGGCCTCATCTTAGGCGGATGTTATCTCCTAGGTTGGCTCTG |
| **newll66-solexa-F-CAG (SEQ ID NO: 99):** |
| TCTTGGtccgacGGCGCACTGGCCTCATCTTAGGCCAGTGTTATCTCCTAGGTTGGCTCTG **newl166_solexa_F_CGC (SEQ ID NO: 100):** |
| TCTTGGtccgacGGCGCACTGGCCTCATCTTAGGCCGCTGTTATCTCCTAGGTTGGCTCTG |
| **new1166_solexa_F_GCC (SEQ ID NO: 101):** |
| TCTTGGtccgacGGCGCACTGGCCTCATCTTAGGCGCCTGTTATCTCCTAGGTTGGCTCTG |
| **new1166_solexa_F_GTT (SEQ ID NO: 102):** |
| TCTTGGtccgacGGCGCACTGGCCTCATCTTAGGCGTTTGTTATCTCCTAGGTTGGCTCTG |
| *B. Reverse primer:* |
| **1166_solexa_R (SEQ ID NO: 103):** |
| GCACAGCAGGCCAGTGTG |
| *C. Nested forward primer:* |
| **Solexa1_F (SEQ ID NO: 104):** |
| CTGGCCTCATCTTAG |
| **Amplification conditions:** |
| **Step 1:** 95°C for 3 min, then 20 cycles of 95°C for 20 sec, 62°C for 20 sec and 68°C for 30 secs. |
| **Step 2:** 95°C for 3 min, then 10 cycles of 95°C for 20 sec, 50°C for 10 sec and 68°C for 10 secs. End with 68°C for 5 min and 18°C, hold. |
| **Note:** |
| Forward oligos: Mmel site shown in box, 3 base solexa barcode underlined. |
| Mmel cuts at 5'-TCCRAC(N₂₀)-3'; 3'-AGGYTG(N₁₈)-5' (i.e. at **TA**) |

### 7.2 Illumina sequencing and basecalling - sequencing

PCR templates from 7.1.1 and 7.1.2 (see Table 12) were used for sequencing. These PCR templates were processed for Solexa adapter ligation and prepared for 104bp paired-end reads, as follows.

PCR products that contained single-read Solexa adapters on either end were mixed 1:1 with a phiX Solexa library (Illumina). Single-read v4 flowcells for the Genome Analyzer were used. After loading DNA at a concentration of 7 pM per flow cell lane, clusters were generated in the Illumina cluster station according to the recommendation of the manufacturer. Sequencing was performed on the Illumina Genome Analyzer IIx with TrueSeq SBS v5 sequencing chemistry, using a 104-cycle recipe. Basecalling was performed using SCS2.8.

**Table 12: PCR templates for sequencing**

| |
|---|
| **184bp-wt template (SEQ ID NO: 105)** |
| CTGGCCTCATCTTAGGCxxxTGTTATCTCCTAGGTTGGCTCTGACTGTACCACCATCCACTACAACTACATG TGTAACAGTTCCTGCATGGGCGGCATGAACCGGAGGCCCATCCTCACCATCATCACACTGGAAGACTCCAGG TCAGGAGCCACTTGCCACCCTGCACACTGGCCTGCTGTGC |
| **184bp-mut template (SEQ ID NO: 106)** |
| CTGGCCTCATCTTAGGCxxxTGTTATCTCCTAGGTTGGCTCTGACTGTACGACGATTCATTACAACTATATG TGCAATTCCTCCTGCATGGGCGGCATGAACCGGAGGCCCATCCTCACCATCATCACACTGGAAGACTCCAGG TCAGGAGCCACTTGCCACCCTGCACACTGGCCTGCTGTGC |
| **Key:** |
| a. ZFN binding site highlighted in bold |
| b. Solexa barcodes: xxx |
| c. For data processing: (i) NHEJ 1st filter (wt): prefix GGCXXXTGT and suffix *TTCCTGCAT*: (ii) NHEJ 2nd filter (30bp around ZFN site): prefix *CTGTAC* and suffix *TTCCTG*: (iii) homologous recombination filter: prefix GGCXXXTGT and suffix GACGATTCAT |

### 7.3 Measuring non-homologous end-joining by deep sequencing

Processing the data for measuring NHEJ required two steps. First, the different barcoded samples were extracted using filters for any sequences containing a 9bp prefix (with the 3bp unique sequencing barcode) and a 9bp suffix (after the ZFP binding site): i.e. prefix: NHEJ 1st filter (wt) GGCXXXTGT; suffix: TTCCTGCAT. Secondly, to reduce random sequencing errors (proportional to read length), the short, approximately 30bp region spanning the cutting site was filtered for sequences containing a new 6bp prefix-suffix combination (prefix: NHEJ 2nd filter (30bp around ZFN site): CTGTAC; suffix: TTCCTG). The percentage of sequences containing insertions or deletions was then calculated (see Figure 7B).

Using the 31 bp read protocol based on the template prepared in 7.1.1, NHEJ was observed from ZFNs in HEK293T cells (see Figure 7A). *Mme*I digestion allowed sequencing-adapter ligation as close to the region of interest as possible but, as a result, the method was qualitative rather than quantitative. Nonetheless, the method showed that ZFN treatment was required to observe insertions and deletions around the genomic cutting site.

Next, a series of experiments using an improved protocol with a 104bp read length was achieved using a Solexa Genome Analyser IIx. After external genomic PCR, a second internal PCR introduced a 3bp sequencing barcode (as previously described); the longer read length removed the need for *Mme*I digestion. Interestingly, we found that the slightly higher error rate of the newer HiSeq Solexa machine was suboptimal for this task, so the GA IIx was preferred. To achieve high-quality long reads with highly similar PCR products, the samples were 'spiked' with random DNA (phiX DNA fragments; 50% of total input DNA). In each flow cell lane, after computationally filtering out of phiX sequences, around 5 million sequences in the correct orientation (approx. 50%) were obtained. Thus it was possible to mix up to 8 sequencing barcodes per lane (each representing a different sample, under different conditions), resulting in around 600,000 reads each.

Different cancer cell lines (SF268, K562 and BT549) were tested, and ZFN-dependent indels were detected in all three; although they were much more frequent at 30°C (transient cold shock) than at constant 37°C, as has been recently reported (Doyon et al. (2010), Nat. Methods 7: 459-460). Furthermore, NHEJ was observed with both wild-type and obligate heterodimer Fokl. The increase in NHEJ signal with ZFN was up to 30-fold over background, indicating that next generation sequencing can be used reliably to measure this activity.

### 7.4 Measuring gene repair by deep sequencing

ZFN-driven gene repair was quantified in two human cancer cell lines (SF268, and BT-549). The cell lines were either transfected with z1166-ZFNs alone or with both ZFN and donor plasmid (to quantify ZFN-induced homologous recombination).

First, control reactions were carried out to test the ability of the Solexa system to detect proportions of wild-type or mutant DNA. Plasmid samples were mixed at ratios of 1:100 or 1:1000, and were then processed as if they were genomic PCRs (see Figure 7C). To process the data for measuring homologous recombination the different barcoded samples were extracted using filters for any sequences containing a 9bp prefix (with the 3bp unique sequencing barcode) and a 9bp suffix (after the ZFP binding site): barcode filter (gene repair) GGCXXXTGT; suffix: GACGATTCAT. The observed detection rate was found to be similar to that expected (i.e. close to 100:1 and 1000:1, respectively), despite the PCR amplification and adapter ligation steps during sample preparation.

Next, a variety of constructs with different promoters and *Fok*I nuclease variants were tested for their ability to induce homologous recombination (i.e. insertion of the donor plasmid sequence), and the results are illustrated in Figures 7D and 7E. Notably, the best results were obtained with the ZFNs comprising obligate heterodimer (ObH) *Fok*I nuclease (Miller et al. (2007), Nat. Biotechnol. 25: 778-785), under a PGK promoter.

Collecting the genomic DNA 7 days after the ZFN and donor plasmid transfection also helped to reduce background (Sigma Aldrich; see Compo-Zr instructions). Although the absolute rates of homologous recombination were apparently quite low (approximately 0.1%), this still represents an approximately 100-fold improvement over background, indicating that the ZFNs are functional at this locus (see Figure 7E).

In summary, we were able to use a novel yeast one-hybrid assay to engineer and select ZFNs from a library to bind to and cut p53 chromosomal targets, and thus, we were able to demonstrate their use for modifying genomes at the selected loci.

### Example 8

### Measurement of double-stranded breaks in ZFN-treated cells - ZFN toxicity

Initially, expression of the ZFN constructs was driven by a strong CMV promoter in order to produce high levels of ZFNs for use in assays. However, this was found to be suboptimal. It is possible that high expression levels of the nucleases were not well tolerated by the cells, and this may have led to their elimination over time through accumulation of non-specific double strand breaks (Alwin et al. (2005), Mol. Ther. 12: 610-617). Alternatively, the CMV promoter expressing ZFNs might compete with the CMV-driven GFP reporter, possibly leading to a decrease in expression of both peptides. For example, it was observed that CMV-HcRed plasmid, used as a transfection marker, had reduced activity when co-transfected with other CMV-driven plasmids, but not with PGK-driven plasmids. Possible adverse cellular effects from expressing high levels of ZFNs in cells were tested in a cellular toxicity assay.

HEK293T cells were grown as described above and transfected with a total of 200 ng ZFN expression vectors. As controls 200 ng of I-Scel expression vector and 200 ng of pUC19 were used. As a positive control for the induction of double stranded breaks, cells were treated with etoposide (1 mM) for 5 hrs. After 48 hrs cells were harvested and subjected to staining with antibodies against γH2A.X using the H2A.X Phosphorylation Assay Kit for Flow Cytometry (Upstate 17-344) following the manufacturer's protocol. Stained cells were analysed using a Guava Easycyte system, and the results are illustrated in Figure 8. These data show that pPGK constructs had lower toxicity than pCMV constructs. Thus, expression of ZFNs under the PGK promoter was preferred for *in vivo* assays and studies.

### Example 9

### Rational design of human p53 ZFP to bind other mammalian p53 gene sequences

The binding, cleavage and repair of human p53 gene sequences, as described in the Examples above, has particular utility in therapeutic and diagnostic applications in humans. However, it would be useful for the study, treatment and diagnosis of p53-associated genetic disorders (such as cancers) to be able to target p53 gene sequences in other organisms, particularly other mammals including pigs (porcine), sheep (ovine) and mouse (murine).

Sequence alignments of the p53 mutation hotspots around the z771 and z1166 ZFP binding sequences in human, porcine and ovine show that the sequences are fairly well conserved. In fact, the staggered 12bp binding sites targeted by the z771 L, z771 R and z1166R peptides (i.e. bs771L, bs771R and bs1166R, respectively) are identical in human, porcine and ovine p53 genes, which means that these ZFPs and ZFNs should be suitable for use in a variety of mammalian species. However, the staggered 12bp binding site targeted by ZFP z1166L (i.e. bs1166L) is not identical across these species, as shown in Table 13 below. Therefore, the publicly available information on the zinc finger-nucleic acid recognition code was used to rationally re-design the potentially suboptimal binding interaction that may otherwise result between z1166L selected to bind the human p53 gene sequence and the porcine and ovine p53 gene sequences at the z1166 position.

As indicated, one sequence difference at the z1166 binding site is a change from T to C in the nucleic acid position immediately adjacent the 3' end of the 12bp target site for z1166L. Since the complement of this residue (i.e. G on the complementary strand) may be recognised by the ++2 amino acid residue (i.e. the amino acid in the +2 position of finger 1), it was decided to rationally design an amino acid change from Asp to Ser at that position. A second change was T to C in the sixth position of the z1166L target site of the porcine p53 gene sequence. This residue may be recognised by the +6 position of finger 2, and by the ++2 position (i.e. the +2 position of finger 3). Since the amino acid at position +6 in finger 2 is Thr, which should be able to accommodate either a T or a C in this position, only the ++2 position was changed from Asp to Ser (as in finger 1 above).

For convenience the porcine / ovine targeting z1166L ZFP is termed z1166Lp.

**Table 13: z1166 binding site alignment, showing differences between human, porcine and ovine sequences; as well as differences between the α-helix nucleic acid recognition sequences for the z1166L ZFPs.**

| **Mammalian species** | **z1166 target binding site coding strand (5' to 3')** | α**-helix amino acids sequences for z1166L ZFP** |
|---|---|---|
| human | C CACCATCCACTA CAACTA CATGTGTAACAG T | F1 = RSDNLIV |
| | | F2 = QNANRNT |
| | | F3 = RSDALSR |
| | | F4 = NSSNRTV |
| porcine | C CACCATCCACTA CAACTt CATGTGcAACAG c | F1 = RS**S**NLIV |
| | | F2 = QNANRNT |
| | | F3 = RS**S**ALSR |
| | | F4 = NSSNRTV |
| ovine | C CACCATCCACTA CAACTt CATGTGTAACAG c | F1 = RS**S**NLIV |
| | | F2 = QNANRNT |
| | | F3 = RS**S**ALSR |
| | | F4 = NSSNRTV |
| **Key:** | | |
| a. z1166R binding site CACCATCCACTA (boxed) | | |
| b. z1166L binding site (underlined) | | |
| c. sequence variations between human, porcine and ovine p53 gene sequences indicated (lowercase in porcine and ovine sequences) | | |
| d. DNA-recognition α-helix sequences of fingers 1 to 4 (F1 to F4) of ZFP z1166L indicated, with rational mutations in porcine and ovine z1166L ZFP sequences shown (bold, underlined) | | |

The amino acid sequence of zinc finger domains 1 to 4 of z1166Lp (SEQ ID NO: 15) is shown in Table 14. Full nucleic acid and amino acid sequences of the ZFN comprising z1166Lp ZFP with obligate dimer Fokl nuclease domains are also illustrated in Table 14 (SEQ ID NOs: 9 and 10, respectively). ZFPs and ZFNs comprising the z1166Lp ZFP sequence can be constructed as previously described.

**Table 14: SEQ ID NOs: 9 and 10 are ZFN nucleic acid and corresponding protein sequences for z1166Lp for targeting porcine and/or ovine p53 gene sequences. These sequences comprise the sequence of zinc finger domains 1 to 4 of z1166Lp (SEQ ID NO: 15); and the Fokl obligate dimer nuclease domain sequences (Miller et al. (2007) Nat. Biotech., PMID 17603475). Alternatively, standard FokI domain could be used in place of the obligate dimer sequence. The target sites for z771 R, z771 L and z1166R peptides in sheep and pig are identical to the corresponding human sequences and so the ZFPs and ZFNs selected against the human sequences (particularly z1166R ZFP and ZFN) can be used in conjunction with z1166Lp.**

| |
|---|
| **SEQ ID NO: 9 (z1166Lp ZFN - nucleic acid sequence 5'-3')** |
| ATGGCCCCCAAAAAGAAGCGCAAGGTCGCCGAGGAACGCCCCTTCCAGTGTCGCATCTGTATGCGCAATTTC AGCCGCAGCtccAACCTGATAGTGCACATTCGCACGCACACCGGCGAAAAGCCCTTTGCCTGTGACATTTGT GGGCGCAAGTTCGCGCAGAACGCCAACCGGAACACGCATACCAAAATTCACACCGGATCTGAGCGCCCGTTT CAGTGCAGGATTTGCATGAGGAACTTCTCCCGGTCCagcGCCCTGAGCCGCCATATCAGGACCCATACCGGG GAGAAACCTTTCGCGTGCGATATCTGCGGGAGGAAATTCGCCAACAGCAGCAACCGGACAGTGCACACGAAG ATCCATCAGAACAAGAAGCAACTAGTcaaaagtgaactggaggagaagaaatctgaacttcgtcataaattg aaatatgtgcctcatgaatatattgaattaattgaaattgccagaaattccactcaggatagaattcttgaa atgaaggtaatggaatttttatgaaagtttatggatatagaggtaaacatttgggtggatcaaggaaaccg gacggagcaatttatactgtcggatctcctattgattacggtgtgatcgtggatactaaagcttatagcgga ggttataatctgccaattggccaagcagatgaaatgcaacgatatgtcaaagaaaatcaaacacgaaacaaa catatcaaccctaatgaatggtggaaagtctatccatcttctgtaacggaatttaagtttttatttgtgagt ggtcactttaaaggaaactacaaagctcagcttacacgattaaatcataagactaattgtaatggagctgtt cttagtgtagaagagcttttaattggtggagaaatgattaaagccggcacattaaccttagaggaagtgaga cggaaatttaataacggcgagataaactttTAA |
| **SEQ ID NO: 10 (z1166Lp ZFN - amino acid sequence N-C)** |
| MAPKKKRKVAEERP |
| FQCRICMRNFSRSSNLIVHIRTHTGEKP FACDICGRKFAQNANRNTHTKIHTGSERP FQCRICMRNFSRSSALSRHIRTHTGEKP FACDICGRKFANSSNRTVHTKIHQNKKQ lvkseleekkselrhklkyvpheyielieiarnstqdrilemkvmeffmkvygyrgkhlggsrkpdgaiytv gspidygvivdtkaysggynlpigqademqryvkenqtrnkhinpnewwkvypssvtefkflfvsghfkgny kaqltrlnhktncngavlsveelliggemikagtltleevrrkfnngeinf* |
| **z1166Lp ZFP (SEQ ID NO: 15)** |
| FQCRICMRNFSRSSNLIVHIRTHTGEKP FACDICGRKFAQNANRNTHTKIHTGSERP FQCRICMRNFSRSSALSRHIRTHTGEKP FACDICGRKFANSSNRTVHTKIH |
| **Key:** |
| a. ***ATG*** (nucleic acid sequences) = start codon |
| b. **PKKKRKV** (peptide sequences) = SV40 nuclear localisation signal |
| c. ZFP peptide sequence α-helices underlined from position -1 to position +6 |
| d. ACTAGT = *Spe*I site |
| e. *Fok*I sequences for obligate dimer shown (lower case in polynucleotide and peptide sequences); although normal *Fok*I sequences could be substituted according to preference |
| f. ***TAA*** = stop codon (not part of SEQ ID NOs) |

Although the z1166L binding sites are not identical between porcine and ovine p53 gene sequences, the modified porcine / ovine z1166L ZFP and corresponding ZFNs (also including z1166R ZFN, constructed similarly to that described in the Examples above) were tested *in vitro* and found to be active against nucleic acids having either porcine or ovine gene sequences in the z1166 region.

### Example 10

### Selection of six-finger ZFPs to bind EGFP gene sequence

The yeast one-hybrid assay described in Example 2 was modified in order to demonstrate its utility in selecting and maturing ZFPs having six zinc finger domains.

In this assay, the bait element comprised a single copy of the selected 18bp target site 5'- ATGGCGGACTTGAAGAAG -3' (SEQ ID NO: 107) from the EGFP gene sequence (see Figure 9A). In order to ensure that the selection is optimal for the correct genetic context of the EGFP gene target site, the one or two nucleic acids flanking the 3' and optionally the 5' end of the target sequence in the EGFP gene may also be included in the bait element; such that the full bait sequence from the EGFP gene used was a 19 nucleic acid stretch (i.e. 5'- ATGGCGGACTTGAAGAAGt -3'; SEQ ID NO: 108).

A six-finger ZFP was constructed from a zinc finger framework comprising three two-finger subdomains, similarly to that described for the four-finger peptides above. Rational design based on publicly available information on the zinc finger-nucleic acid recognition code was used to construct a six-finger peptide for binding to the target sequence. To mature (i.e. improve or optimise) the rationally design ZFP, a library was created by introducing diversifications (i.e. randomisations) at positions -1, +2, +3 and +6 of each of the six zinc finger domains. The ZFP library construct was constructed by PCR amplification to add suitable homology arms for homologous recombination at the 5' end of the Gal4 activation domain.

The yeast one-hybrid assay was performed as previously described, and ZFP constructs were isolated and sequenced from colonies growing on selective media.

Two six-finger ZFPs were selected, ZFP_GFPb249(L8G2-9)* and ZFP_GFPb249(L8F3-11), having the amino acid sequences shown in Figure 9B in the α-helix recognition regions of fingers 1 to 6. Both ZFPs were demonstrated to bind to the correct target nucleic acid sequence in gel-shift assays, as shown in Figure 9C.

These results demonstrate that the yeast one-hybrid assay may be used to select and/or mature DNA binding domains against a range of different target nucleic acid sequences and lengths. In particular, the yeast one-hybrid assay of the invention may be used successfully to select and mature ZFPs having four or six zinc finger domains. Therefore, ZFPs having arrays of four or more zinc finger domains can beneficially be selected and/or matured as a whole, rather than in smaller subdomains or arrays of one-or two-finger units.

While certain representative embodiments and details have been shown for purposes of illustrating the invention, it will be apparent to those skilled in the art that various changes may be made without departing from the scope of the invention, which is defined in the appended claims.

## Claims

1. A method for altering a p53 gene sequence, the method comprising:
contacting a nucleic acid molecule comprising the p53 gene sequence with one or more zinc finger nuclease (ZFN);
wherein the ZFN comprises a zinc finger peptide (ZFP) portion comprising four or more zinc finger domains which recognises at least 12bp of the p53 gene sequence, and one or more endonuclease portion; and
wherein binding of the one or more ZFN to the p53 gene sequence enables the one or more endonuclease portion to cleave the p53 gene sequence; and wherein the method is not a method for medical treatment of the human or animal body by surgery or therapy.

2. The method of Claim 1, wherein:
(a) the step of contacting a nucleic acid molecule comprising the p53 gene sequence with one or more ZFN is performed inside a cell, such that cleavage of the p53 gene sequence by the ZFN causes the p53 gene sequence to be mutated via non-homologous end joining (NHEJ); or
(b) the step of contacting a nucleic acid molecule comprising the p53 gene sequence with one or more ZFN is performed in the presence of a donor nucleic acid comprising an exogenous nucleic acid sequence, such that cleavage of the p53 gene sequence by the ZFN causes replacement of the p53 gene sequence by the exogenous nucleic acid sequence via homologous recombination.

3. The method of Claim 2(b), wherein the p53 gene sequence is a mutated p53 gene sequence and the exogenous nucleic acid sequence is a wild-type p53 gene sequence or encodes at least a portion of a wild-type p53 protein.

4. The method of any of Claims 1 to 3, wherein the nucleic acid molecule comprising the p53 gene sequence is chromosomal DNA and the method is performed within an animal cell.

5. The method of any of Claims 1 to 4, which comprises contacting the nucleic acid molecule comprising the p53 gene sequence with a first and a second ZFN, the first ZFN comprising a first ZFP portion and a first endonuclease portion, and the second ZFN comprising a second ZFP portion and a second endonuclease portion;
wherein the first and second ZFPs each comprise four or more zinc finger domains, the first ZFP portion recognising a first p53 gene sequence comprising at least 12bp and the second ZFP portion recognising a second p53 gene sequence comprising at least 12bp, the first and second p53 gene sequences not overlapping one another and being located within approximately 100bp of each other in the p53 gene sequence; and
wherein the first and second endonuclease portions of each polypeptide are partial nucleic acid cleavage domains which are capable of dimerising to form a functional endonuclease, such that the p53 gene sequence is cleaved when the first and second ZFNs are simultaneously bound to the first and second p53 gene sequences.

6. The method of any of Claims 1 to 5, wherein the ZFP comprising four or more zinc finger domains; and wherein the amino acid sequence of the nucleic acid recognition region (i.e. positions -1,+1,+2,+3,+4,+5,+6) of each of the four zinc finger domains (numbered F1, F2, F3, F4 in N to C terminal order) is identical in at least 6 of the 7 positions of the corresponding zinc finger nucleic acid recognition region sequences in the groups selected from:
(i) F1: RSSHLSR (SEQ ID NO: 16); F2: RNDNRKT (SEQ ID NO: 17); F3: RSSNLSQ (SEQ ID NO: 18); F4: DNSSRIR (SEQ ID NO: 19);
(ii) F1: RNSSLTN (SEQ ID NO: 20); F2: ATNSLIE (SEQ ID NO: 21); F3: RS(G/C)HLKT (SEQ ID NO: 22); F4: RSDNLKT (SEQ ID NO: 23);
(iii) F1: RSDTLSR (SEQ ID NO: 24); F2: RKDARIN (SEQ ID NO: 25); F3: RSSHLST (SEQ ID NO: 26); F4: KSDNRTT (SEQ ID NO: 27);
(iv) F1: RSDNLIV (SEQ ID NO: 28); F2: QNANRNT (SEQ ID NO: 29); F3: RSDALSR (SEQ ID NO: 30); F4: NSSNRTV (SEQ ID NO: 31);
(v) F1: RSSNLIV (SEQ ID NO: 32); F2: QNANRNT (SEQ ID NO: 33); F3: RSSALSR (SEQ ID NO: 34); F4: NSSNRTV (SEQ ID NO: 35).

7. A zinc finger nuclease (ZFN) for use in altering a p53 gene sequence, comprising:
contacting a nucleic acid molecule comprising the p53 gene sequence with one or more zinc finger nuclease (ZFN);
wherein the ZFN comprises a zinc finger peptide (ZFP) portion comprising four or more zinc finger domains which recognises at least 12bp of the p53 gene sequence, and one or more endonuclease portion; and
wherein binding of the one or more ZFN to the p53 gene sequence enables the one or more endonuclease portion to cleave the p53 gene sequence; and optionally
wherein:
(a) the step of contacting a nucleic acid molecule comprising the p53 gene sequence with one or more ZFN is performed inside a cell, such that cleavage of the p53 gene sequence by the ZFN causes the p53 gene sequence to be mutated via non-homologous end joining (NHEJ); or
(b) the step of contacting a nucleic acid molecule comprising the p53 gene sequence with one or more ZFN is performed in the presence of a donor nucleic acid comprising an exogenous nucleic acid sequence, such that cleavage of the p53 gene sequence by the ZFN causes replacement of the p53 gene sequence by the exogenous nucleic acid sequence via homologous recombination.

8. A polypeptide comprising a non-naturally occurring zinc finger peptide (ZFP), the ZFP comprising four or more zinc finger domains; and wherein the amino acid sequence of the nucleic acid recognition region (i.e. positions -1,+1,+2,+3,+4,+5,+6) of each of the four zinc finger domains (numbered F1, F2, F3, F4 in N to C terminal order) is identical in at least 6 of the 7 positions of the corresponding zinc finger nucleic acid recognition region sequences in the groups selected from:
(i) F1: RSSHLSR (SEQ ID NO: 16); F2: RNDNRKT (SEQ ID NO: 17); F3: RSSNLSQ (SEQ ID NO: 18); F4: DNSSRIR (SEQ ID NO: 19);
(ii) F1: RNSSLTN (SEQ ID NO: 20); F2: ATNSLIE (SEQ ID NO: 21); F3: RS(G/C)HLKT (SEQ ID NO: 22); F4: RSDNLKT (SEQ ID NO: 23);
(iii) F1: RSDTLSR (SEQ ID NO: 24); F2: RKDARIN (SEQ ID NO: 25); F3: RSSHLST (SEQ ID NO: 26); F4: KSDNRTT (SEQ ID NO: 27);
(iv) F1: RSDNLIV (SEQ ID NO: 28); F2: QNANRNT (SEQ ID NO: 29); F3: RSDALSR (SEQ ID NO: 30); F4: NSSNRTV (SEQ ID NO: 31);
(v) F1: RSSNLIV (SEQ ID NO: 32); F2: QNANRNT (SEQ ID NO: 33); F3: RSSALSR (SEQ ID NO: 34); F4: NSSNRTV (SEQ ID NO: 35).

9. The polypeptide of Claim 8, which is a zinc finger nuclease (ZFN) comprising a ZFP portion and one or more endonuclease portions; preferably wherein the endonuclease portion comprises a Fokl partial endonuclease domain.

10. The polypeptide of Claim 8 or Claim 9, the method of any of Claims 1 to 6, or the ZFN of Claim 7, wherein the ZFP portion recognises a target nucleic acid sequence comprising one or more sequence selected from: (i) 5'- GCCAAGCAGGGG -3' (SEQ ID NO: 41); (ii) 5'- CAGTGGCTCATG -3' (SEQ ID NO: 42); (iii) 5'- TAGTGGATGGTG -3' (SEQ ID NO: 43); (iv) 5'- CATGTGTAACAG -3' (SEQ ID NO: 44); and (v) 5'-CATGTGCAACAG -3' (SEQ ID NO: 45).

11. The polypeptide or ZFN of any of Claims 7 to 10, or a polypeptide comprising any of SEQ ID NOs: 2, 4, 6, 8, 10, 11, 12, 13, 14 or 15, for use in medicine or for a non-medical purpose.

12. A polynucleotide comprising a nucleic acid sequence encoding the ZFN of any of Claims 1 to 7, or one or more polypeptide according to any of Claims 8 to 11.

13. The polypeptide of Claim 11 or the polynucleotide of Claim 12 for use in treating a cancer in a subject, wherein the cancer is associated with aberrant expression of a p53 gene, or expression of an aberrant p53 gene.

14. A method for identifying a polypeptide that binds to a nucleic acid bait sequence element within a cell, the method comprising:
providing a cell comprising a nucleic acid bait construct capable of expressing a detectable reporter gene when the polypeptide binds to the bait sequence element, the nucleic acid bait construct comprising the reporter gene and a single copy of the bait sequence element; and
transforming the cell with an expression vector comprising the polypeptide gene and capable of expressing the polypeptide in the cell;
wherein expression of the reporter gene indicates that the polypeptide has bound to the bait sequence element in the cell.

15. The method of Claim 14, wherein the cell is a yeast cell; wherein the polypeptide comprises a zinc finger peptide; and wherein the expression vector is formed by homologous recombination within the yeast cell, between a nucleic acid prey construct comprising a nucleic acid sequence encoding a transcription factor activation domain and a nucleic acid construct comprising a nucleic acid sequence encoding the polypeptide, and wherein the homologous recombination results in the nucleic acid sequence encoding the polypeptide being recombined in-frame with and N-terminal to a transcription factor activation domain in the prey construct.
